# EUROPEAN PATENT APPLICATION

(11) **EP 4 001 417 A1**
(43) Date of publication of application: **25.05.2022**
(21) Application number: 20207352.4
(22) Date of filing: 13.11.2020
(51) Int. Cl.: C12N 15/81

(54) **YEAST PLATFORM FOR THE PRODUCTION OF VACCINES**

(71) Applicant: serYmun Yeast GmbH, 06406 Bernburg (Saale) (DE)
(72) Inventor: Mehlgarten, Constance, 06108 Halle (Saale) (DE); Breunig, Karin, 14109 Berlin (DE); Zabel, René, 06193 Petersberg (DE); Franke, Volker, 30938 Burgwedel (DE); Kleindienst, Carolin, 06108 Halle (Saale) (DE)
(74) Representative: Greiner, Elisabeth

(57) **Abstract**

The invention relates to the provision of recombinant yeast cells for the efficient and stable expression of transgenes, preferably for the expression of one or more immunogenic polypeptide(s) derived from a pathogen. The invention further relates to vaccine compositions comprising said recombinant yeast cells, uses of said recombinant yeast cells in methods for vaccination and methods for the production of a whole yeast vaccine comprising at least one diploid recombinant yeast cell of the invention. Further encompassed are methods for the provision of a diploid yeast cell from a wild type yeast strain.

SEQUENCE LISTING

<110> SerYmun Yeast GmbH

<120> YEAST PLATFORM FOR THE PRODUCTION OF VACCINES

<130> SER24286EP

<160> 37

<170> BiSSAP 1.3.6

<210> 1
<211> 890
<212> PRT
<213> Reoviridae

<400> 1
Met Ala Tyr Arg Lys Arg Gly Ala Lys Arg Glu Asn Ser Ser Gln Gln
1               5                   10                  15
Asn Glu Arg Leu Gln Glu Lys Glu Val Glu Lys Asn Ile Asp Ala Ser
            20                  25                  30
Met Glu Asn Lys Ala Asn Asn Lys Lys Gln Gln Leu Pro Asp Lys Val
        35                  40                  45
Leu Ser Gln Lys Glu Glu Ile Ile Thr Asp Ala Gln Asp Asp Val Lys
    50                  55                  60
Ile Thr Asp Glu Val Lys Lys Ser Ser Lys Glu Glu Ser Lys Gln Leu
65                  70                  75                  80
Leu Glu Ile Leu Lys Thr Lys Glu Asp His Gln Lys Glu Ile Gln Tyr
                85                  90                  95
Glu Ile Leu Gln Lys Thr Ile Pro Thr Phe Glu Pro Lys Glu Ser Ile
            100                 105                 110
Leu Lys Lys Leu Glu Asp Ile Lys Pro Glu Gln Ala Lys Lys Gln Thr
        115                 120                 125
Lys Leu Phe Arg Ile Phe Glu Pro Arg Gln Leu Pro Ile Tyr Arg Ala
    130                 135                 140
Asn Gly Glu Arg Glu Leu Arg Asn Arg Trp Tyr Trp Lys Leu Lys Arg
145                 150                 155                 160
Asp Thr Leu Pro Asp Gly Asp Tyr Asp Val Arg Glu Tyr Phe Leu Asn
                165                 170                 175
Leu Tyr Asp Gln Ile Leu Ile Glu Met Pro Asp Tyr Leu Leu Leu Lys
            180                 185                 190
Asp Met Ala Val Glu Asn Lys Asn Ser Arg Asp Ala Gly Lys Val Val
        195                 200                 205
Asp Ser Glu Thr Ala Ser Ile Cys Asp Ala Ile Phe Gln Asp Glu Glu
    210                 215                 220
Thr Glu Gly Val Ile Arg Arg Phe Ile Ala Asp Met Arg Gln Gln Val
225                 230                 235                 240
Gln Ala Asp Arg Asn Val Val Asn Tyr Pro Ser Ile Leu His Pro Ile
                245                 250                 255
Asp His Ala Phe Asn Glu Tyr Phe Leu Asn His Gln Leu Val Glu Pro
            260                 265                 270
Leu Asn Asn Glu Ile Ile Phe Asn Tyr Ile Pro Glu Arg Ile Arg Asn
        275                 280                 285
Asp Val Asn Tyr Ile Leu Asn Met Asp Met Asn Leu Pro Ser Thr Ala
    290                 295                 300
Arg Tyr Ile Arg Pro Asn Leu Leu Gln Asp Arg Leu Asn Leu His Asp
305                 310                 315                 320
Asn Phe Glu Ser Leu Trp Asp Thr Ile Thr Thr Ser Asn Tyr Ile Leu
                325                 330                 335
Ala Arg Ser Val Val Pro Asp Leu Lys Glu Lys Glu Leu Val Ser Thr
            340                 345                 350

Glu Ala Gln Ile Gln Lys Met Ser Gln Asp Leu Gln Leu Glu Ala Leu
        355                 360                 365
Thr Ile Gln Ser Glu Thr Gln Phe Leu Ala Gly Ile Asn Ser Gln Ala
    370                 375                 380
Ala Asn Asp Cys Phe Lys Thr Leu Ile Ala Ala Met Leu Ser Gln Arg
385                 390                 395                 400
Thr Met Ser Leu Glu Phe Val Thr Thr Asn Tyr Met Ser Leu Ile Ser
                405                 410                 415
Gly Met Trp Leu Leu Thr Val Ile Pro Asn Asp Met Phe Leu Arg Glu
            420                 425                 430
Ser Leu Val Ala Cys Glu Leu Ala Ile Ile Asn Thr Ile Val Tyr Pro
        435                 440                 445
Ala Phe Gly Met Gln Arg Met His Tyr Arg Asn Gly Asp Pro Gln Thr
    450                 455                 460
Pro Phe Gln Ile Ala Glu Gln Gln Ile Gln Asn Phe Gln Val Ala Asn
465                 470                 475                 480
Trp Leu His Phe Ile Asn Asn Asn Arg Phe Arg Gln Val Val Ile Asp
                485                 490                 495
Gly Val Leu Asn Gln Thr Leu Asn Asp Asn Ile Arg Asn Gly Gln Val
            500                 505                 510
Ile Asn Gln Leu Met Glu Ala Leu Met Gln Leu Ser Arg Gln Gln Phe
        515                 520                 525
Pro Thr Met Pro Val Asp Tyr Lys Arg Ser Ile Gln Arg Gly Ile Leu
    530                 535                 540
Leu Leu Ser Asn Arg Leu Gly Gln Leu Val Asp Leu Thr Arg Leu Leu
545                 550                 555                 560
Ser Tyr Asn Tyr Glu Thr Leu Met Ala Cys Ile Thr Met Asn Met Gln
                565                 570                 575
His Val Gln Thr Leu Thr Thr Glu Lys Leu Gln Leu Thr Ser Val Thr
            580                 585                 590
Ser Leu Cys Met Leu Ile Gly Asn Thr Thr Val Ile Pro Ser Pro Gln
        595                 600                 605
Thr Leu Phe His Tyr Tyr Asn Val Asn Val Asn Phe His Ser Asn Tyr
    610                 615                 620
Asn Glu Arg Ile Asn Asp Ala Val Ala Ile Ile Thr Ala Ala Asn Arg
625                 630                 635                 640
Leu Asn Leu Tyr Gln Lys Lys Met Lys Ser Ile Val Glu Glu Phe Leu
                645                 650                 655
Lys Arg Leu Gln Ile Phe Asp Val Pro Arg Val Pro Asp Asp Gln Met
            660                 665                 670
Tyr Arg Leu Arg Asp Arg Leu Arg Leu Leu Pro Val Glu Arg Arg Arg
        675                 680                 685
Leu Asp Ile Phe Asn Leu Ile Leu Met Asn Met Glu Gln Ile Glu Arg
    690                 695                 700
Ala Ser Asp Lys Ile Ala Gln Gly Val Ile Ile Ala Tyr Arg Asp Met
705                 710                 715                 720
Gln Leu Glu Arg Asp Glu Met Tyr Gly Tyr Val Asn Ile Ala Arg Asn
                725                 730                 735
Leu Asp Gly Tyr Gln Gln Ile Asn Leu Glu Glu Leu Met Arg Thr Gly
            740                 745                 750
Asp Tyr Gly Gln Ile Thr Asn Met Leu Leu Asn Asn Gln Pro Val Ala
        755                 760                 765
Leu Val Gly Ala Leu Pro Phe Val Thr Asp Ser Ser Val Ile Ser Leu
    770                 775                 780
Ile Ala Lys Leu Asp Ala Thr Val Phe Ala Gln Ile Val Lys Leu Arg
785                 790                 795                 800
Lys Val Asp Thr Leu Lys Pro Ile Leu Tyr Lys Ile Asn Ser Asp Ser
                805                 810                 815
Asn Asp Phe Tyr Leu Val Ala Asn Tyr Asp Trp Ile Pro Thr Ser Thr
            820                 825                 830
Thr Lys Val Tyr Lys Gln Val Pro Gln Pro Phe Asp Phe Arg Ala Ser
        835                 840                 845
Met His Met Leu Thr Ser Asn Leu Thr Phe Thr Val Tyr Ser Asp Leu

850                 855                 860
Leu Ala Phe Val Ser Ala Asp Thr Val Glu Pro Ile Asn Ala Val Ala
865                 870                 875                 880
Phe Asp Asn Met Arg Ile Met Asn Glu Leu
                885                 890

<210> 2
<211> 776
<212> PRT
<213> Reoviridae

<400> 2
Met Ala Ser Leu Ile Tyr Arg Gln Leu Leu Thr Asn Ser Tyr Thr Val
1               5                   10                  15
Asn Leu Ser Asp Glu Ile Gln Glu Ile Gly Ser Ala Lys Ser Gln Asp
            20                  25                  30
Val Thr Ile Asn Pro Gly Pro Phe Ala Gln Thr Gly Tyr Ala Pro Val
        35                  40                  45
Asn Trp Gly Ala Gly Glu Thr Asn Asp Ser Thr Thr Val Glu Pro Leu
    50                  55                  60
Leu Asp Gly Pro Tyr Gln Pro Thr Thr Phe Asn Pro Pro Thr Ser Tyr
65                  70                  75                  80
Trp Val Leu Leu Ala Pro Thr Val Glu Gly Val Ile Ile Gln Gly Thr
                85                  90                  95
Asn Asn Thr Asp Arg Trp Leu Ala Thr Ile Leu Ile Glu Pro Asn Val
            100                 105                 110
Gln Thr Thr Asn Arg Ile Tyr Asn Leu Phe Gly Gln Gln Val Thr Leu
        115                 120                 125
Ser Val Glu Asn Thr Ser Gln Thr His Trp Lys Phe Ile Asp Val Ser
    130                 135                 140
Thr Thr Thr Pro Thr Gly Ser Tyr Thr Gln His Gly Pro Leu Phe Ser
145                 150                 155                 160
Thr Pro Lys Leu Tyr Ala Val Met Lys Phe Ser Gly Arg Ile Tyr Thr
                165                 170                 175
Tyr Asn Gly Thr Thr Pro Asn Ala Thr Thr Gly Tyr Tyr Ser Thr Thr
            180                 185                 190
Asn Tyr Asp Thr Val Asn Met Thr Ser Phe Cys Asp Phe Tyr Ile Ile
        195                 200                 205
Pro Arg Asn Gln Glu Glu Lys Cys Thr Glu Tyr Ile Asn His Gly Leu
    210                 215                 220
Pro Pro Ile Gln Asn Thr Arg Asn Val Val Pro Val Ser Leu Ser Ala
225                 230                 235                 240
Arg Glu Ile Val His Thr Arg Ala Gln Val Asn Glu Asp Ile Val Val
                245                 250                 255
Ser Lys Thr Ser Leu Trp Lys Glu Met Gln Cys Asn Arg Asp Ile Thr
            260                 265                 270
Ile Arg Phe Lys Phe Asp Arg Thr Ile Ile Lys Ala Gly Gly Leu Gly
        275                 280                 285
Tyr Lys Trp Ser Glu Ile Ser Phe Lys Pro Ile Thr Tyr Gln Tyr Thr
    290                 295                 300
Tyr Ala Arg Asp Gly Glu Gln Ile Thr Ala His Thr Thr Cys Ser Val
305                 310                 315                 320
Asn Gly Val Asn Asn Phe Ser Tyr Asn Gly Gly Ser Leu Pro Thr Asp
                325                 330                 335
Phe Ala Ile Ser Arg Tyr Glu Val Ile Lys Glu Asn Ser Phe Val Tyr
            340                 345                 350
Ile Asp Tyr Trp Asp Asp Ser Gln Ala Phe Arg Asn Met Val Tyr Val
        355                 360                 365
Arg Ser Leu Ala Ala Asn Leu Asn Thr Val Thr Cys Thr Gly Gly Ser
    370                 375                 380
Tyr Ser Phe Ala Leu Pro Leu Gly His Tyr Pro Val Met Thr Gly Gly
385                 390                 395                 400

Thr Val Ser Leu His Pro Ala Gly Val Thr Leu Ser Thr Gln Phe Thr
                405                 410                 415
Asp Phe Val Ser Leu Asn Ser Leu Arg Phe Arg Phe Arg Leu Thr Val
            420                 425                 430
Gly Glu Pro Ser Phe Ser Ile Thr Arg Thr Arg Val Ser Arg Leu Tyr
        435                 440                 445
Gly Leu Pro Ala Ala Asn Pro Asn Asn Gln Arg Glu Tyr Tyr Glu Ile
    450                 455                 460
Ser Gly Arg Phe Ser Leu Ile Ser Leu Val Pro Ser Asn Asp Asp Tyr
465                 470                 475                 480
Gln Thr Pro Ile Met Asn Ser Val Thr Val Arg Gln Asp Leu Glu Arg
                485                 490                 495
Gln Leu Gly Glu Leu Arg Asp Glu Phe Asn Ser Leu Ser Gln Gln Ile
            500                 505                 510
Ala Ile Ser Gln Leu Ile Asp Leu Ala Leu Leu Pro Leu Asp Met Phe
        515                 520                 525
Ser Met Phe Ser Gly Ile Lys Ser Thr Ile Asp Ala Ala Lys Ser Met
    530                 535                 540
Ala Thr Asn Val Met Lys Arg Phe Lys Arg Ser Asn Leu Ala Ser Ser
545                 550                 555                 560
Val Ser Thr Leu Thr Asp Ala Met Ser Asp Ala Ala Ser Ser Ile Ser
                565                 570                 575
Arg Ser Ser Ser Ile Arg Ser Ile Gly Ser Ser Ala Ser Ala Trp Thr
            580                 585                 590
Glu Val Ser Asn Ser Ile Ala Asp Val Ser Thr Thr Val Asp Thr Val
        595                 600                 605
Ser Thr Gln Thr Ala Thr Ile Ala Lys Arg Leu Arg Leu Lys Glu Ile
    610                 615                 620
Ala Thr Gln Thr Asp Gly Met Asn Phe Asp Asp Ile Ser Ala Ala Val
625                 630                 635                 640
Leu Lys Thr Lys Ile Asp Lys Ser Val Gln Ile Thr Pro Asn Thr Leu
                645                 650                 655
Pro Glu Ile Val Thr Glu Ala Ser Glu Lys Phe Ile Pro Asn Arg Thr
            660                 665                 670
Tyr Arg Val Ile Asn Asn Asp Glu Val Phe Glu Val Gly Met Asp Gly
        675                 680                 685
Lys Phe Phe Ala Tyr Arg Val Asp Thr Phe Asp Glu Ile Pro Phe Asp
    690                 695                 700
Val Gln Lys Phe Ala Asp Leu Val Thr Asp Ser Pro Val Ile Ser Ala
705                 710                 715                 720
Ile Ile Asp Leu Lys Thr Leu Lys Asn Leu Lys Asp Asn Tyr Gly Ile
                725                 730                 735
Ser Lys Gln Gln Ala Phe Asp Leu Leu Arg Ser Asp Pro Arg Val Leu
            740                 745                 750
Arg Glu Phe Ile Asn Gln Asn Asn Pro Ile Ile Arg Asn Arg Ile Glu
        755                 760                 765
Asn Leu Ile Met Gln Cys Arg Leu
    770                 775

<210> 3
<211> 397
<212> PRT
<213> Reoviridae

<400> 3
Met Glu Val Leu Tyr Ser Leu Ser Lys Thr Leu Lys Asp Ala Arg Asp
1               5                   10                  15
Lys Ile Val Glu Gly Thr Leu Tyr Ser Asn Ile Ser Asp Leu Ile Gln
            20                  25                  30
Gln Phe Asn Gln Met Ile Val Thr Met Asn Gly Asn Asp Phe Gln Thr
        35                  40                  45
Gly Gly Ile Gly Asn Leu Pro Ile Arg Asn Trp Asn Phe Asp Phe Gly

50                  55                  60
Leu Leu Gly Thr Thr Leu Leu Asn Ile Asp Ala Asn Tyr Val Glu Asn
65                  70                  75                  80
Ala Arg Thr Thr Ile Glu Tyr Phe Ile Asp Phe Ile Asp Asn Val Cys
                85                  90                  95
Met Asp Glu Met Ala Arg Glu Ser Gln Arg Asn Gly Ile Ala Pro Gln
            100                 105                 110
Ser Glu Ala Leu Arg Lys Leu Ser Gly Ile Lys Phe Lys Arg Ile Asn
        115                 120                 125
Phe Asp Asn Ser Ser Asp Tyr Ile Glu Asn Trp Asn Leu Gln Asn Arg
    130                 135                 140
Arg Gln Arg Thr Gly Phe Val Phe His Lys Pro Asn Ile Leu Pro Tyr
145                 150                 155                 160
Ser Ala Ser Phe Thr Leu Asn Arg Ser Gln Pro Ala His Asp Asn Leu
                165                 170                 175
Met Gly Thr Met Trp Ile Asn Ala Gly Ser Glu Ile Gln Val Ala Gly
            180                 185                 190
Phe Asp Tyr Ser Cys Ala Phe Asn Ala Pro Ala Asn Ile Gln Gln Phe
        195                 200                 205
Glu His Val Val Pro Leu Arg Arg Ala Leu Thr Thr Ala Thr Ile Thr
    210                 215                 220
Leu Leu Pro Asp Ala Glu Arg Phe Ser Phe Pro Arg Val Ile Asn Ser
225                 230                 235                 240
Ala Asp Gly Ala Thr Thr Trp Tyr Phe Asn Pro Val Ile Ile Arg Pro
                245                 250                 255
Ser Asn Val Glu Val Glu Phe Leu Leu Asn Gly Gln Ile Ile Asn Thr
            260                 265                 270
Tyr Gln Ala Arg Phe Gly Thr Ile Ile Ala Arg Asn Phe Asp Thr Ile
        275                 280                 285
Arg Leu Ser Phe Gln Leu Val Arg Pro Pro Asn Met Thr Pro Ala Val
    290                 295                 300
Ala Asn Leu Phe Pro Gln Ala Pro Pro Phe Ile Phe His Ala Thr Val
305                 310                 315                 320
Gly Leu Thr Leu Arg Ile Glu Ser Ala Val Cys Glu Ser Val Leu Ala
                325                 330                 335
Asp Ala Ser Glu Thr Leu Leu Ala Asn Val Thr Ala Val Arg Gln Glu
            340                 345                 350
Tyr Ala Ile Pro Val Gly Pro Val Phe Pro Pro Gly Met Asn Trp Thr
        355                 360                 365
Glu Leu Ile Thr Asn Tyr Ser Pro Ser Arg Glu Asp Asn Leu Gln Arg
    370                 375                 380
Val Phe Thr Val Ala Ser Ile Arg Ser Met Leu Ile Lys
385                 390                 395

<210> 4
<211> 326
<212> PRT
<213> Reoviridae

<400> 4
Met Tyr Gly Ile Glu Tyr Thr Thr Val Leu Thr Phe Leu Ile Ser Leu
1               5                   10                  15
Val Phe Val Asn Tyr Ile Leu Lys Ser Val Thr Arg Thr Met Asp Phe
            20                  25                  30
Ile Ile Tyr Arg Phe Leu Leu Val Ile Val Val Leu Ala Pro Leu Ile
        35                  40                  45
Lys Ala Gln Asn Tyr Gly Ile Asn Leu Pro Ile Thr Gly Ser Met Asp
    50                  55                  60
Thr Pro Tyr Met Asn Ser Thr Thr Ser Glu Thr Phe Leu Thr Ser Thr
65                  70                  75                  80
Leu Cys Leu Tyr Tyr Pro Asn Glu Ala Ala Thr Glu Ile Ala Asp Thr
                85                  90                  95

Lys Trp Thr Glu Thr Leu Ser Gln Leu Phe Leu Thr Lys Gly Trp Pro
            100                 105                 110
Thr Gly Ser Val Tyr Phe Lys Gly Tyr Ala Asp Ile Ala Ser Phe Ser
        115                 120                 125
Val Glu Pro Gln Leu Tyr Cys Asp Tyr Asn Ile Val Leu Met Lys Tyr
    130                 135                 140
Asp Gly Asn Leu Gln Leu Asp Met Ser Glu Leu Ala Asp Leu Ile Leu
145                 150                 155                 160
Asn Glu Trp Leu Cys Asn Pro Met Asp Ile Met Leu Tyr Tyr Tyr Gln
                165                 170                 175
Gln Thr Asp Glu Ala Asn Lys Trp Ile Ser Met Gly Thr Ser Cys Thr
            180                 185                 190
Ile Lys Val Cys Pro Leu Asn Thr Gln Thr Leu Gly Ile Gly Cys Ser
        195                 200                 205
Thr Thr Asp Ile Asn Ser Phe Glu Thr Val Ala Asn Ala Glu Lys Leu
    210                 215                 220
Ala Ile Thr Asp Val Val Asp Gly Val Asn His Lys Leu Asp Val Thr
225                 230                 235                 240
Thr Ser Thr Cys Thr Ile Arg Asn Cys Lys Lys Leu Gly Pro Arg Glu
                245                 250                 255
Asn Val Ala Val Ile Gln Val Gly Gly Pro Asn Ile Leu Asp Ile Thr
            260                 265                 270
Ala Asp Pro Thr Thr Ala Pro Gln Thr Glu Arg Met Met Arg Ile Asn
        275                 280                 285
Trp Lys Arg Trp Trp Gln Val Phe Tyr Thr Ile Val Asp Tyr Val Asn
    290                 295                 300
Gln Ile Val Gln Val Met Ser Lys Arg Ser Arg Ser Leu Asp Ser Ala
305                 310                 315                 320
Ala Phe Tyr Tyr Arg Val
                325

<210> 5
<211> 317
<212> PRT
<213> Reoviridae

<400> 5
Met Ala Glu Leu Ala Cys Phe Cys Tyr Pro His Leu Glu Asn Asp Ser
1               5                   10                  15
Tyr Lys Phe Ile Pro Phe Asn Asn Leu Ala Ile Lys Cys Met Leu Thr
            20                  25                  30
Ala Lys Val Asp Lys Lys Asp Gln Asp Lys Phe Tyr Asn Ser Ile Val
        35                  40                  45
Tyr Gly Ile Ala Pro Pro Pro Gln Phe Lys Lys Arg Tyr Asn Thr Ser
    50                  55                  60
Asp Asn Ser Arg Gly Met Asn Tyr Val Thr Thr Met Phe Asn Lys Val
65                  70                  75                  80
Ala Ile Leu Ile Cys Glu Ala Leu Asn Ser Ile Lys Val Thr Gln Ser
                85                  90                  95
Asp Val Ala Asn Val Leu Ser Arg Val Val Ser Val Arg His Leu Glu
            100                 105                 110
Asn Leu Val Leu Arg Lys Glu Asn His Gln Asp Val Leu Phe His Ser
        115                 120                 125
Lys Glu Leu Leu Leu Lys Ser Val Leu Ile Ala Ile Gly Gln Ser Lys
    130                 135                 140
Glu Ile Glu Thr Thr Ala Thr Ala Glu Gly Gly Glu Ile Val Phe Gln
145                 150                 155                 160
Asn Ala Ala Phe Thr Met Trp Lys Leu Thr Tyr Leu Asp His Lys Leu
                165                 170                 175
Met Pro Ile Leu Asp Gln Asn Phe Thr Glu Tyr Lys Ile Thr Leu Asn
            180                 185                 190
Glu Asp Lys Pro Ile Ser Asp Val Cys Val Lys Glu Leu Val Ala Glu

195                 200                 205
Leu Arg Trp Gln Tyr Asn Arg Phe Ala Val Ile Thr His Gly Lys Gly
    210                 215                 220
His Tyr Arg Val Val Lys Tyr Ser Ser Val Ala Asn His Ala Asp Arg
225                 230                 235                 240
Val Phe Ala Thr Tyr Lys Asn Asn Ala Lys Ser Gly Asn Val Thr Asp
                245                 250                 255
Phe Asn Leu Leu Asp Gln Arg Ile Ile Trp Gln Asn Trp Tyr Ala Phe
            260                 265                 270
Thr Ser Ser Met Lys Gln Gly Asn Thr Leu Asp Val Cys Lys Lys Leu
        275                 280                 285
Leu Phe Gln Lys Met Lys Gln Glu Lys Asn Pro Phe Lys Gly Leu Ser
    290                 295                 300
Thr Asp Arg Lys Met Asp Glu Val Ser His Val Gly Ile
305                 310                 315

<210> 6
<211> 175
<212> PRT
<213> Reoviridae

<400> 6
Met Asp Lys Leu Ala Asp Leu Asn Tyr Thr Leu Ser Val Ile Thr Leu
1               5                   10                  15
Met Asn Asp Thr Leu His Ser Ile Ile Gln Asp Pro Gly Met Ala Tyr
            20                  25                  30
Phe Pro Tyr Ile Ala Ser Val Leu Thr Val Leu Phe Thr Leu His Lys
        35                  40                  45
Ala Ser Ile Pro Thr Met Lys Ile Ala Leu Lys Thr Ser Lys Cys Ser
    50                  55                  60
Tyr Lys Val Ile Lys Tyr Cys Met Val Thr Ile Ile Asn Thr Leu Leu
65                  70                  75                  80
Lys Leu Ala Gly Tyr Lys Glu Gln Val Thr Thr Lys Asp Glu Ile Glu
                85                  90                  95
Gln Gln Met Asp Arg Ile Ile Lys Glu Met Arg Arg Gln Leu Glu Met
            100                 105                 110
Ile Asp Lys Leu Thr Thr Arg Glu Ile Glu Gln Val Glu Leu Leu Lys
        115                 120                 125
Arg Ile His Asp Lys Leu Ala Ala Arg Ser Val Asp Ala Ile Asp Met
    130                 135                 140
Ser Lys Glu Phe Asn Gln Lys Asn Ile Arg Thr Leu Asp Glu Trp Glu
145                 150                 155                 160
Ser Gly Lys Asn Pro Tyr Glu Pro Ser Glu Val Thr Ala Ser Met
                165                 170                 175

<210> 7
<211> 2676
<212> DNA
<213> Reoviridae

<400> 7
tactaacagt tataccaaac gacagctaaa cgtgaaaact catcacaaca gaatgaacgt      60

ctgcaagaaa aagaagttga aaagaacata gacgcatcta tggagaataa agctaataat     120

aaaaagcaac aattacctga taaagtgtta tcacaaaaag aagagataat aactgacgca     180

caagatgatg ttaagataac tgatgaggtt aagaaatcat caaaagaaga atcgaaacag     240

cttttggaga tattgaaaac taaagaagat catcagaaag agatacaata tgaaatttta     300

cagaaaacga taccaacttt tgaaccaaaa gaatcaattt tgaaaaaatt agaagacata 360

aaaccagaac aggcaaaaaa acaaactaaa ttgtttagaa tatttgaacc aagacaatta 420

ccaatttatc gggcaaatgg tgagagagaa ttgagaaata gatggtattg gaaattgaaa 480

agagacacgc tgccagatgg agattatgat gtccgagaat acttcttaaa tttatatgat 540

cagatattga tagaaatgcc agattattta ttattgaaag atatggcagt ggagaataaa 600

aattctaggg atgctggtaa agtcgtggat tccgagacag caagtatttg tgatgccata 660

tttcaagatg aggaaacaga gggagtcatt agaaggttca ttgcagatat gaggcaacaa 720

gttcaagctg acagaaatgt tgttaactat ccatcaattt tacatccaat tgatcatgca 780

ttcaatgaat actttttaaa tcaccaatta gttgaaccat taaataatga aataattttt 840

aattatatac cagaaaggat aagaaacgat gttaattaca ttctaaacat ggatatgaat 900

ctaccatcaa cagctagata tattagacca aatttgctgc aagatagact aaatttgcat 960

gataattttg aatcattatg ggatacgata acaacatcaa actatatatt agctagatca 1020

gttgtgcctg atctgaagga aaaggagtta gtgtcaaccg aagctcaaat acagaaaatg 1080

tcccaagatt tgcaacttga agcactaact atacaatctg aaacgcagtt tctggctggt 1140

ataaattcac aagcagcaaa tgattgtttt aaaacgctaa tagcagccat gttaagccaa 1200

cgtacaatgt cattagaatt tgtgactaca aattacatgt cacttatatc tggtatgtgg 1260

ttactaacag ttataccaaa cgacatgttt cttcgtgaat cgctagttgc atgcgagttg 1320

gctataataa acactattgt ttatccagcg ttcggaatgc aaaggatgca ttatagaaat 1380

ggtgatccac agactccatt ccaaatagca gaacagcaga tacaaaattt tcaagtagct 1440

aattggctac actttatcaa caataataga tttagacaag ttgtgatcga cggagtgttg 1500

aatcaaacac ttaatgataa tattagaaat ggacaagtta ttaatcagtt gatggaagcg 1560

ttaatgcaac tatctagaca acagtttcca actatgccag ttgattataa aagatcaata 1620

caaagaggaa tattactatt gtcaaacaga ttaggtcaat tagttgactt aacaagacta 1680

ttatcatata actatgaaac actgatggct tgcataacca tgaatatgca acatgtacaa 1740

actcttacta ctgaaaaatt acaattaact tctgttacat ctttgtgtat gttaattgga 1800

aatactacag ttattccaag tccacaaaca ttgtttcact attacaatgt aaatgtaaat 1860

tttcattcaa actataacga acggattaac gatgcagtag caattattac agcagccaat 1920

agactgaatt tatatcagaa gaagatgaag tcaatagttg aagaattttt gaagaggctg 1980

caaattttcg atgtgccacg agtgcccgat gatcaaatgt atagactgag agatagactt 2040

agattgttgc cggttgaaag acggagactt gatatattca atttaatatt aatgaatatg 2100

gagcagatcg agcgagcttc agataaaatt gcacaaggtg taataattgc ttatagagat 2160

atgcaactag agagagatga aatgtatgga tacgtcaata ttgctagaaa tctggatgga 2220

taccaacaaa ttaatttaga agaattaatg aggacaggag attatggtca aattactaac 2280

atgctattga acaaccagcc agtggctcta gtaggagcat tgccattcgt aacagactca 2340

tcagttatat cactcattgc taagctggat gctacagttt tcgcccagat agtcaaactt 2400

aggaaagtag ataccttaaa accgatattg tataaaataa actctgattc taatgatttc 2460

tatttagttg caaattacga ttggatacca acttccacta caaaagtgta caaacaggta 2520

ccgcaaccct ttgactttag agcgtcaatg catatgttaa catctaacct cacttttact 2580

gtttattccg atttgttagc ttttgtttct gcagatacag ttgaaccgat caacgcagtt 2640

gcttttgaca atatgcgcat tatgaacgaa ctgtaa 2676

<210> 8
<211> 2331
<212> DNA
<213> Reoviridae

<400> 8
atggcttcgc tcatttatag acaactactt actaattcat acacagtcaa tctttctgac 60

gaaattcaag agattggatc ggctaagtca caggatgtta ctataaatcc tggtccattc 120

gcacaaacag gttatgcacc agttaattgg ggagcaggtg agactaatga ctccacaact 180

gtcgagccgt tattagatgg tccataccaa ccaaccactt tcaatccacc aacaagctat 240

tgggtactac ttgcgccaac tgtagagggc gtaattattc aaggaacaaa caataccgat 300

agatggttgg ccactatact aattgaacca aacgtacaaa caactaacag aatatacaat 360

ctttttggtc agcaagtaac tttatcggtg gagaatacgt cacagacaca ttggaagttc 420

attgatgtga gtacaactac gccaacagga agttatacgc agcacggacc attgttctct 480

acaccaaaat tatacgctgt aatgaaattc agtggtagaa tatatacata taatggaacc 540

acaccaaacg caacaacagg atactattca actactaatt atgacacagt aaatatgaca 600

tcattttgtg atttttatat tataccaaga aatcaagaag aaaaatgtac tgagtatatc 660

aatcatggat tacctcctat acaaaataca aggaatgttg tgccagtatc tttatcggct 720

agagagatag tgcacacaag agctcaagtt aatgaggata ttgttgtttc aaaaacttca 780

ctttggaaag aaatgcaatg caacagagac ataaccataa gattcaaatt tgatagaaca 840

attattaaag ctggaggatt aggatacaaa tggtcagaaa tatcttttaa gccaattact 900

tatcagtaca catacgctag agatggagaa caaattacag cgcacaccac atgctcagtt 960

aatggagtta acaattttag ttacaatggc ggttcgctgc cgacggactt cgccatatca 1020

agatatgaag tgattaaaga aaattcattt gtttacattg attactggga tgattcacaa 1080

gcattcagaa acatggtata tgttcggtca cttgctgcta atttgaatac agtaacgtgt 1140

actggtggta gttacagctt cgcgttgcct ttaggtcatt atccagttat gactggtggt 1200

acagtttcac tacacccagc tggagttaca ttatctaccc aattcactga tttcgtatct 1260

cttaattcat tacgctttag attcaggtta actgtaggag aaccttcatt ttctataacg 1320

aggactaggg taagtaggtt atacggactt ccagcagcta atccaaacaa ccaaagagaa 1380

tattatgaaa taagcggtag gttttcatta atatcattag tgccatcaaa tgatgattat 1440

caaacaccaa ttatgaattc agttactgtg aggcaagatc tagagagaca attgggagag 1500

ctacgagatg agttcaattc attgtcacag caaatagcga tatcacaatt gattgatctg 1560

gctctattgc cattagatat gttttcaatg ttctcaggaa ttaaaagtac aatagatgct 1620

gcgaaatcca tggcaacgaa cgtgatgaaa agatttaaac ggtcaaacct agccagctca 1680

gtttctacat taactgatgc aatgtctgat gcagcatcgt ctatttcaag aagttcatca 1740

atacgatcaa taggatcatc agcatccgct tggacggaag tgtcaaattc gatcgcagat 1800

gtttctacga cagttgatac agtatcaacg caaactgcta ctattgctaa gcggttacga 1860

ttgaaagaga ttgcaactca aaccgatggt atgaattttg atgacatatc tgcagcagtg 1920

ttaaaaacga aaattgacaa atccgtgcaa ataacaccaa atacattacc ggaaatagtt 1980

actgaagctt ctgagaaatt tatcccaaat agaacatata gggttattaa caatgatgaa 2040

gtatttgaag ttggaatgga tggtaagttt ttcgcatatc gtgtagacac attcgatgaa 2100

ataccatttg atgtacagaa attcgcagat ttagttacag attcaccagt catttctgca 2160

ataattgatc ttaagacgtt aaaaaatctg aaagataatt atggaataag taagcagcaa 2220

gcttttgact tactacgatc agatccaagg gtattacgtg aatttatcaa tcagaataat 2280

ccaataatac gaaatagaat tgaaaactta atcatgcaat gtagactgta a 2331

<210> 9
<211> 1194
<212> DNA
<213> Reoviridae

<400> 9
atggaggttc tgtactcatt gtcaaaaacc ttgaaagatg ctagagataa aattgttgaa 60

ggtacattat actcaaatat aagtgattta attcaacaat tcaatcaaat gatagttacc 120

atgaatggaa atgattttca aacgggagga ataggaaatt tgccaatcag aaactggaat 180

tttgatttcg gattacttgg cactacttta cttaatatag atgcaaatta tgttgaaaat 240

gctagaacta ccattgaata tttcattgat tttatagata atgtgtgtat ggatgaaatg 300

gctagagagt cgcaacgaaa cggaatagct ccacaatctg aagcactgag aaagctgtca 360

ggtattaagt ttaagagaat taattttgac aattcatctg attacattga gaattggaat 420

ttacaaaata gacgacagcg tactggattc gtgttccata agccaaatat acttccatac 480

tcagcatcat tcactttgaa tagatcacag ccggcacatg ataatttaat ggggactatg 540

tggattaacg ctggatcaga aattcaggtg gctggatttg attattcgtg tgcttttaat 600

gcaccggcaa atattcagca gtttgaacat gtcgtgccat taagacgtgc acttacgaca 660

gctacaatta ctttgctacc agatgctgag aggttcagtt tcccaagagt gattaattca 720

gctgatggcg ctactacatg gtacttcaat ccagttatca taagaccaag taatgttgaa 780

gttgaatttt tgttgaatgg gcaaataatt aatacgtacc aagcgcgatt tggaaccatc 840

atagctagaa attttgatac tattcggtta tcatttcaat tagtacgacc accgaatatg 900

acaccagcag ttgcaaacct atttccgcaa gcaccaccat ttatatttca tgctacagtt 960

ggactcacac tacgaattga atctgcagtt tgtgaatctg tgcttgcgga cgcttccgaa 1020

actttattgg caaatgtgac tgcagttcgt caagagtatg ctataccggt agggccagtg 1080

ttcccaccag gtatgaattg gacagaattg attaccaatt attcaccttc aagagaagat 1140

aacttgcaac gtgttttcac agtagcttcc attagaagca tgttgattaa gtga 1194

<210> 10
<211> 981
<212> DNA
<213> Reoviridae

<400> 10
atgtatggta ttgaatatac cacagttcta acttttttga tatcgcttgt atttgtcaat 60

tatatactga aatcagttac tagaacaatg gactttatca tttatagatt cttattggtt 120

atagtcgtac ttgcaccgct cattaaagct caaaattacg gaattaattt accaataact 180

ggatctatgg atacgccata tatgaattca actacaagtg aaacattttt gacttcgaca 240

ttatgtctat attatccaaa tgaagcagct acagaaattg cagatacaaa atggacagaa 300

acattgtcgc agttgttttt aacaaaagga tggccaacag ggtcagttta ttttaaagga 360

tatgcagata ttgcgtcatt ttctgtagaa ccgcagttat actgcgacta taatattgta 420

ctaatgaaat atgatggaaa tttacagtta gacatgtctg aattggctga tttaatattg 480

aatgaatggc tatgtaatcc aatggatata atgctatatt attatcagca aacagatgaa 540

gctaataaat ggatatcaat gggtacatca tgtacgatta aagtatgtcc tctaaatacg 600

cagactctcg ggataggatg ttcgactaca gacataaatt catttgaaac agtggccaat 660

gcagagaaat tagctataac tgatgttgtc gatggagtca atcataaatt agacgtaaca 720

acgagtacat gtactataag aaattgtaaa aaacttggac caagagaaaa tgtcgctgta 780

attcaggtag gaggtccaaa catactcgac ataacagctg atccaacaac tgcaccacaa 840

actgaaagaa tgatgcgtat aaattggaag agatggtggc aagtctttta tacaatagtt 900

gattatgtca atcaaattgt acaagtcatg tccaagcgat cacgctcctt agattctgct 960

gcctttatt accgagtcta g 981

<210> 11
<211> 954
<212> DNA
<213> Reoviridae

<400> 11
   atggctgagc tagcttgctt ttgttatcct catttggaga acgatagcta taaatttatt 60
   ccttttaata atttagcaat aaaatgcatg ttgacagcta aagtggataa aaaagatcaa 120
   gataaatttt acaattctat tgtttatgga attgcgccac caccacaatt taaaaaacgt 180
   tataatacta gtgataattc aaggggcatg aattatgtaa cgactatgtt taataaggtg 240
   gccatcttaa tttgtgaagc gctcaattca attaaagtta cccaatctga cgttgctaat 300
   gttctttcaa gagtggtttc cgttagacat tttggaaatt tggtgttaag aaaagaaaac 360
   catcaagatg tgctgtttca ttcgaaagaa ctactcttaa aatctgtttt gatagctatt 420
   ggtcaatcaa aagaaattga aactactgcc actgctgaag gaggagaaat agtatttcag 480
   aatgcagctt ttactatgtg gaaattgacg tatttagatc ataaattgat gcctattttg 540
   gatcagaact tcactgaata taaaattaca ttgaatgaag ataaaccaat ttcagatgta 600
   tgtgttaaag aacttgtcgc tgaattgaga tggcagtaca acagatttgc tgtaataaca 660
   catggtaaag gtcactatag agttgttaaa tattcatcag ttgctaatca tgcagacaga 720
   gtgtttgcta catataaaaa taatgctaag agtggcaatg ttactgactt taacttgttg 780
   gaccaaagaa ttatttggca aaattggtat gcttttacat cctcaatgaa acaaggtaat 840
   acacttgatg tatgtaagaa actgctcttc caaaagatga aacaggagaa aaatccgttc 900
   aaaggactgt caactgatag aaaaatggat gaagtttcac atgttggaat ttaa 954

<210> 12
<211> 528
<212> DNA
<213> Reoviridae

<400> 12
atggataagc ttgccgacct caattacaca ttgagcgtaa tcactttaat gaatgacaca 60

ctacactcta ttattcaaga tccaggaatg gcgtattttc catatattgc atctgttctg 120

actgttttat ttactctaca taaagcatca attccaacaa tgaaaatagc gttaaaaacg 180

tcaaagtgtt cgtacaaagt aattaaatat tgcatggtta caatcattaa tactcttctg 240

aagttggctg gttataaaga acaggttact actaaggatg aaattgaaca acagatggac 300

agaattatta aagagatgag acgtcaactg gaaatgattg acaaattgac aactcgtgaa 360

attgaacagg ttgaattact taaacgtata catgacaaat tagctgctag atcagttgac 420

gctatagata tgtcgaaaga atttaatcag aaaaatattc gaacgctaga tgaatgggaa 480

agtggaaaaa atccatatga accgtcggaa gtaactgcgt ctatgtga 528

<210> 13
<211> 2673
<212> DNA
<213> Artificial Sequence

<220>
<223> PRVA VP2 optimized

<400> 13
atggcataca gaaagagagg tgcaaaaaga gaaaactcat cacaacaaaa cgaaagatta 60

caagaaaagg aagtagaaaa gaacattgat gcatctatgg aaaataaggc taacaataag 120

aaacaacaat tgccagataa ggttttgtca caaaaggaag aaatcatcac tgatgctcaa 180

gatgatgtta agatcacaga tgaagttaag aaatcttcaa aagaagaatc taagcaattg 240

ttggaaattt tgaagactaa agaagatcat caaaaagaaa ttcaatacga aattttacaa 300

aagactatcc caacattcga accaaaggaa tcaatcttga agaaattgga agatatcaag 360

ccagaacaag ctaagaaaca aactaagttg tttagaatct tcgaaccaag acaattgcca 420

atctatagag ctaatggtga aagagaattg agaaacagat ggtactggaa gttgaagaga 480

gatacattgc cagatggtga ctatgatgtt agagaatact ttttgaattt gtacgatcaa 540

attttaatcg aaatgccaga ttatttgttg ttgaaggata tggcagttga aaataagaac 600

tctagagatg ctggtaaagt tgttgattct gaaactgcat caatctgtga tgctatcttc 660

caagatgaag aaacagaagg tgttattaga agattcattg cagatatgag acaacaagtt 720

caagctgata gaaacgttgt taactaccca tctatcttgc atccaatcga tcatgctttt 780

aatgaatact ttttgaacca tcaattggtt gaaccattga acaacgaaat cattttcaat 840

tacattccag aaagaattag aaatgatgtt aattatattt tgaatatgga tatgaatttg 900

ccatctactg ctagatacat tagaccaaat ttgttgcaag atagattgaa tttgcatgat 960

aacttcgaat cattgtggga tactatcact acatctaact acatcttggc aagatcagtt 1020

gttccagatt tgaaggaaaa ggaattagtt tctacagaag ctcaaatcca aaagatgtca 1080

caagatttgc aattggaagc attgactatc caatctgaaa cacaattctt ggctggtatt 1140

aattcacaag ctgcaaacga ttgtttcaag actttgatcg ctgcaatgtt gtctcaaaga 1200

acaatgtcat tggaattcgt tactacaaac tacatgtctt tgatttcagg catgtggttg 1260

ttgactgtta ttccaaacga tatgttcttg agagaatctt tggttgcttg tgaattggca 1320

attattaaca ctatcgttta cccagctttc ggtatgcaaa gaatgcatta cagaaatggt 1380

gacccacaaa caccatttca aattgcagaa caacaaatcc aaaacttcca agttgctaac 1440

tggttgcatt tcatcaacaa caacagattc agacaagttg ttattgatgg tgttttgaac 1500

caaactttga acgataacat cagaaatggt caagttatta atcaattgat ggaagctttg 1560

atgcaattgt ctagacaaca attcccaaca atgccagttg attacaagag atcaatccaa 1620

agaggtatct tattgttgtc aaacagattg ggtcaattgg ttgatttgac tagattgttg 1680

tcatacaact acgaaacttt gatggcttgt atcacaatga acatgcaaca tgttcaaaca 1740

ttgactacag aaaagttgca attgacttct gttacatcat tgtgtatgtt gatcggtaac 1800

actacagtta ttccatctcc acaaactttg ttccattact acaacgttaa cgttaacttc 1860

cattcaaatt acaatgaaag aattaatgat gctgttgcaa tcatcacagc tgcaaacaga 1920

ttgaatttgt accaaaagaa aatgaaatct attgttgaag aatttttaaa aagattgcaa 1980

atttttgatg ttccaagagt tccagatgat caaatgtaca gattgagaga tagattgaga 2040

ttgttgccag ttgaaagaag aagattggat atttttaatt tgatcttgat gaatatggaa 2100

caaatcgaaa gagcatctga taagatcgct caaggtgtta ttatcgcata cagagatatg 2160

caattggaaa gagatgaaat gtacggttac gttaacatcg ctagaaattt ggatggttac 2220

caacaaatta atttggaaga attgatgaga actggtgact atggtcaaat cacaaacatg 2280

ttgttgaaca accaaccagt tgctttggtt ggtgcattgc cattcgttac tgattcttca 2340

gttatttctt tgatcgctaa gttggatgca actgttttcg ctcaaatcgt taagttgaga 2400

aaagttgata cattgaaacc aatcttgtac aagattaatt ctgattcaaa tgatttttac 2460

ttagttgcaa attatgattg gatcccaaca tcaactacaa aggtttacaa gcaagttcca 2520

caaccattcg atttcagagc ttctatgcat atgttgactt caaatttgac ttttacagtt 2580

tactctgatt tgttggcatt tgtttcagct gatacagttg aaccaattaa tgctgttgca 2640

ttcgataaca tgagaatcat gaacgaattg tga 2673

<210> 14
<211> 2331
<212> DNA
<213> Artificial Sequence

<220>
<223> PRVA VP4 optimized

<400> 14
atggcttctt tgatctatag acaattgttg actaactcat acacagttaa tttgtctgat 60

gaaatccaag aaatcggttc tgctaagtct caagatgtta ctattaatcc aggtccattt 120

gctcaaacag gttacgcacc agttaattgg ggtgcaggtg aaactaatga ttctactaca 180

gttgaaccat tgttagatgg tccataccaa ccaactactt ttaatccacc aacttcttat 240

tgggttttgt tagctccaac agttgaaggt gttattatcc aaggtacaaa caacactgat 300

agatggttgg caactatctt gatcgaacca aacgttcaaa ctacaaacag aatctataat 360

ttgtttggtc aacaagttac attgtcagtt gaaaacacat ctcaaactca ttggaagttt 420

attgatgttt caactacaac tccaactggt tcttacacac aacatggtcc attattttca 480

actccaaagt tgtacgctgt tatgaaattt tctggtagaa tctatacata caatggtaca 540

actccaaatg caacaactgg ttactactca acaactaact acgatactgt taacatgaca 600

tctttctgtg atttctacat tattccaaga aaccaagaag aaaagtgtac tgaatacatc 660

aaccatggtt taccaccaat ccaaaacaca agaaacgttg ttccagtttc attgtctgct 720

agagaaattg ttcatactag agcacaagtt aacgaagata tcgttgtttc aaagacatct 780

ttatggaaag aaatgcaatg taacagagat atcactatca gattcaaatt cgatagaaca 840

attattaagg ctggtggttt gggttataaa tggtcagaaa tctcttttaa accaatcact 900

taccaataca catacgctag agatggtgaa caaattactg cacatacaac ttgttctgtt 960

aacggtgtta acaacttctc atacaacggt ggttctttgc caacagattt cgctatctca 1020

agatacgaag ttattaaaga aaattctttt gtttacattg attattggga tgattcacaa 1080

gcttttagaa acatggttta cgtcagatct ttggctgcaa atttgaatac agttacttgt 1140

acaggtggtt catactcttt tgctttgcca ttaggtcatt atccagttat gacaggtggt 1200

actgtttcat tacatccagc aggtgttaca ttgtctactc aattcactga tttcgtttca 1260

ttgaactctt tgagattcag attcagatta actgttggtg aaccatcatt ttctatcact 1320

agaacaagag tttctagatt gtacggttta ccagctgcaa atccaaacaa ccaaagagaa 1380

tactacgaaa tctcaggtag attttcattg atctctttgg ttccatctaa cgatgattac 1440

caaactccaa tcatgaactc agttacagtt agacaagatt tggaaagaca attgggtgaa 1500

ttgagagatg aattcaattc attgtctcaa caaatcgcta tctctcaatt gatcgatttg 1560

gcattgttgc cattggatat gttctcaatg ttctctggta ttaaatcaac tatcgatgct 1620

gcaaagtcta tggctacaaa cgttatgaag agattcaaaa gatcaaattt ggcttcttca 1680

gtttctactt tgacagatgc aatgtcagat gctgcatctt caatctctag atcttcatct 1740

atcagatcaa ttggttcatc tgcttctgca tggactgaag tttcaaattc tatcgctgat 1800

gtttcaacaa ctgttgatac agtttctact caaacagcta ctatcgcaaa gagattgaga 1860

ttgaaggaaa tcgcaacaca aactgatggt atgaacttcg atgatatctc tgctgcagtt 1920

ttgaagacta agatcgataa gtcagttcaa attactccaa atacattgcc agaaatcgtt 1980

actgaagctt ctgaaaagtt tattccaaac agaacataca gagttattaa taacgatgaa 2040

gttttcgaag ttggtatgga tggtaaattt ttcgcttaca gagttgatac tttcgatgaa 2100

atcccattcg atgttcaaaa gttcgctgat ttggttactg attcaccagt tatttctgca 2160

atcatcgatt tgaagacatt gaaaaatttg aaagataatt atggtatctc aaagcaacaa 2220

gctttcgatt tgttaagatc tgatccaaga gttttgagag aattcattaa ccaaaacaac 2280

ccaatcatca gaaatagaat tgaaaatttg attatgcaat gtagattgta a 2331

<210> 15
<211> 1194
<212> DNA
<213> Artificial Sequence

<220>
<223> PRVA VP6 optimized

<400> 15
atggaagttt tgtactcttt gtccaagact ttgaaggatg ccagagataa gatcgttgaa 60

ggtactttgt actctaacat ttctgatttg atccaacaat tcaaccaaat gattgttacc 120

atgaacggta acgacttcca aactggtggt atcggtaact tgccaattag aaactggaac 180

ttcgacttcg gtttgttggg taccactttg ttgaacatcg atgctaacta cgttgaaaac 240

gctagaacca ctatcgaata cttcatcgac ttcatcgata acgtttgtat ggatgaaatg 300

gctagagaat ctcaaagaaa cggtatcgct ccacaatctg aagccttgag aaagttgtcc 360

ggtatcaagt tcaagagaat taacttcgac aactcttccg attacatcga aaactggaac 420

ttgcaaaaca gaagacaaag aaccggtttc gttttccaca agccaaacat tttgccatac 480

tctgcttcct tcaccttgaa cagatcccaa ccagctcacg acaacttgat gggtactatg 540

tggatcaacg ctggttctga aattcaagtt gctggtttcg attactcctg tgctttcaac 600

gctccagcta acatccaaca attcgaacac gttgttccat tgagaagagc tttgaccact 660

gctaccatca ctttgttgcc agacgctgaa agattctctt tcccaagagt tattaactcc 720

gctgatggtg ctaccacttg gtacttcaac ccagttatca ttagaccatc taacgttgaa 780

gttgaatttt tgttgaacgg tcaaatcatt aacacctacc aagctagatt cggtactatc 840

attgctagaa acttcgacac catcagattg tccttccaat tggttagacc accaaacatg 900

actccagctg ttgctaactt gttcccacaa gctccaccat tcatcttcca cgctaccgtt 960

ggtttgactt tgagaattga atctgctgtt tgtgaatccg ttttggctga tgcttctgaa 1020

accttgttgg ctaacgttac tgctgttaga caagaatacg ctatcccagt tggtccagtt 1080

ttcccaccag gtatgaactg gaccgaattg attactaact actctccatc cagagaagac 1140

aacttgcaaa gagttttcac cgttgcttct atcagatcca tgttgatcaa gtaa 1194

<210> 16
<211> 981
<212> DNA

<213> Artificial Sequence

<220>
<223> PRVA VP7 optimized

<400> 16
atgtacggta tcgaatacac tacagttttg acatttttga tttctttggt tttcgttaac 60

tacatcttga agtcagttac tagaacaatg gattttatta tctatagatt tttgttagtt 120

attgttgttt tggctccatt gattaaagca caaaactacg gtattaattt gccaatcact 180

ggttctatgg atacaccata catgaactct actacatcag aaacattttt gacttcaaca 240

ttgtgtttgt actacccaaa cgaagctgca actgaaattg ctgatacaaa gtggactgaa 300

acattgtctc aattgttttt aactaaaggt tggccaacag gttcagttta cttcaagggt 360

tacgctgata tcgcatcttt ttcagttgaa ccacaattgt actgtgatta caacatcgtt 420

ttgatgaaat atgatggtaa tttgcaattg gatatgtctg aattggctga tttgatcttg 480

aacgaatggt tgtgtaaccc aatggatatc atgttgtact actaccaaca aactgatgaa 540

gcaaataagt ggatctctat gggtacttca tgtacaatta aagtttgtcc attgaatact 600

caaacattgg gtattggttg ttctactaca gatatcaact cattcgaaac tgttgctaac 660

gcagaaaagt tggctatcac agatgttgtt gatggtgtta accataagtt ggatgttact 720

acatcaactt gtacaatcag aaactgtaag aaattgggtc caagagaaaa tgttgcagtt 780

attcaagttg gtggtccaaa tattttggat attactgctg atccaactac agcaccacaa 840

acagaaagaa tgatgagaat taattggaag agatggtggc aagttttcta tactatcgtt 900

gattacgtta accaaatcgt tcaagttatg tctaagagat ctagatcatt ggattcagct 960

gcattttact atagagttta a 981

<210> 17
<211> 954
<212> DNA
<213> Artificial Sequence

<220>
<223> PRVA NSP2 optimized

<400> 17
atggccgaat tagcatgttt ctgttacccc cacttagaga atgatagtta caagtttata 60

cccttcaaca atctggcaat taagtgtatg cttacagcca aagtggacaa gaaggatcaa 120

gataagttct ataatagcat agtctatggt atagcccctc ctcctcagtt caaaaagagg 180

tataatactt cggataactc aagaggcatg aactatgtaa ccaccatgtt caacaaggtg 240

gccattttga tttgcgaggc tctaaactca attaaagtga cacagagcga tgttgcaaat 300

gtgttatcaa gggtagtctc agtgaggcac cttgaaaacc tggtgttgag gaaagagaac 360

catcaggatg tactgttcca ctcaaaagaa ctactactta agtctgtttt gatagcaata 420

ggacaatcga aagaaataga aacaactgct acggccgagg gcggtgagat cgtgtttcag 480

aatgctgctt tcacaatgtg gaagctaaca tacctagacc ataaactgat gcctatttta 540

gatcagaatt ttaccgagta taagattact cttaacgagg ataagccaat cagtgatgta 600

tgtgtaaagg agttggttgc tgagcttcgt tggcaatata acagattcgc cgttataacc 660

cacggcaaag gacattacag agtcgtgaag tatagctcag tcgctaacca tgccgacagg 720

gtatttgcca cctataagaa taacgcaaaa tctggcaatg ttacagattt caacttgcta 780

gaccagagga taatatggca aaattggtac gcttttacgt cctccatgaa acagggaaat 840

acgctagatg tctgcaagaa attgcttttc cagaaaatga agcaggagaa gaaccccttt 900

aagggactat cgaccgatag aaaaatggac gaggtctccc acgtaggcat ttga 954

<210> 18
<211> 528
<212> DNA
<213> Artificial Sequence

<220>
<223> PRVA NSP4 optimized

<400> 18
atggataaac ttgctgacct gaattatacc ttgtcggtta taacgttaat gaatgacacc 60

ttgcattcta ttatccaaga ccccggtatg gcctacttcc cttacattgc atcagtgtta 120

accgtgctat tcacgttgca caaagcatcc atacccacga tgaaaatagc attaaagacc 180

tccaagtgtt cgtataaagt cattaagtac tgtatggtca ctatcatcaa tactcttcta 240

aaattggctg gatacaaaga gcaggtcacc acgaaagatg aaatagaaca gcagatggat 300

aggattatca aagaaatgcg taggcagtta gaaatgatcg acaagttaac aacgcgtgaa 360

atcgaacagg tagaattgct aaagagaata catgacaagt tagccgctag gtccgttgac 420

gcaatagata tgtcgaagga gttcaatcaa aagaacatca ggactctgga cgagtgggaa 480

agcggaaaga acccctacga gccaagtgag gtaactgcct cgatgtag 528

<210> 19
<211> 934
<212> PRT
<213> Artificial Sequence

<220>
<223> PRVA VP2 modified

<400> 19
Met Ala Tyr Arg Lys Arg Gly Ala Lys Arg Glu Asn Ser Ser Gln Gln
1               5                   10                  15

Asn Glu Arg Leu Gln Glu Lys Glu Val Glu Lys Asn Ile Asp Ala Ser
                20                  25                  30
Met Glu Asn Lys Ala Asn Asn Lys Lys Gln Gln Leu Pro Asp Lys Val
            35                  40                  45
Leu Ser Gln Lys Glu Glu Ile Ile Thr Asp Ala Gln Asp Asp Val Lys
    50                  55                  60
Ile Thr Asp Glu Val Lys Lys Ser Ser Lys Glu Glu Ser Lys Gln Leu
65                  70                  75                  80
Leu Glu Ile Leu Lys Thr Lys Glu Asp His Gln Lys Glu Ile Gln Tyr
                85                  90                  95
Glu Ile Leu Gln Lys Thr Ile Pro Thr Phe Glu Pro Lys Glu Ser Ile
            100                 105                 110
Leu Lys Lys Leu Glu Asp Ile Lys Pro Glu Gln Ala Lys Lys Gln Thr
        115                 120                 125
Lys Leu Phe Arg Ile Phe Glu Pro Arg Gln Leu Pro Ile Tyr Arg Ala
    130                 135                 140
Asn Gly Glu Arg Glu Leu Arg Asn Arg Trp Tyr Trp Lys Leu Lys Arg
145                 150                 155                 160
Asp Thr Leu Pro Asp Gly Asp Tyr Asp Val Arg Glu Tyr Phe Leu Asn
                165                 170                 175
Leu Tyr Asp Gln Ile Leu Ile Glu Met Pro Asp Tyr Leu Leu Leu Lys
            180                 185                 190
Asp Met Ala Val Glu Asn Lys Asn Ser Arg Asp Ala Gly Lys Val Val
        195                 200                 205
Asp Ser Glu Thr Ala Ser Ile Cys Asp Ala Ile Phe Gln Asp Glu Glu
    210                 215                 220
Thr Glu Gly Val Ile Arg Arg Phe Ile Ala Asp Met Arg Gln Gln Val
225                 230                 235                 240
Gln Ala Asp Arg Asn Val Val Asn Tyr Pro Ser Ile Leu His Pro Ile
                245                 250                 255
Asp His Ala Phe Asn Glu Tyr Phe Leu Asn His Gln Leu Val Glu Pro
            260                 265                 270
Leu Asn Asn Glu Ile Ile Phe Asn Tyr Ile Pro Glu Arg Ile Arg Asn
        275                 280                 285
Asp Val Asn Tyr Ile Leu Asn Met Asp Met Asn Leu Pro Ser Thr Ala
    290                 295                 300
Arg Tyr Ile Arg Pro Asn Leu Leu Gln Asp Arg Leu Asn Leu His Asp
305                 310                 315                 320
Asn Phe Glu Ser Leu Trp Asp Thr Ile Thr Thr Ser Asn Tyr Ile Leu
                325                 330                 335
Ala Arg Ser Val Val Pro Asp Leu Lys Glu Lys Glu Leu Val Ser Thr
            340                 345                 350
Glu Ala Gln Ile Gln Lys Met Ser Gln Asp Leu Gln Leu Glu Ala Leu
        355                 360                 365
Thr Ile Gln Ser Glu Thr Gln Phe Leu Ala Gly Ile Asn Ser Gln Ala
    370                 375                 380
Ala Asn Asp Cys Phe Lys Thr Leu Ile Ala Ala Met Leu Ser Gln Arg
385                 390                 395                 400
Thr Met Ser Leu Glu Phe Val Thr Thr Asn Tyr Met Ser Leu Ile Ser
                405                 410                 415
Gly Met Trp Leu Leu Thr Val Ile Pro Asn Asp Met Phe Leu Arg Glu
            420                 425                 430
Ser Leu Val Ala Cys Glu Leu Ala Ile Ile Asn Thr Ile Val Tyr Pro
        435                 440                 445
Ala Phe Gly Met Gln Arg Met His Tyr Arg Asn Gly Asp Pro Gln Thr
    450                 455                 460
Pro Phe Gln Ile Ala Glu Gln Gln Ile Gln Asn Phe Gln Val Ala Asn
465                 470                 475                 480
Trp Leu His Phe Ile Asn Asn Asn Arg Phe Arg Gln Val Val Ile Asp
                485                 490                 495
Gly Val Leu Asn Gln Thr Leu Asn Asp Asn Ile Arg Asn Gly Gln Val
            500                 505                 510
Ile Asn Gln Leu Met Glu Ala Leu Met Gln Leu Ser Arg Gln Gln Phe

515                 520                 525
Pro Thr Met Pro Val Asp Tyr Lys Arg Ser Ile Gln Arg Gly Ile Leu
    530                 535                 540
Leu Leu Ser Asn Arg Leu Gly Gln Leu Val Asp Leu Thr Arg Leu Leu
545                 550                 555                 560
Ser Tyr Asn Tyr Glu Thr Leu Met Ala Cys Ile Thr Met Asn Met Gln
                565                 570                 575
His Val Gln Thr Leu Thr Thr Glu Lys Leu Gln Leu Thr Ser Val Thr
            580                 585                 590
Ser Leu Cys Met Leu Ile Gly Asn Thr Thr Val Ile Pro Ser Pro Gln
        595                 600                 605
Thr Leu Phe His Tyr Tyr Asn Val Asn Val Asn Phe His Ser Asn Tyr
    610                 615                 620
Asn Glu Arg Ile Asn Asp Ala Val Ala Ile Ile Thr Ala Ala Asn Arg
625                 630                 635                 640
Leu Asn Leu Tyr Gln Lys Lys Met Lys Ser Ile Val Glu Glu Phe Leu
                645                 650                 655
Lys Arg Leu Gln Ile Phe Asp Val Pro Arg Val Pro Asp Asp Gln Met
            660                 665                 670
Tyr Arg Leu Arg Asp Arg Leu Arg Leu Leu Pro Val Glu Arg Arg Arg
        675                 680                 685
Leu Asp Ile Phe Asn Leu Ile Leu Met Asn Met Glu Gln Ile Glu Arg
    690                 695                 700
Ala Ser Asp Lys Ile Ala Gln Gly Val Ile Ile Ala Tyr Arg Asp Met
705                 710                 715                 720
Gln Leu Glu Arg Asp Glu Met Tyr Gly Tyr Val Asn Ile Ala Arg Asn
                725                 730                 735
Leu Asp Gly Tyr Gln Gln Ile Asn Leu Glu Glu Leu Met Arg Thr Gly
            740                 745                 750
Asp Tyr Gly Gln Ile Thr Asn Met Leu Leu Asn Asn Gln Pro Val Ala
        755                 760                 765
Leu Val Gly Ala Leu Pro Phe Val Thr Asp Ser Ser Val Ile Ser Leu
    770                 775                 780
Ile Ala Lys Leu Asp Ala Thr Val Phe Ala Gln Ile Val Lys Leu Arg
785                 790                 795                 800
Lys Val Asp Thr Leu Lys Pro Ile Leu Tyr Lys Ile Asn Ser Asp Ser
                805                 810                 815
Asn Asp Phe Tyr Leu Val Ala Asn Tyr Asp Trp Ile Pro Thr Ser Thr
            820                 825                 830
Thr Lys Val Tyr Lys Gln Val Pro Gln Pro Phe Asp Phe Arg Ala Ser
        835                 840                 845
Met His Met Leu Thr Ser Asn Leu Thr Phe Thr Val Tyr Ser Asp Leu
    850                 855                 860
Leu Ala Phe Val Ser Ala Asp Thr Val Glu Pro Ile Asn Ala Val Ala
865                 870                 875                 880
Phe Asp Asn Met Arg Ile Met Asn Glu Leu Gly Gly Gly Gly Ser Gly
                885                 890                 895
Gly Gly Gly Ser Gly Tyr Pro Tyr Asp Val Pro Asp Tyr Ala Gly Tyr
            900                 905                 910
Pro Tyr Asp Val Pro Asp Tyr Ala Gly Ser Tyr Pro Tyr Asp Val Pro
        915                 920                 925
Asp Tyr Ala Ala Gln Lys
    930

<210> 20
<211> 820
<212> PRT
<213> Artificial Sequence

<220>
<223> PRVA VP4 modified

<400> 20
Met Ala Ser Leu Ile Tyr Arg Gln Leu Leu Thr Asn Ser Tyr Thr Val
1               5                   10                  15
Asn Leu Ser Asp Glu Ile Gln Glu Ile Gly Ser Ala Lys Ser Gln Asp
            20                  25                  30
Val Thr Ile Asn Pro Gly Pro Phe Ala Gln Thr Gly Tyr Ala Pro Val
        35                  40                  45
Asn Trp Gly Ala Gly Glu Thr Asn Asp Ser Thr Thr Val Glu Pro Leu
    50                  55                  60
Leu Asp Gly Pro Tyr Gln Pro Thr Thr Phe Asn Pro Pro Thr Ser Tyr
65                  70                  75                  80
Trp Val Leu Leu Ala Pro Thr Val Glu Gly Val Ile Ile Gln Gly Thr
                85                  90                  95
Asn Asn Thr Asp Arg Trp Leu Ala Thr Ile Leu Ile Glu Pro Asn Val
            100                 105                 110
Gln Thr Thr Asn Arg Ile Tyr Asn Leu Phe Gly Gln Gln Val Thr Leu
        115                 120                 125
Ser Val Glu Asn Thr Ser Gln Thr His Trp Lys Phe Ile Asp Val Ser
    130                 135                 140
Thr Thr Thr Pro Thr Gly Ser Tyr Thr Gln His Gly Pro Leu Phe Ser
145                 150                 155                 160
Thr Pro Lys Leu Tyr Ala Val Met Lys Phe Ser Gly Arg Ile Tyr Thr
                165                 170                 175
Tyr Asn Gly Thr Thr Pro Asn Ala Thr Thr Gly Tyr Tyr Ser Thr Thr
            180                 185                 190
Asn Tyr Asp Thr Val Asn Met Thr Ser Phe Cys Asp Phe Tyr Ile Ile
        195                 200                 205
Pro Arg Asn Gln Glu Glu Lys Cys Thr Glu Tyr Ile Asn His Gly Leu
    210                 215                 220
Pro Pro Ile Gln Asn Thr Arg Asn Val Val Pro Val Ser Leu Ser Ala
225                 230                 235                 240
Arg Glu Ile Val His Thr Arg Ala Gln Val Asn Glu Asp Ile Val Val
                245                 250                 255
Ser Lys Thr Ser Leu Trp Lys Glu Met Gln Cys Asn Arg Asp Ile Thr
            260                 265                 270
Ile Arg Phe Lys Phe Asp Arg Thr Ile Ile Lys Ala Gly Gly Leu Gly
        275                 280                 285
Tyr Lys Trp Ser Glu Ile Ser Phe Lys Pro Ile Thr Tyr Gln Tyr Thr
    290                 295                 300
Tyr Ala Arg Asp Gly Glu Gln Ile Thr Ala His Thr Thr Cys Ser Val
305                 310                 315                 320
Asn Gly Val Asn Asn Phe Ser Tyr Asn Gly Gly Ser Leu Pro Thr Asp
                325                 330                 335
Phe Ala Ile Ser Arg Tyr Glu Val Ile Lys Glu Asn Ser Phe Val Tyr
            340                 345                 350
Ile Asp Tyr Trp Asp Asp Ser Gln Ala Phe Arg Asn Met Val Tyr Val
        355                 360                 365
Arg Ser Leu Ala Ala Asn Leu Asn Thr Val Thr Cys Thr Gly Gly Ser
    370                 375                 380
Tyr Ser Phe Ala Leu Pro Leu Gly His Tyr Pro Val Met Thr Gly Gly
385                 390                 395                 400
Thr Val Ser Leu His Pro Ala Gly Val Thr Leu Ser Thr Gln Phe Thr
                405                 410                 415
Asp Phe Val Ser Leu Asn Ser Leu Arg Phe Arg Phe Arg Leu Thr Val
            420                 425                 430
Gly Glu Pro Ser Phe Ser Ile Thr Arg Thr Arg Val Ser Arg Leu Tyr
        435                 440                 445
Gly Leu Pro Ala Ala Asn Pro Asn Asn Gln Arg Glu Tyr Tyr Glu Ile
    450                 455                 460
Ser Gly Arg Phe Ser Leu Ile Ser Leu Val Pro Ser Asn Asp Asp Tyr
465                 470                 475                 480
Gln Thr Pro Ile Met Asn Ser Val Thr Val Arg Gln Asp Leu Glu Arg
                485                 490                 495

Gln Leu Gly Glu Leu Arg Asp Glu Phe Asn Ser Leu Ser Gln Gln Ile
                500                 505                 510
Ala Ile Ser Gln Leu Ile Asp Leu Ala Leu Leu Pro Leu Asp Met Phe
            515                 520                 525
Ser Met Phe Ser Gly Ile Lys Ser Thr Ile Asp Ala Ala Lys Ser Met
    530                 535                 540
Ala Thr Asn Val Met Lys Arg Phe Lys Arg Ser Asn Leu Ala Ser Ser
545                 550                 555                 560
Val Ser Thr Leu Thr Asp Ala Met Ser Asp Ala Ala Ser Ser Ile Ser
                565                 570                 575
Arg Ser Ser Ser Ile Arg Ser Ile Gly Ser Ser Ala Ser Ala Trp Thr
                580                 585                 590
Glu Val Ser Asn Ser Ile Ala Asp Val Ser Thr Thr Val Asp Thr Val
            595                 600                 605
Ser Thr Gln Thr Ala Thr Ile Ala Lys Arg Leu Arg Leu Lys Glu Ile
    610                 615                 620
Ala Thr Gln Thr Asp Gly Met Asn Phe Asp Asp Ile Ser Ala Ala Val
625                 630                 635                 640
Leu Lys Thr Lys Ile Asp Lys Ser Val Gln Ile Thr Pro Asn Thr Leu
                645                 650                 655
Pro Glu Ile Val Thr Glu Ala Ser Glu Lys Phe Ile Pro Asn Arg Thr
                660                 665                 670
Tyr Arg Val Ile Asn Asn Asp Glu Val Phe Glu Val Gly Met Asp Gly
            675                 680                 685
Lys Phe Phe Ala Tyr Arg Val Asp Thr Phe Asp Glu Ile Pro Phe Asp
    690                 695                 700
Val Gln Lys Phe Ala Asp Leu Val Thr Asp Ser Pro Val Ile Ser Ala
705                 710                 715                 720
Ile Ile Asp Leu Lys Thr Leu Lys Asn Leu Lys Asp Asn Tyr Gly Ile
                725                 730                 735
Ser Lys Gln Gln Ala Phe Asp Leu Leu Arg Ser Asp Pro Arg Val Leu
                740                 745                 750
Arg Glu Phe Ile Asn Gln Asn Asn Pro Ile Ile Arg Asn Arg Ile Glu
            755                 760                 765
Asn Leu Ile Met Gln Cys Arg Leu Gly Gly Gly Gly Ser Gly Gly Gly
    770                 775                 780
Gly Ser Gly Tyr Pro Tyr Asp Val Pro Asp Tyr Ala Gly Tyr Pro Tyr
785                 790                 795                 800
Asp Val Pro Asp Tyr Ala Gly Ser Tyr Pro Tyr Asp Val Pro Asp Tyr
                805                 810                 815
Ala Ala Gln Lys
                820

<210> 21
<211> 441
<212> PRT
<213> Artificial Sequence

<220>
<223> PRVA VP6 modified

<400> 21
Met Glu Val Leu Tyr Ser Leu Ser Lys Thr Leu Lys Asp Ala Arg Asp
1               5                   10                  15
Lys Ile Val Glu Gly Thr Leu Tyr Ser Asn Ile Ser Asp Leu Ile Gln
            20                  25                  30
Gln Phe Asn Gln Met Ile Val Thr Met Asn Gly Asn Asp Phe Gln Thr
        35                  40                  45
Gly Gly Ile Gly Asn Leu Pro Ile Arg Asn Trp Asn Phe Asp Phe Gly
    50                  55                  60
Leu Leu Gly Thr Thr Leu Leu Asn Ile Asp Ala Asn Tyr Val Glu Asn
65                  70                  75                  80

Ala Arg Thr Thr Ile Glu Tyr Phe Ile Asp Phe Ile Asp Asn Val Cys
                85                  90                  95
Met Asp Glu Met Ala Arg Glu Ser Gln Arg Asn Gly Ile Ala Pro Gln
            100                 105                 110
Ser Glu Ala Leu Arg Lys Leu Ser Gly Ile Lys Phe Lys Arg Ile Asn
        115                 120                 125
Phe Asp Asn Ser Ser Asp Tyr Ile Glu Asn Trp Asn Leu Gln Asn Arg
    130                 135                 140
Arg Gln Arg Thr Gly Phe Val Phe His Lys Pro Asn Ile Leu Pro Tyr
145                 150                 155                 160
Ser Ala Ser Phe Thr Leu Asn Arg Ser Gln Pro Ala His Asp Asn Leu
                165                 170                 175
Met Gly Thr Met Trp Ile Asn Ala Gly Ser Glu Ile Gln Val Ala Gly
            180                 185                 190
Phe Asp Tyr Ser Cys Ala Phe Asn Ala Pro Ala Asn Ile Gln Gln Phe
        195                 200                 205
Glu His Val Val Pro Leu Arg Arg Ala Leu Thr Thr Ala Thr Ile Thr
    210                 215                 220
Leu Leu Pro Asp Ala Glu Arg Phe Ser Phe Pro Arg Val Ile Asn Ser
225                 230                 235                 240
Ala Asp Gly Ala Thr Thr Trp Tyr Phe Asn Pro Val Ile Ile Arg Pro
                245                 250                 255
Ser Asn Val Glu Val Glu Phe Leu Leu Asn Gly Gln Ile Ile Asn Thr
            260                 265                 270
Tyr Gln Ala Arg Phe Gly Thr Ile Ile Ala Arg Asn Phe Asp Thr Ile
        275                 280                 285
Arg Leu Ser Phe Gln Leu Val Arg Pro Pro Asn Met Thr Pro Ala Val
    290                 295                 300
Ala Asn Leu Phe Pro Gln Ala Pro Pro Phe Ile Phe His Ala Thr Val
305                 310                 315                 320
Gly Leu Thr Leu Arg Ile Glu Ser Ala Val Cys Glu Ser Val Leu Ala
                325                 330                 335
Asp Ala Ser Glu Thr Leu Leu Ala Asn Val Thr Ala Val Arg Gln Glu
            340                 345                 350
Tyr Ala Ile Pro Val Gly Pro Val Phe Pro Pro Gly Met Asn Trp Thr
        355                 360                 365
Glu Leu Ile Thr Asn Tyr Ser Pro Ser Arg Glu Asp Asn Leu Gln Arg
    370                 375                 380
Val Phe Thr Val Ala Ser Ile Arg Ser Met Leu Ile Lys Gly Gly Gly
385                 390                 395                 400
Gly Ser Gly Gly Gly Gly Ser Gly Tyr Pro Tyr Asp Val Pro Asp Tyr
                405                 410                 415
Ala Gly Tyr Pro Tyr Asp Val Pro Asp Tyr Ala Gly Ser Tyr Pro Tyr
            420                 425                 430
Asp Val Pro Asp Tyr Ala Ala Gln Cys
        435                 440

<210> 22
<211> 370
<212> PRT
<213> Artificial Sequence

<220>
<223> PRVA VP7 modified

<400> 22
Met Tyr Gly Ile Glu Tyr Thr Thr Val Leu Thr Phe Leu Ile Ser Leu
1               5                   10                  15
Val Phe Val Asn Tyr Ile Leu Lys Ser Val Thr Arg Thr Met Asp Phe
            20                  25                  30
Ile Ile Tyr Arg Phe Leu Leu Val Ile Val Val Leu Ala Pro Leu Ile
        35                  40                  45

Lys Ala Gln Asn Tyr Gly Ile Asn Leu Pro Ile Thr Gly Ser Met Asp
    50                  55                  60
Thr Pro Tyr Met Asn Ser Thr Thr Ser Glu Thr Phe Leu Thr Ser Thr
65                  70                  75                  80
Leu Cys Leu Tyr Tyr Pro Asn Glu Ala Ala Thr Glu Ile Ala Asp Thr
                85                  90                  95
Lys Trp Thr Glu Thr Leu Ser Gln Leu Phe Leu Thr Lys Gly Trp Pro
            100                 105                 110
Thr Gly Ser Val Tyr Phe Lys Gly Tyr Ala Asp Ile Ala Ser Phe Ser
        115                 120                 125
Val Glu Pro Gln Leu Tyr Cys Asp Tyr Asn Ile Val Leu Met Lys Tyr
    130                 135                 140
Asp Gly Asn Leu Gln Leu Asp Met Ser Glu Leu Ala Asp Leu Ile Leu
145                 150                 155                 160
Asn Glu Trp Leu Cys Asn Pro Met Asp Ile Met Leu Tyr Tyr Tyr Gln
                165                 170                 175
Gln Thr Asp Glu Ala Asn Lys Trp Ile Ser Met Gly Thr Ser Cys Thr
            180                 185                 190
Ile Lys Val Cys Pro Leu Asn Thr Gln Thr Leu Gly Ile Gly Cys Ser
        195                 200                 205
Thr Thr Asp Ile Asn Ser Phe Glu Thr Val Ala Asn Ala Glu Lys Leu
    210                 215                 220
Ala Ile Thr Asp Val Val Asp Gly Val Asn His Lys Leu Asp Val Thr
225                 230                 235                 240
Thr Ser Thr Cys Thr Ile Arg Asn Cys Lys Lys Leu Gly Pro Arg Glu
                245                 250                 255
Asn Val Ala Val Ile Gln Val Gly Gly Pro Asn Ile Leu Asp Ile Thr
            260                 265                 270
Ala Asp Pro Thr Thr Ala Pro Gln Thr Glu Arg Met Met Arg Ile Asn
        275                 280                 285
Trp Lys Arg Trp Trp Gln Val Phe Tyr Thr Ile Val Asp Tyr Val Asn
    290                 295                 300
Gln Ile Val Gln Val Met Ser Lys Arg Ser Arg Ser Leu Asp Ser Ala
305                 310                 315                 320
Ala Phe Tyr Tyr Arg Val Gly Gly Gly Gly Ser Gly Gly Gly Gly Ser
                325                 330                 335
Gly Tyr Pro Tyr Asp Val Pro Asp Tyr Ala Gly Tyr Pro Tyr Asp Val
            340                 345                 350
Pro Asp Tyr Ala Gly Ser Tyr Pro Tyr Asp Val Pro Asp Tyr Ala Ala
        355                 360                 365
Gln Lys
    370

<210> 23
<211> 361
<212> PRT
<213> Artificial Sequence

<220>
<223> PRVA NSP2 modified

<400> 23
Met Ala Glu Leu Ala Cys Phe Cys Tyr Pro His Leu Glu Asn Asp Ser
1               5                   10                  15
Tyr Lys Phe Ile Pro Phe Asn Asn Leu Ala Ile Lys Cys Met Leu Thr
            20                  25                  30
Ala Lys Val Asp Lys Lys Asp Gln Asp Lys Phe Tyr Asn Ser Ile Val
        35                  40                  45
Tyr Gly Ile Ala Pro Pro Pro Gln Phe Lys Lys Arg Tyr Asn Thr Ser
    50                  55                  60
Asp Asn Ser Arg Gly Met Asn Tyr Val Thr Thr Met Phe Asn Lys Val
65                  70                  75                  80

Ala Ile Leu Ile Cys Glu Ala Leu Asn Ser Ile Lys Val Thr Gln Ser
                85                  90                  95
Asp Val Ala Asn Val Leu Ser Arg Val Val Ser Val Arg His Leu Glu
            100                 105                 110
Asn Leu Val Leu Arg Lys Glu Asn His Gln Asp Val Leu Phe His Ser
        115                 120                 125
Lys Glu Leu Leu Leu Lys Ser Val Leu Ile Ala Ile Gly Gln Ser Lys
    130                 135                 140
Glu Ile Glu Thr Thr Ala Thr Ala Glu Gly Gly Glu Ile Val Phe Gln
145                 150                 155                 160
Asn Ala Ala Phe Thr Met Trp Lys Leu Thr Tyr Leu Asp His Lys Leu
                165                 170                 175
Met Pro Ile Leu Asp Gln Asn Phe Thr Glu Tyr Lys Ile Thr Leu Asn
            180                 185                 190
Glu Asp Lys Pro Ile Ser Asp Val Cys Val Lys Glu Leu Val Ala Glu
        195                 200                 205
Leu Arg Trp Gln Tyr Asn Arg Phe Ala Val Ile Thr His Gly Lys Gly
    210                 215                 220
His Tyr Arg Val Val Lys Tyr Ser Ser Val Ala Asn His Ala Asp Arg
225                 230                 235                 240
Val Phe Ala Thr Tyr Lys Asn Asn Ala Lys Ser Gly Asn Val Thr Asp
                245                 250                 255
Phe Asn Leu Leu Asp Gln Arg Ile Ile Trp Gln Asn Trp Tyr Ala Phe
            260                 265                 270
Thr Ser Ser Met Lys Gln Gly Asn Thr Leu Asp Val Cys Lys Lys Leu
        275                 280                 285
Leu Phe Gln Lys Met Lys Gln Glu Lys Asn Pro Phe Lys Gly Leu Ser
    290                 295                 300
Thr Asp Arg Lys Met Asp Glu Val Ser His Val Gly Ile Gly Gly Gly
305                 310                 315                 320
Gly Ser Gly Gly Gly Gly Ser Gly Tyr Pro Tyr Asp Val Pro Asp Tyr
                325                 330                 335
Ala Gly Tyr Pro Tyr Asp Val Pro Asp Tyr Ala Gly Ser Tyr Pro Tyr
            340                 345                 350
Asp Val Pro Asp Tyr Ala Ala Gln Cys
        355                 360

<210> 24
<211> 219
<212> PRT
<213> Artificial Sequence

<220>
<223> PRVA NSP4 modified

<400> 24
Met Asp Lys Leu Ala Asp Leu Asn Tyr Thr Leu Ser Val Ile Thr Leu
1               5                   10                  15
Met Asn Asp Thr Leu His Ser Ile Ile Gln Asp Pro Gly Met Ala Tyr
            20                  25                  30
Phe Pro Tyr Ile Ala Ser Val Leu Thr Val Leu Phe Thr Leu His Lys
        35                  40                  45
Ala Ser Ile Pro Thr Met Lys Ile Ala Leu Lys Thr Ser Lys Cys Ser
    50                  55                  60
Tyr Lys Val Ile Lys Tyr Cys Met Val Thr Ile Ile Asn Thr Leu Leu
65                  70                  75                  80
Lys Leu Ala Gly Tyr Lys Glu Gln Val Thr Thr Lys Asp Glu Ile Glu
                85                  90                  95
Gln Gln Met Asp Arg Ile Ile Lys Glu Met Arg Arg Gln Leu Glu Met
            100                 105                 110
Ile Asp Lys Leu Thr Thr Arg Glu Ile Glu Gln Val Glu Leu Leu Lys
        115                 120                 125

Arg Ile His Asp Lys Leu Ala Ala Arg Ser Val Asp Ala Ile Asp Met
    130                 135                 140
Ser Lys Glu Phe Asn Gln Lys Asn Ile Arg Thr Leu Asp Glu Trp Glu
145                 150                 155                 160
Ser Gly Lys Asn Pro Tyr Glu Pro Ser Glu Val Thr Ala Ser Met Gly
                165                 170                 175
Gly Gly Gly Ser Gly Gly Gly Gly Ser Gly Tyr Pro Tyr Asp Val Pro
            180                 185                 190
Asp Tyr Ala Gly Tyr Pro Tyr Asp Val Pro Asp Tyr Ala Gly Ser Tyr
        195                 200                 205
Pro Tyr Asp Val Pro Asp Tyr Ala Ala Gln Cys
    210                 215

<210> 25
<211> 1612
<212> DNA
<213> Artificial Sequence

<220>
<223> Promoter Sequence

<400> 25
attagctggt taataggtat aataataata ataataataa taacaatagc aaattatagt      60

agaaacgcta ttctatttct attttaattt ttgatttgat tttttagggt agcacactag     120

acacgcttct gaaaagatgt gtgtatgtgt ctcttcttgt ttgacagttt gatatgacta     180

agtgtctatt ttctgtttct gaagttctcc cccataaaaa taacacaact gacaacaaca     240

catgtcaaat cccgtcatga tcccactccc ggtctccagg tccccaatgc tcaaacaccc     300

atgtctttta tatcttttgc gctgcccatt ttacaaagaa accttgtacc tttaaaaagt     360

gatatgacga aaaaaagaac aaattaaaaa acagtggggc cctccaaaga atcagtcaat     420

aatcacaaaa cttacattac agcacggcac agagcgcatg ccgcatagta gaaggggagc     480

tacagaagag aaaaaaaaaa aaaaaaaaca aaaaaaaaaa aactaaggta acgtaaagta     540

agtataataa aaataatctc atgagcacca ccaccgctcg ccgcgatata caaggtggga     600

tgggatggga ttattccagg atgcggcgcg gcgcggcgta ggaaagcagt agcaagcccg     660

gtactagact gtactagact gtactaacta accccagccg atcgccaatg gaacaatggc     720

atgcggggac ggcccactgt gttccgctta cacgtgatca aattcggccg cgtactcccc     780

gggttcggac aggtatggtc cgtgcgcacc cggtgcactc caccccgcgc gcggcccact     840

gccttccgga aaccaaaccc aaaaaccaaa accaaaacca aaaccaaaac caaagaaacc     900

aaaacacata cctaaaccac ccagaaaagc ccgcgcaaga aaccgtttct ccccctctct     960

ctctgccccg ggtttccagg tattctcact caaggcatcc caattccagg cttcgcttct    1020

agcagcacct gtggtgctgc ttgctggtgt ggtaccagcg tttctttacg cctggccact    1080

gggcactgtg cgccgaaacg cgcaagaaca gtagtagtag tagtggcaca ttagcaagct    1140

tttctgctgt gaatgtgatt gatggatgga tgattggcac tccatccgtt ccttgcttac    1200

gcaagctggc atatggcaat gtgtatgtgt gtgccccgaa gtataggcag ctaagcatat 1260

gatagttggc tcgctctgcc tttgcagtgt cagatataca cagtatataa ggagttgcct 1320

gaatcttgtc agccggttct tagaagtctt cagaaacact ttcgattcgg gcacgttgta 1380

taaataagtg tgttgtgttt cagtttggag agagtgttgt tattactatt attattattc 1440

gtgtgtatgt ccatttgaaa gtatatatat atatatatat gtatatatgt ctggttagtt 1500

tgtttgtcta taacaaccaa aaggtaaaat agtaatagtc tggagaccct aaaagtatag 1560

gattccaagc aaactagaaa aaaccaaaaa aaacatcaag aacaatcaag aa 1612

<210> 26
<211> 3193
<212> DNA
<213> Artificial Sequence

<220>
<223> Promoter Sequence

<400> 26
atctttcagt tctcgatgag tatgtgtgtt tatttttttt tttttttgcc aaattctgct 60

ctttcctaaa tttcaagtgt taagcgcttt tgtctcttca ccttttccaa ctacaagcgc 120

aacataagaa aaggataata attctactag gaaacacaaa cctcatatac ctttcgagtt 180

agggaagaac accttcacaa tgataacaca ttgattcgag ctattaaata cctttttctc 240

aatcgaaatc tcaagtaaaa cagcgatgaa aacattacgt aactaatggt gttcaccact 300

agaaatcata cccttcacac tcgacttcaa gtagtgaatg gtgtagcaac aaagtccaaa 360

tgccaatatc aaccaagtaa ccaaccgcgc tactagagaa agacgctgtt gctcggacca 420

caaatttccg ctacactttt cacaactata ctgaatatac aaaaaaacgt ttgtgtgggt 480

atggctggct accaggtcgc ctggttaaac caagtcaacg tgatacatat gtacattcca 540

acactaagcc taccctaagt tttagctcac aggctaggct attattaaca tacaagacaa 600

gggagaagca aagaaaagac caaccgaaag ccaccagagc accctgaact ttgcggtgaa 660

cagaattccg caacatatct gaggattcca tgatctcggt ttcctactcc aaatgggaat 720

cacccactgt tgtccgtaaa tatgactaaa ttcctacctt gattcctcac gaacaatcgc 780

agtccgaaaa gccgttccaa aagccagtcc acagttcatc gattggtatg attattgttt 840

ttttgttcaa actgacacac taacggtgtt gtatgcgaag agtgagctta cccttctcct 900

ctttgctagc actacttgcc tacctaccta ctctactcta ctacgctgcc atattgtcta 960

acattctgct ttctctattt ctacctggcc tggatggctc cgtctcgccc gcctcacacg 1020

cacacacata caaacacatt ccttccccct ctcgcctgcc ccataataat taaactagtt 1080

aacaaaaggc tttacgtctt ccgctacctc tccaatctca tacgactctc ctttcatccg 1140

acttacccaa gaagatgcag gatctaagtc aaagatcctg cctgccctgg ctgttgtcta 1200

ctgtacacac gagatttgga aggccagggg actgtcgcat gattattcgc cgggaacccg 1260

gaagttccct gttcttacct ttcaaactag gcagcaaacc aggtgaacac attttgatgt 1320

agtaggcctt tgaagattcg gggagctagc tagccacgcg aatgtaacaa cagaacattt 1380

atttttgtgg gggtggaaaa gtcgattatg atcttgcagc acagaaactg cgcaggggct 1440

tttttcatct tggagaagca actggctaaa ttcgacacaa acaaaaactg aaaaatggaa 1500

aataaaaaat gaaaaagcaa gctgaattcg aagaagggag aattccgcct tctgcaacca 1560

cactaatggt tggtagtcaa tggatacgca ttagaaggtg actattttat gagtcgatcc 1620

ccgcggaaat ttaggaattt taaacttggg cttgatatac gtaggtatag tgtatctggt 1680

atttttgcca atggttcctg tgcccgtgcc catgtccgtg tgctcatgcg atgcactgtg 1740

actactgctt ggcaaggcaa atcggcggcc tgcggccgta ctctaaagcc ggacaggcat 1800

ggtggggtgc cttttcggtc cataagtgta gctagatagc tagctagcta cacgtatgtt 1860

ctgtgcgaag atggtaaagg ttgcatacag tcaggctgct gtctttggac ctggtctgac 1920

ctgacctagc ataatattgc atagcgtata cccacttgga tggatgcagg agtgctacaa 1980

atcggcgaac agttctcctt gtttgttttt ggtcatagtc acgtccggct acacacaaga 2040

tattgcacag tatgtgtaga tacgagcgaa ggtacggctg gaaaacaggt gggttttgga 2100

tgatagacag aaggcaagag ggtgggcaag aagtcggcca cacaggcaag tattagcatg 2160

gtcaaaccag attaaggtct aggccaagaa caattttcgg ccccccactg actcccgcca 2220

aaaaaaaaaa aaaaaaaaaa aacctaattt gacagtctcg gaattttatc tatctgttcc 2280

tttcatttgg taactagtat cgtcactttt tttttggaat tggcaagcta cctgcatggc 2340

cgtataccga atgaacccca gaaaatggta tagctaagct ttcgtacgac ggaaatatgt 2400

agagcgaagg gttcttggtc tgtgaaaggg gttttctgta ctacgtaaag aggggtcttc 2460

attatagctt ttcaatggat tcgaaatgga agtaagcgta agagaatgaa aaacaagaaa 2520

atagttagaa ataaaaaaaa aaaaaaacac ttggctgtct gccggacttg tcttgtcttg 2580

gtggaaatca ttactccttg gcgggcgatg aatgtgcttc tgcttctttt cattaataca 2640

taccgtcatt ggtagtattc tgatttcatt tctccacacc attttttttg cgttagtcgt 2700

ctgcggtata tgcgaccctg gccgttcggt gcgccgagtg cgtttacctg tgtaggcgct 2760

tttgtaagag agaaactcgg gtctgggtgg gcctgcaacg tgacttagca atacccgcgc 2820

gtcagattta acatattcgg gattcttccc tccgtagtag aaagttgaca ataagatgtc 2880

catactgttg ttgatacctt gctactctag ctcattatta caaggacagc cggttaattg 2940

gaggcctaaa tggtgtatca aggtatataa agccaataac tttcaatgtt gagttgggct 3000

gtagttcaag gtggttgtag cttgtggaac atatatatgc tattcaatat agctagaaag 3060

tgtaagctaa acagcaagct aaacagcaaa caaaagtctg tgtgtgttgt cttataatta 3120

tatccttcag tcagtgttaa taattaaaga cagaattacg aaaaataaaa caggacgata 3180

atcagttttc ata 3193

<210> 27
<211> 2822
<212> DNA
<213> Artificial Sequence

<220>
<223> Promoter Sequence

<400> 27
atctttcagt tctcgatgag tatgtgtgtt tatttttttt tttttttgcc aaattctgct 60

ctttcctaaa tttcaagtgt taagcgcttt tgtctcttca ccttttccaa ctacaagcgc 120

aacataagaa aaggataata attctactag gaaacacaaa cctcatatac ctttcgagtt 180

agggaagaac accttcacaa tgataacaca ttgattcgag ctattaaata cctttttctc 240

aatcgaaatc tcaagtaaaa cagcgatgaa aacattacgt aactaatggt gttcaccact 300

agaaatcata cccttcacac tcgacttcaa gtagtgaatg gtgtagcaac aaagtccaaa 360

tgccaatatc aaccaagtaa ccaaccgcgc tactagagaa agacgctgtt gctcggacca 420

caaatttccg ctacactttt cacaactata ctgaatatac aaaaaaacgt ttgtgtgggt 480

atggctggct accaggtcgc ctggttaaac caagtcaacg tgatacatat gtacattcca 540

acactaagcc taccctaagt tttagctcac aggctaggct attattaaca tacaagacaa 600

gggagaagca aagaaaagac caaccgaaag ccaccagagc accctgaact ttgcggtgaa 660

cagaattccg caacatatct gaggattcca tgatctcggt ttcctactcc aaatgggaat 720

cacccactgt tgtccgtaaa tatgactaaa ttcctacctt gattcctcac gaacaatcgc 780

agtccgaaaa gccgttccaa aagccagtcc acagttcatc gattggtatg attattgttt 840

ttttgttcaa actgacacac taacggtgtt gtatgcgaag agtgagctta cccttctcct 900

ctttgctagc actacttgcc tacctaccta ctctactcta ctacgctgcc atattgtcta 960

acattctgct ttctctattt ctacctggcc tggcctgcag gggctccgtc tcgcccgcct 1020

cacacacata cattcctccc cctctcgcct gccccataat aattaaacaa gttaacaaaa 1080

ggcgttacct cttccgcatc ctctccaatc tcatacgatt cccctttcat ccgacttacc 1140

caacaagata caggatctca gtgaaagatc cttcctgccc tccctgtctg ttgtctactc 1200

tacatgcgac ttggaaggcc aaaggactat cgcatgatta ttcgccggga acccgcgagt 1260

tccctgctct tttctttcaa accaggcagc aaaccaggtg aacacactct gatgtagtgc 1320

agtccctaag tcctttgaag attcggggag ctagctaccc acgcgaatgt aacaaaagaa 1380

catttacttt tgtggggggt ggaaaagtcg attaggatct tgcagcacag aaactgcgca 1440

ggggtttttt tcatcttgga gaagcaactg gctaaattcg acacaaacaa aaactgaaaa 1500

atggaaaata aaaaatgaaa aagcaagctg aattcgaaga agggagaatt ccgccttctg 1560

caaccacact aatggttggt agtcaataga tacgcattag aaggttacta ttttatgagt 1620

cgatccccgc ggaaatttag gaattttaaa cttgggcttg atatacgtcg gtatagtgta 1680

tctggtattt ttgccaatgg ttcctgtgcc cgtgcccgtg cccgtgtgct catgcgatgc 1740

attgtgacta ctgcttggca aggcaaatca gcggtctgcg gccgtactct aaagccggac 1800

agacatggtg gggtgccttt tcggtccata agtgtaacta gctagctaca agtatgttct 1860

gtgcaaagat ggtaaaggtt gcatacagtc aagttgctgt ccttggacct ggtctgacct 1920

gacctagctt aacattgcat agcgcatacc cacttggatg gatgcaggag tgttacaaat 1980

cggcgaacag ttctccttgt ttgtttttgg tcatagtcac gtccggctac acacaagata 2040

tggcacagta tgtgtagatg cgagcgaaga tacggctgga aaacaggtgg gttttgaagg 2100

atagacagaa ggcaagaggg tgggcaaaaa gtcgggcact caggcaagta ttggcatgat 2160

caaaccagat taagttctag gccaagaaca tatttcggcc cactgactcc cgccaaaaaa 2220

aaaaaaaaaa aaacctaatt tgacagtctc ggaattttat ctatctgttc ctttcctttg 2280

gttactagta tcgtcattta attttttttg gaattggcaa gctacctgcg tggctctata 2340

ccgaatgaac cccgaccctg gccgttcggt gcaccgagcg cgtttacgta tgtacacgct 2400

tttgtaagaa agaaactcgg gtctgcgtgg gcctgcaacg tgacttagca atacccgcgc 2460

gtcagattta acatattccg gattcttccc tccgtactag aaagttgaca ataagatgtc 2520

catactgttg ttgatacctt gctactctag ctcattatga caaggacaat cggttaattg 2580

gaggtctaaa tggtgtatca aggtatataa aggcagtaac tttcaatgtt gagttgggct 2640

gtagttcaag gtggttgtag cttctggaac atatatatgc tattcaatat agctggaaag 2700

tgtaagctaa acagcaaaca aaagtctctg tgtgttgtct tataattata tccttcagtc 2760

agtgttaatg attaaagaca gaattacgaa aaaataaaac aggacgataa tcagttttca 2820

ta 2822

<210> 28
<211> 2361
<212> DNA
<213> Artificial Sequence

<220>
<223> Promoter Sequence

<400> 28
ttctagttgt tggttgttgt ttttggagtt agtttgtagc tagcgtttta atgcttgatt 60

gctggtgttt gtagttcaaa agatattata attgagaaac aatgggacaa gtaacgagat 120

gggttaggat tggattgttg taaattgggt caatttgctg gtccttatat acagattgaa 180

cgacgaagat gttggatctc gttcctcaaa gtgttgccga tatgttgggg caagcaaaca 240

agcaggcagg caggctgcca ggcgggcagc cagtcagtca atcagtcagt cagttagcca 300

gccatcagct gtccaatatt agctacctta gcttaaggca aacacaaaca cacacggttt 360

ccgcgcggga agcccaaagc ccagtaaacg cagcagtacc tgcgtgagca tgtaagaagc 420

actaagacgt gatcttgatc ttgaacttga actactacca ctgcagcgcc tgctgcccgt 480

ctgtttgggt tttggcttgg cttttgggct tcgaaggaag cattgggctt tcacagacaa 540

ccgcgtagac ccctcacttg tggtcaaaaa agaacccttc gaaaaacaaa acagggaaaa 600

atggaaaaaa aaaccgtatg agaaaaaggt atggttccac tcaatcaccg gccatctcgc 660

actggtgatc tcacaaagag gaagtgtttg agaacgaata aaaaaaaaat ccgagcccag 720

tgcacgccct gcagtaggtg ttgtagcagt gggtgggtgg gtattgctta aacggaaaaa 780

cttacgagaa tcaacggaaa aaaaaaaaat taacaaaacc aaaccaaaca aacctgggtc 840

tgagatagga tagggcaggc agtgagtatt tggttggttg gtgggtgggt ggttacgagc 900

cagcagcacc agggaaacga gaggagtaat gagaaatgaa gactgcgaac cacttctgga 960

agaaacaaca tacacggacg agagagatca ggttacttaa aagaggaagc tgcgggccag 1020

ctgatttcgg actacaggaa gctgaaggtg tgttttcaag gaatccggag aggaggccgc 1080

cagaggctag gccagaggct aggccagagg cgaggcaaaa ggcgaagcta aaaaaaagga 1140

atccggacaa ctctcctacc cccctattat ttgcaatact ggaacccagg ctggtgagcg 1200

agcgcgagta cgtgtgctct ctttagtgga acccaccgga actcactgga agccagcttc 1260

ctgtcacgtg acaggaagcc agaacccctc ctcgcctctc cttgccccct ccaataagaa 1320

tcgttacata ccaacaaaaa cacccacacc cacacaccca caactacact cctatcgcac 1380

aggaagccag gactccgaac agctagctgc ttgcttgctg ctcgcttgtt gcctgcttgc 1440

tgcctgccta caaaccaacc aaccttaaaa cgctctctgt tgccttctac caccgcacca 1500

gcccagaacc ccacgcacca ccagtggcac catctccgag aatcgtccct cccccggacg 1560

tccttaagtc ataccactgc aaccactcaa ctctgaaacg cacctggctt ccacttttct 1620

gcacaggctc ccagctacca catttcccca gaaattctcc catccatgcc cctacaatta 1680

aaaatcacct tcccacaact ccgcagagaa ccagagcaca tgattggcat ccctagcccc 1740

catcactact ggattctcgg ttgttacact gcaacgtgca ttcttctatg ccactctacg 1800

ctgtggtttc ctagtgtttc caaaccagat ttcttggtgt ttcgtggcga tttcctgatt 1860

tcctgatttc ctggtttcct gattccttcc ttccttcctg gtggtgtctc tctttctgtg 1920

tgttttttct ttctttcttt ctttattgct tgcttgctgc catgctgcta ccttaattct 1980

tcttttttcc taccctaatt cttgtaattt gtaatgaaag acaagaccac catacataca 2040

cacatacatg atatcgtggc attgtgccat ggcaccatgg aatgggatat gggatatatg 2100

gcaccgtctt tacttagctc cgaagctttg aggatccgaa atttggtata taaggggtgg 2160

aacattgtca gaaagaagca atccatccaa gttgtcttga tgtcagtttt agtttagatg 2220

tttcagcatt tagttttagt gttagcttgt ctactctccc caaggaacaa ggacactttt 2280

tcatacaaag tcttctgttt gcacgaattt aattgcgtac gttctccccc acacacaact 2340

caactcagat aattttaaaa g 2361

<210> 29
<211> 250
<212> DNA
<213> Artificial Sequence

<220>
<223> Terminator Sequence

<400> 29
tcagtactga caataaaaag attcttgttt tcaagaactt gtcatttgta tagttttttt 60

atattgtagt tgttctattt taatcaaatg ttagcgtgat ttatattttt tttcgcctcg 120

acatcatctg cccagatgcg aagttaagtg cgcagaaagt aatatcatgc gtcaatcgta 180

tgtgaatgct ggtcgctata ctgctgtcga ttcgatacta acgccgccat ccagtgtcga 240

aaacgagctc 250

<210> 30
<211> 250
<212> DNA
<213> Artificial Sequence

<220>
<223> Terminator Sequence

<400> 30
aagtgtcgaa cttgaaaata tcaatggaaa agaatcaatc agtggattcg aagaaagttc 60

tactttctaa ctcagcttca cccgtacctc tagtcgaaac cacattggag ctaaacatta 120

gaagccagtt aggagatttc ttttcttcaa gggtaggatg ttagtgtaat atatttatat 180

tcatacatat atatatatgt atttacgagt gttttaaggt gatatgaaat tccttatcaa 240

acctatcaat 250

<210> 31
<211> 252
<212> DNA
<213> Artificial Sequence

<220>
<223> Terminator Sequence

<400> 31
atagtttgtc caaatctgtc gttaacaaaa actatacaaa ccagattttt atgagctttc 60

tgtaaaaaaa taacaatata aatgttttca aaacgagtaa gcagtctaac tctgcttgga 120

acctatatat acgcgtctct tgtaaacacc atatgtagtg tcagaataac ttgtcagctc 180

aactgaaata gtactaggat ttataaaaaa aatataataa ccgggtaaca aattttgttc 240

tggtcgtttt tg 252

<210> 32
<211> 190
<212> DNA
<213> Artificial Sequence

<220>
<223> Terminator Sequence

<400> 32
atccgctcta accgaaaagg aaggagttag acaacctgaa gtctaggtcc ctatttattt 60

ttttatagtt atgttagtat taagaacgtt atttatattt caaatttttc ttttttttct 120

gtacagacgc gtgtacgcat gtaacattat actgaaaacc ttgcttgaga aggttttggg 180

acgctcgaag 190

<210> 33
<211> 188
<212> DNA
<213> Artificial Sequence

<220>
<223> Terminator Sequence

<400> 33
gcgaatttct tatgatttat gatttttatt attaaataag ttataaaaaa aataagtgta 60

tacaaatttt aaagtgactc ttaggtttta aaacgaaaat tcttgttctt gagtaactct 120

ttcctgtagg tcaggttgct ttctcaggta tagcatgagg tcgctcttat tgaccacacc 180

tctaccgg 188

<210> 34
<211> 2805
<212> DNA
<213> Artificial Sequence

<220>
<223> PRVA VP2 modified sequence

<400> 34
atggcataca gaaagagagg tgcaaaaaga gaaaactcat cacaacaaaa cgaaagatta 60

caagaaaagg aagtagaaaa gaacattgat gcatctatgg aaaataaggc taacaataag 120

aaacaacaat tgccagataa ggttttgtca caaaaggaag aaatcatcac tgatgctcaa 180

gatgatgtta agatcacaga tgaagttaag aaatcttcaa aagaagaatc taagcaattg 240

ttggaaattt tgaagactaa agaagatcat caaaaagaaa ttcaatacga aattttacaa 300

aagactatcc caacattcga accaaaggaa tcaatcttga agaaattgga agatatcaag 360

ccagaacaag ctaagaaaca aactaagttg tttagaatct tcgaaccaag acaattgcca 420

atctatagag ctaatggtga aagagaattg agaaacagat ggtactggaa gttgaagaga 480

gatacattgc cagatggtga ctatgatgtt agagaatact ttttgaattt gtacgatcaa 540

attttaatcg aaatgccaga ttatttgttg ttgaaggata tggcagttga aaataagaac 600

tctagagatg ctggtaaagt tgttgattct gaaactgcat caatctgtga tgctatcttc 660

caagatgaag aaacagaagg tgttattaga agattcattg cagatatgag acaacaagtt 720

caagctgata gaaacgttgt taactaccca tctatcttgc atccaatcga tcatgctttt 780

aatgaatact ttttgaacca tcaattggtt gaaccattga acaacgaaat cattttcaat 840

tacattccag aaagaattag aaatgatgtt aattatattt tgaatatgga tatgaatttg 900

ccatctactg ctagatacat tagaccaaat ttgttgcaag atagattgaa tttgcatgat 960

aacttcgaat cattgtggga tactatcact acatctaact acatcttggc aagatcagtt 1020

gttccagatt tgaaggaaaa ggaattagtt tctacagaag ctcaaatcca aaagatgtca 1080

caagatttgc aattggaagc attgactatc caatctgaaa cacaattctt ggctggtatt 1140

aattcacaag ctgcaaacga ttgtttcaag actttgatcg ctgcaatgtt gtctcaaaga 1200

acaatgtcat tggaattcgt tactacaaac tacatgtctt tgatttcagg catgtggttg 1260

ttgactgtta ttccaaacga tatgttcttg agagaatctt tggttgcttg tgaattggca 1320

attattaaca ctatcgttta cccagctttc ggtatgcaaa gaatgcatta cagaaatggt 1380

gacccacaaa caccatttca aattgcagaa caacaaatcc aaaacttcca agttgctaac 1440

tggttgcatt tcatcaacaa caacagattc agacaagttg ttattgatgg tgttttgaac 1500

caaactttga acgataacat cagaaatggt caagttatta atcaattgat ggaagctttg 1560

atgcaattgt ctagacaaca attcccaaca atgccagttg attacaagag atcaatccaa 1620

agaggtatct tattgttgtc aaacagattg ggtcaattgg ttgatttgac tagattgttg 1680

tcatacaact acgaaacttt gatggcttgt atcacaatga acatgcaaca tgttcaaaca 1740

ttgactacag aaaagttgca attgacttct gttacatcat tgtgtatgtt gatcggtaac 1800

actacagtta ttccatctcc acaaactttg ttccattact acaacgttaa cgttaacttc 1860

cattcaaatt acaatgaaag aattaatgat gctgttgcaa tcatcacagc tgcaaacaga 1920

ttgaatttgt accaaaagaa aatgaaatct attgttgaag aatttttaaa aagattgcaa 1980

atttttgatg ttccaagagt tccagatgat caaatgtaca gattgagaga tagattgaga 2040

ttgttgccag ttgaaagaag aagattggat atttttaatt tgatcttgat gaatatggaa 2100

caaatcgaaa gagcatctga taagatcgct caaggtgtta ttatcgcata cagagatatg 2160

caattggaaa gagatgaaat gtacggttac gttaacatcg ctagaaattt ggatggttac 2220

caacaaatta atttggaaga attgatgaga actggtgact atggtcaaat cacaaacatg 2280

ttgttgaaca accaaccagt tgctttggtt ggtgcattgc cattcgttac tgattcttca 2340

gttatttctt tgatcgctaa gttggatgca actgttttcg ctcaaatcgt taagttgaga 2400

aaagttgata cattgaaacc aatcttgtac aagattaatt ctgattcaaa tgatttttac 2460

ttagttgcaa attatgattg gatcccaaca tcaactacaa aggtttacaa gcaagttcca 2520

caaccattcg atttcagagc ttctatgcat atgttgactt caaatttgac ttttacagtt 2580

tactctgatt tgttggcatt tgtttcagct gatacagttg aaccaattaa tgctgttgca 2640

ttcgataaca tgagaatcat gaacgaattg ggtggtggtg gttctggtgg tggtggttca 2700

ggttatccat acgatgttcc agattatgct ggttatccat acgatgtccc tgattatgct 2760

ggttcttatc cttacgatgt ccctgactat gctgctcaaa agtga 2805

<210> 35
<211> 2463
<212> DNA
<213> Artificial Sequence

<220>
<223> PRVA VP4 modified sequence

<400> 35
atggcttctt tgatctatag acaattgttg actaactcat acacagttaa tttgtctgat 60

gaaatccaag aaatcggttc tgctaagtct caagatgtta ctattaatcc aggtccattt 120

gctcaaacag gttacgcacc agttaattgg ggtgcaggtg aaactaatga ttctactaca 180

gttgaaccat tgttagatgg tccataccaa ccaactactt ttaatccacc aacttcttat 240

tgggttttgt tagctccaac agttgaaggt gttattatcc aaggtacaaa caacactgat 300

agatggttgg caactatctt gatcgaacca aacgttcaaa ctacaaacag aatctataat 360

ttgtttggtc aacaagttac attgtcagtt gaaaacacat ctcaaactca ttggaagttt 420

attgatgttt caactacaac tccaactggt tcttacacac aacatggtcc attattttca 480

actccaaagt tgtacgctgt tatgaaattt tctggtagaa tctatacata caatggtaca 540

actccaaatg caacaactgg ttactactca acaactaact acgatactgt taacatgaca 600

tctttctgtg atttctacat tattccaaga aaccaagaag aaaagtgtac tgaatacatc 660

aaccatggtt taccaccaat ccaaaacaca agaaacgttg ttccagtttc attgtctgct 720

agagaaattg ttcatactag agcacaagtt aacgaagata tcgttgtttc aaagacatct 780

ttatggaaag aaatgcaatg taacagagat atcactatca gattcaaatt cgatagaaca 840

attattaagg ctggtggttt gggttataaa tggtcagaaa tctcttttaa accaatcact 900

taccaataca catacgctag agatggtgaa caaattactg cacatacaac ttgttctgtt 960

aacggtgtta acaacttctc atacaacggt ggttctttgc caacagattt cgctatctca 1020

agatacgaag ttattaaaga aaattctttt gtttacattg attattggga tgattcacaa 1080

gcttttagaa acatggttta cgtcagatct ttggctgcaa atttgaatac agttacttgt 1140

acaggtggtt catactcttt tgctttgcca ttaggtcatt atccagttat gacaggtggt 1200

actgtttcat tacatccagc aggtgttaca ttgtctactc aattcactga tttcgtttca 1260

ttgaactctt tgagattcag attcagatta actgttggtg aaccatcatt ttctatcact 1320

agaacaagag tttctagatt gtacggttta ccagctgcaa atccaaacaa ccaaagagaa 1380

tactacgaaa tctcaggtag attttcattg atctctttgg ttccatctaa cgatgattac 1440

caaactccaa tcatgaactc agttacagtt agacaagatt tggaaagaca attgggtgaa 1500

ttgagagatg aattcaattc attgtctcaa caaatcgcta tctctcaatt gatcgatttg 1560

gcattgttgc cattggatat gttctcaatg ttctctggta ttaaatcaac tatcgatgct 1620

gcaaagtcta tggctacaaa cgttatgaag agattcaaaa gatcaaattt ggcttcttca 1680

gtttctactt tgacagatgc aatgtcagat gctgcatctt caatctctag atcttcatct 1740

atcagatcaa ttggttcatc tgcttctgca tggactgaag tttcaaattc tatcgctgat 1800

gtttcaacaa ctgttgatac agtttctact caaacagcta ctatcgcaaa gagattgaga 1860

ttgaaggaaa tcgcaacaca aactgatggt atgaacttcg atgatatctc tgctgcagtt 1920

ttgaagacta agatcgataa gtcagttcaa attactccaa atacattgcc agaaatcgtt 1980

actgaagctt ctgaaaagtt tattccaaac agaacataca gagttattaa taacgatgaa 2040

gttttcgaag ttggtatgga tggtaaattt ttcgcttaca gagttgatac tttcgatgaa 2100

atcccattcg atgttcaaaa gttcgctgat ttggttactg attcaccagt tatttctgca 2160

atcatcgatt tgaagacatt gaaaaatttg aaagataatt atggtatctc aaagcaacaa 2220

gctttcgatt tgttaagatc tgatccaaga gttttgagag aattcattaa ccaaaacaac 2280

ccaatcatca gaaatagaat tgaaaatttg attatgcaat gtagattggg tggtggtggt 2340

tctggtggtg gtggttccgg ttacccatat gatgttccag attacgctgg ttatccttat 2400

gatgttccag attatgcagg ttcctatcca tacgacgttc cagattatgc tgcacaaaaa 2460

taa                                                                 2463

<210> 36
<211> 1326
<212> DNA
<213> Artificial Sequence

<220>
<223> PRVA VP6 modified sequence

<400> 36
atggaagttt tgtactcttt gtccaagact ttgaaggatg ccagagataa gatcgttgaa      60

ggtactttgt actctaacat ttctgatttg atccaacaat tcaaccaaat gattgttacc     120

atgaacggta acgacttcca aactggtggt atcggtaact tgccaattag aaactggaac     180

ttcgacttcg gtttgttggg taccactttg ttgaacatcg atgctaacta cgttgaaaac     240

gctagaacca ctatcgaata cttcatcgac ttcatcgata acgtttgtat ggatgaaatg     300

gctagagaat ctcaaagaaa cggtatcgct ccacaatctg aagccttgag aaagttgtcc     360

ggtatcaagt tcaagagaat taacttcgac aactcttccg attacatcga aaactggaac     420

ttgcaaaaca gaagacaaag aaccggtttc gttttccaca agccaaacat tttgccatac     480

tctgcttcct tcaccttgaa cagatcccaa ccagctcacg acaacttgat gggtactatg     540

tggatcaacg ctggttctga aattcaagtt gctggtttcg attactcctg tgctttcaac     600

gctccagcta acatccaaca attcgaacac gttgttccat tgagaagagc tttgaccact     660

gctaccatca ctttgttgcc agacgctgaa agattctctt tcccaagagt tattaactcc     720

gctgatggtg ctaccacttg gtacttcaac ccagttatca ttagaccatc taacgttgaa     780

gttgaatttt tgttgaacgg tcaaatcatt aacacctacc aagctagatt cggtactatc     840

attgctagaa acttcgacac catcagattg tccttccaat tggttagacc accaaacatg     900

actccagctg ttgctaactt gttcccacaa gctccaccat tcatcttcca cgctaccgtt     960

ggtttgactt tgagaattga atctgctgtt tgtgaatccg ttttggctga tgcttctgaa    1020

accttgttgg ctaacgttac tgctgttaga caagaatacg ctatcccagt tggtccagtt    1080

ttcccaccag gtatgaactg gaccgaattg attactaact actctccatc cagagaagac    1140

aacttgcaaa gagttttcac cgttgcttct atcagatcca tgttgatcaa gggtggtggt    1200

ggttctggtg gtggtggttc cggttaccca tacgacgttc cagattacgc tggttaccca    1260

tacgatgttc cagactacgc cggttcctac ccatacgatg ttccagacta cgctgcccaa    1320

aagtaa                                                              1326

<210> 37
<211> 1113
<212> DNA

<213> Artificial Sequence

<220>
<223> PRVA VP7 modified sequence

<400> 37
atgtacggta tcgaatacac tacagttttg acatttttga tttctttggt tttcgttaac 60

tacatcttga agtcagttac tagaacaatg gattttatta tctatagatt tttgttagtt 120

attgttgttt tggctccatt gattaaagca caaaactacg gtattaattt gccaatcact 180

ggttctatgg atacaccata catgaactct actacatcag aaacattttt gacttcaaca 240

ttgtgtttgt actacccaaa cgaagctgca actgaaattg ctgatacaaa gtggactgaa 300

acattgtctc aattgttttt aactaaaggt tggccaacag gttcagttta cttcaagggt 360

tacgctgata tcgcatcttt ttcagttgaa ccacaattgt actgtgatta caacatcgtt 420

ttgatgaaat atgatggtaa tttgcaattg gatatgtctg aattggctga tttgatcttg 480

aacgaatggt tgtgtaaccc aatggatatc atgttgtact actaccaaca aactgatgaa 540

gcaaataagt ggatctctat gggtacttca tgtacaatta aagtttgtcc attgaatact 600

caaacattgg gtattggttg ttctactaca gatatcaact cattcgaaac tgttgctaac 660

gcagaaaagt tggctatcac agatgttgtt gatggtgtta accataagtt ggatgttact 720

acatcaactt gtacaatcag aaactgtaag aaattgggtc caagagaaaa tgttgcagtt 780

attcaagttg gtggtccaaa tattttggat attactgctg atccaactac agcaccacaa 840

acagaaagaa tgatgagaat taattggaag agatggtggc aagttttcta tactatcgtt 900

gattacgtta accaaatcgt tcaagttatg tctaagagat ctagatcatt ggattcagct 960

gcattttact atagagttgg tggtggtggt tctggtggtg gtggttcagg ttacccatat 1020

gatgttccag attacgctgg ttatccttat gatgttccag attatgcagg ttcctatcca 1080

tacgacgttc cagattatgc tgcacaaaaa taa 1113

## Description

### Field of the Invention

The present invention relates to recombinant yeast cells for the efficient and stable expression of transgenes, preferably for the expression of one or more immunogenic polypeptide(s) derived from a pathogen, methods of producing recombinant yeast cells and uses thereof as vaccines.

### Background of the Invention

Viral diseases are most effectively combated by vaccines. With classical viral vaccines, a distinction is made between so-called live vaccines and dead vaccines. Live vaccines are so-called "attenuated" viruses, i.e. variants with reduced virulence, which could either be isolated as natural variants, obtained by passengers in cell culture from non-host cells, or genetically engineered by targeted mutagenesis. A characteristic feature of live vaccines is the ability of the vaccination virus to replicate, albeit at a reduced level. In contrast, dead vaccines are based on viruses that have been inactivated (e.g. by chemical or physical treatment) so that they can no longer reproduce.

Although such classical vaccines have been and are used extensively, there are problems that have encouraged the development of new vaccine types in the last decade. On the one hand, vaccinated animals cannot usually be distinguished from animals that have been infected with the field virus, and on the other hand, the production of the vaccine requires the reproduction of the virus in cell cultures or chicken eggs, which requires considerable safety precautions. There are no classical vaccines for viruses that cannot be propagated in eggs or in cell cultures, such as Porcine Rotavirus of genotype C (PRVC).

Subunit vaccines (subunit vaccines; marker vaccines) are vaccines that contain only parts of the virion (the assembled virus particle), usually structural proteins with immunogenic properties on the surface of the virus particles. Subunit vaccines are significantly safer than live vaccines and also safer than dead vaccines, since the vaccine does not contain any viral genetic material that could make replication possible. Since subunit vaccines only contain immunogenic parts of the pathogen, they usually allow the differentiation of a vaccinated animal from an animal infected with the field virus. This capability is commonly termed "DIVA capable" i.e. Differentiating between Infected and Vaccinated Animals). DIVA is of eminent importance in epidemics or monitoring programs.

Viral subunits (i.e., parts of the assembled virus particle) are naturally integrated into multimeric protein complexes. Synthesis without the further parts of the assembled virus particle, i.e., the natural interaction partners can lead to incorrect protein folding, aggregation and/or degradation and may weaken the immunogenic effect. A lower immunogenicity of individual virus components may also result from the dilution effect, which occurs when a viral antigen does not cluster on the surface of the virion to be recognized by immune cells, but as a purified, individualized protein. To counteract this dilution effect, various geometric scaffolds have been developed that can be decorated with the antigen or epitopes from it. In the simplest case, viral antigens are themselves capable of assembling into regular structures and forming so-called VLPs (virus-like particles). Such VLPs are promising approaches for the development of modern subunit vaccines.

Since the genomes of many RNA viruses are segmented, changes in the serotype are not only caused by mutations, but also by so-called reassortments of homologous RNA segments. This results in new antigen combinations, possibly with altered properties (so called emerging virus variants) and may necessitate the rapid development of new vaccines.

For a long time, both prokaryotic and eukaryotic microbes have been established as "cell factories", as they allow comparatively inexpensive cultivation and enrichment of the desired products on an industrial scale.

Yeasts have recently proven to be very useful platforms for the development of subunit vaccines, particularly in the form of VLPs. They combine the advantages of microbial expression systems in terms of development and production costs with the advantages offered by a eukaryotic expression system. In contrast to bacteria, yeasts are eukaryotic cells which, like all animal cells, contain membrane-enveloped organelles that differ considerably from the cytoplasm in pH, redox potential, ionic strength and protein composition. Probably due to the co-evolution of viral and host proteins, many viral proteins can be better expressed or synthesized in yeasts than in bacteria. Stability and biochemical properties of proteinogenic virus components are significantly influenced by their intracellular localization.

The use of yeast cells as a protein factory usually involves the large-scale cultivation of yeast, followed by cell disruption and purification of a protein using biochemical methods. In this case, the protein synthesis capacity of the corresponding host cell is of primary importance. In addition, surface properties of the yeast cells are coming into focus. It is known that dendritic cells of the immune system can be activated by yeast cells. Yeast cells may therefore be advantageously used as a new class of vaccines, the whole yeast(-based) vaccines (WYVs) or whole recombinant yeasts (WRYs), in particular as subunit vaccines.

Characteristic for WYVs is the absence of cell disruption and purification of antigens. Whole yeast cells are inactivated and used for vaccination. One advantage WYVs is that VLPs or other immunogenic protein complexes remain in a physiologic environment. Multiple proteins with immunogenic potential may be expressed in the same yeast cell and ideally these recombinantly expressed antigens self-assemble in the yeast cytosol forming stable virus-like particles (VLPs), subviral particles or other highly immunogenic protein complexes.

To date, little is known whether the immune response upon administration of WYVs differs from that caused by classical vaccines. It is assumed that the yeasts are taken up and destroyed by immune cells, such as dendritic cells or macrophages, so that proteins in the cytoplasm of the yeast cell can be broken down into peptides and presented by the MHC molecules.

Among the so-called non-conventional yeasts, the genus *Kluyveromyces* is of particular interest for biotechnology. The two well studied *Kluyveromyces* species *Kluyveromyces marxianus* and *Kluyveromyces lactis* both have GRAS status (generally regarded as safe) and the ability to metabolize lactose, which is rather rare among yeasts.

Rotaviruses have a non-enveloped but relatively complex capsid structure. Therefore, the co-expression of several antigens of rotaviruses, such as the Porcine Rotavirus (PRV), in the form of VLPs is particularly challenging.

Porcine rotaviruses are the most common cause of viral gastroenteritis in young animals worldwide. Older animals can become resistant through survived infections with subsequent immunity, but also through physiological changes in the intestine. Of the 9 known rotavirus genotypes (A-I), genotypes A and C in particular occur in connection with diarrheal diseases in piglets.

Rotaviruses are double-stranded RNA viruses, which are 60 to 80 nm in size and belong to the Reoviridae family. The viruses have low host-specificity, are very resistant and cause severe gastrointestinal diseases due to their enterotoxin formation (NSP4). Rotaviruses belong to the non-enveloped RNA viruses, they are ubiquitous, almost every adult pig has undergone a rotavirus infection with corresponding antibody formation. They are among the most important pathogens in young animals and remain infectious for a long time in many environments. After oral intake, the viruses penetrate the intestinal cells (enterocytes) and destroy their resorption function.

Vaccination of pregnant animals with a so-called maternal vaccine has proven to be successful in calf rearing. Such vaccines supply the newborn with a large amount of specific rotavirus antibodies via the milk (colostrum) and may thus protect the animal from rotavirus infection in the first weeks of life. Given that there is no approved rotavirus vaccine for the vaccination of sows in various commercially important countries, there is an unmet need for an appropriate vaccine for sows against PRV.

### Figures

**Figure 1****:** Schematic overview of exemplary chromosomal loci for genomic integration of expression cassettes and for use as selection marker genes in yeast, in particular in *K. marxianus.*
**Figure 2****:** Schematic representation of *K. marxianus* mutants constructed herein. (1) *ura3-* (FOA selection); (2) *kat1*Δ *:: loxP-KanR-loxP* / *IoxP* (pSY15-R); (3) *alpha3*Δ *:: loxP-KanR-loxP* / *loxP* (pSY229-R); (4) *ade2*Δ :: *ScURA3* / *Scura3* (pSY109-R); (5) *met5*Δ *:: loxP-KanR-loxP* / *loxP* (pSY129-R); (6) *leu2*Δ :: *loxP-HygR-loxP* / *loxP* (pSY241-R); (7) *his3*Δ *:: loxP-NrsR-loxP* / *loxP* (pSY242-R); (8) *lac4-lac12*Δ *:: ScURA3* / *Scura3* (pSY102-R).
**Figure 3****:** Schematic generation of recombinant *K. marxianus* yeast cells with replacements on *ARO10-ADE2* locus and restoration of *ADE2* gene in strains with MATa-background.
Schematic representation of the *K. marxianus ARO10-ADE2* locus (1) and of the corresponding genomic region after integration of the *ScURA3*-based disruption cassettes indicated as *ade2*Δ*::ScURA3* (2). The localization of the *ARO10* and *ADE2* genes, respectively, and the directions of transcription are indicated by arrows. For the generation of recombinant *K. marxianus* strains expressing two copies of *PRVA-VP4-3HA-opt,* the pUC19-based vector pSY147 (3) is hydrolysed with appropriate restriction enzymes (*Lgu*I + *Kpn*I) and the linear fragment is transformed into SY267. Homologous recombination into the host genome leads to a replacement of the *ScURA3* gene and the restoration of the *ADE2* gene under control of its natural promoter *(P_{ADE2}).* Recombinant clones synthesize adenine (Ade+) and express two transgenes (e.g. *RVA-VP4-3HA-opt)* whose expression is controlled by one of the bidirectional promoters e.g. *P_{LAC4-LAC12}* and the *AgTEF1* terminator (T) (e.g. strains SY277 & SY107) (4).

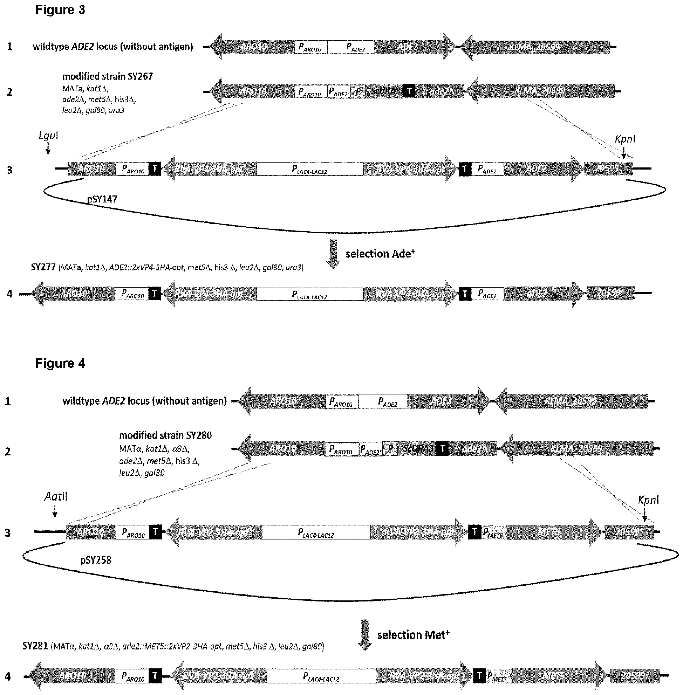

**Figure 4****:** Schematic generation of recombinant *K. marxianus* yeast cells with integration of intact *MET5* gene for selection instead of *ADE2* gene restoration in strains with MATα background.
Schematic representation of the *K. marxianus ARO10-ADE2* locus (1) and of the corresponding genomic region after integration of the *ScURA3*-based disruption cassettes indicated as *ade2*Δ::*ScURA3* (2). The localization of the *ADE2* and *ARO10* genes, respectively, and the directions of transcription are indicated by arrows. For the generation of recombinant *K. marxianus* strains expressing two copies of *PRVA-VP2-3HA-opt,* the pUC19-based vector pSY258 (3) is hydrolysed with appropriate restriction enzymes (*Aat*II + *Kpn*I) and the linear fragment is transformed into SY280. Homologous recombination into the host genome leads to integration of the intact *MET5* gene under control of its natural promoter (*P_{MET5}*) instead of *ADE2* gene. Recombinant clones synthesize methionine (Met+) and express two transgenes (e.g. *RVA-VP2-3HA-opt)* whose expression is controlled by one of the bidirectional promoters e.g. *P_{LAC4-LAC12}* respectively, and the *AgTEF1* terminator (*T*) (e.g. strain SY281 & SY102) (4).

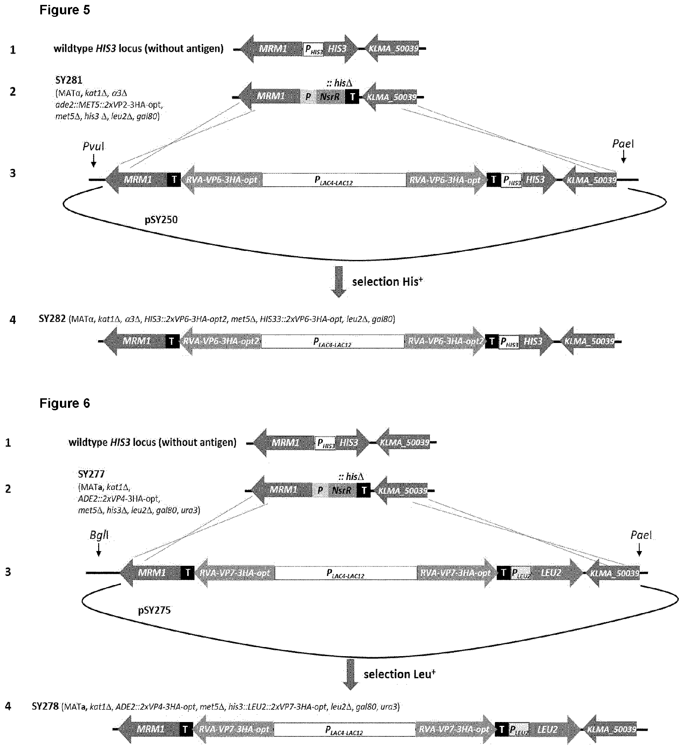

**Figure 5****:** Schematic generation of recombinant *K. marxianus* yeast cells with replacements on *HIS3* locus and restoration of *HIS3* gene in strains with MATα background
Schematic representation of the *K. marxianus MRM1-HIS3-KLMA_50039* locus (1) and of the corresponding genomic region after integration of the Nourseothricin resistance gene *(NrsR)* -based deletion cassette indicated as *his3*Δ::*NsrR* (2). The localization of the *MRM1, HIS3* and *KLMA_50039* genes, respectively, and the directions of transcription are indicated by arrows. For the generation of recombinant *K. marxianus* strains expressing two copies of *PRVA-VP6-3HA-opt2,* the pUC19-based vector pSY250 (3) is hydrolysed with appropriate restriction enzymes (*Pvu*I + *Pae*I) and the linear fragment is transformed into SY281. Homologous recombination into the host genome leads to a replacement of the *NrsR* gene and the restoration of the *HIS3* gene under control of its natural promoter *(P_{His3}).* Recombinant clones synthesize adenine (His+) and express two transgenes (e.g. *RVA-VP6-3HA-opt2*) whose expression is controlled by one of the bidirectional promoters e.g. *P_{LAC4-LAC12}* and the *AgTEF1* terminator (T) (e.g. strain SY282 & SY167) (4).
**Figure 6****:** Schematic generation of recombinant *K. marxianus* yeast cells with integration of intact *LEU2* instead of *HIS3* gene in strains with MATa background.
Schematic representation of the *K. marxianus MRM1-HIS3-KLMA_50039* locus (1) and of the corresponding genomic region after integration of the Nourseothricin resistance gene *(NrsR)* -based deletion cassette indicated as *his3*Δ::*NsrR* (2). The localization of the *MRM1, HIS3* and *KLMA_50039* genes, respectively, and the directions of transcription are indicated by arrows. For the generation of recombinant *K. marxianus* strains expressing two copies of *PRVA-VP7-3HA-opt,* the pUC19-based vector pSY275 (3) is hydrolyzed with appropriate restriction enzymes (*Bgl*I + *Pae*I) and the linear fragment is transformed into SY277. Homologous recombination into the host genome leads to a replacement of the *NrsR* gene and integration of intact *LEU2* under control of its natural promoter *(P_{LEU2})* instead of *HIS3* gene. Recombinant clones synthesize methionine (Leu+) and express two transgenes (e.g. *RVA-VP7-3HA-opf*) whose expression is controlled by one of the bidirectional promoters e.g. *P_{LAC4-LAC12}* and the *AgTEF1* terminator (T) (e.g. strain SY278 & SY171) (4).

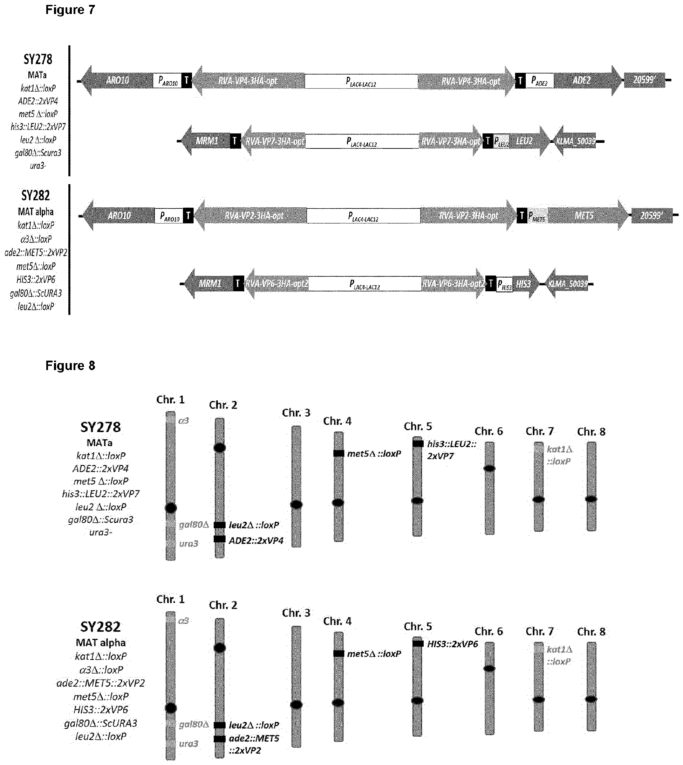

**Figure 7****:** Schematic overview of haploid *K. marxianus* yeast cells expressing *PRVA* antigens before crossing.
**Figure 8****:** Schematic overview of haploid *K. marxianus* yeast cells expressing *PRVA* antigens before crossing (chromosomal loci).

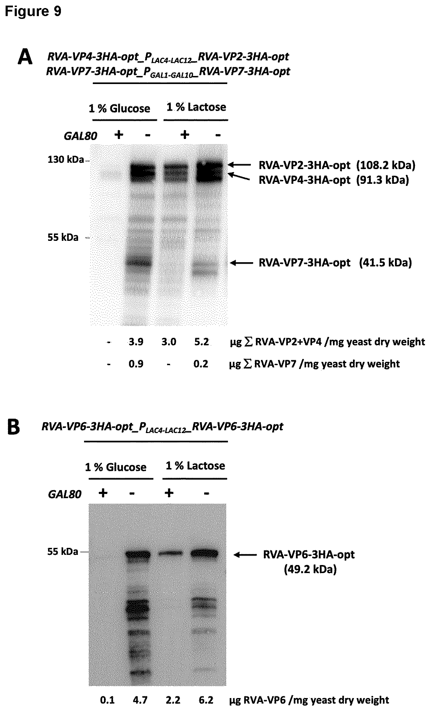

**Figure 9****:** Influence of *GAL80* deletion on recombinant protein expression in *K. marxianus.* Western blot analyses of isogenic yeast strains with (+) and without (-) the chromosomal *GAL80* gene, expressing *PRVA-VP2-3HA-opt, PRVA-VP4-3HA-opt* and *PRVA-VP7-3HA-opt* (A) or *PRVA-VP6-3HA-opt* (B), under control of the bidirectional *KmLAC4-LAC12* promoter after cultivation on glucose and lactose.
0.3 ODU (Optical Density Units) of harvested yeast cultures were loaded onto 4-20% Mini-PROTEAN TGX Stain free gradient gels (Bio-Rad). 1 ODU corresponds to the cell mass, which gives an optical density (OD) of 1 (OD_{600 nm} = 1.0) when resuspended in a volume of 1 ml. The epitope tagged proteins were detected with anti-HA (F-7, sc-7392; 1:3000, Santa Cruz Biotechnology) and HRP-conjugated goat anti-mouse secondary antibody (1:6000, Invitrogen). The antigens are indicated by arrows. Numbers below the gel lanes represent the amount of each antigen (µg per mg yeast dry weight) calculated from the band intensity using an internal standard (MalE) and quantified densitometrically by Imagelab 6.0 software (Biorad).
The deletion of the intact *GAL80* gene (-) results in antigen expression on glucose, to levels that exceed the fully induced levels obtained in lactose medium.

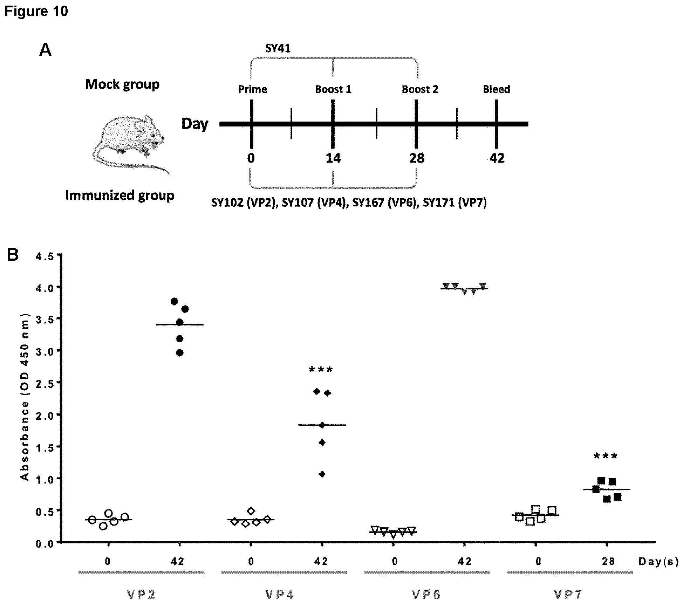

**Figure 10****:** Test of immunogenicity of four *PRVA* antigens individually expressed in yeast strains SY102 (VP2), SY107 (VP4), SY167 (VP6) and SY171 (VP7).
(A) Immunization scheme. Eight weeks old female BALB/c mice were vaccinated subcutaneously three times with either VP2 (SY102), VP4 (SY107) and VP6 (SY167) at day 0, 14 and 28 with 2 to 5 mg yeast dry weight or two times at day 0 and 14 with 2 mg of VP7 (SY171). Control animals received a wildtype strain SY41 without antigen. Sera were collected 14 days after each vaccination for measuring seroconversion.
(B) Mouse sera were analyzed for the presence of *PRVA*-antigen-specific antibody against VP2, VP4, VP6 and VP7 by an IgG ELISA assay. The individual points represent individual absorbance at 450 nm. Data are representative of at least two independent experiments with similar results and average SEM value of five mice (*** = p<0.001). The positive control for the ELISA was a serum of a *PRVA* infected pig, as negative control either zero sera before vaccination (neg. 1) or a non-specific antibody (neg. 2) was used.
After immunization with recombinant haploid yeast cells each of the strains expressing individual *PRVA* antigens elicited an antigen specific immune response.

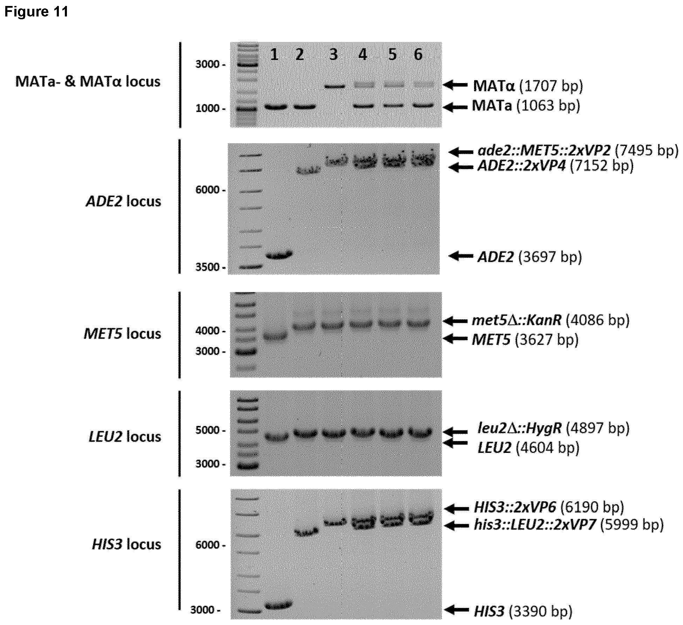

**Figure 11****:** PCR confirmation (agarose gel electrophoresis) of ploidy status of diploid cells. Different primer sets listed in Table 6 were used to confirm the existence of both mating type alleles in the diploid strains and the integration of the transformed DNA cassettes at the expected genomic loci.
Chromosomal yeast DNA of strains (SY41 = lane 1; SY278 = lane 2; SY282 = lane 3; SYD4 = lanes 4-6) was subjected to PCR analysis using specific primer pairs verifying the ploidy state and the presence of different alleles in the haploid and diploid strain background. DNA of SY41 was used as reference for the wild type alleles.
PCR verifies that the diploid strain SYD4 contains genetic elements from both parental haploids.

**Table 1: Overview of PCR samples subjected to agarose gel electrophoresis**

| **Lane** | **Strain** | **MAT** | **Integrations** | ***PRVA* antigens** |
|---|---|---|---|---|
| 1 | SY41 | haploid; MATa | no (*ADE2, MET5, HIS3, LEU2*) | - |
| 2 | SY278 | haploid; MATa | *ADE2::2xVP4, his3::LEU2::2xVP7, met5*Δ::*KanR, leu2*Δ::*HygR, ga180*Δ::*Scura3, ura3-* | 2x VP4, 2x VP7 |
| 3 | SY282 | haploid; MATα | *ade2::MET5::2xVP2, HIS3::2xVP6, met5*Δ*::KanR, leu2Δ::HygR, ga180*Δ*::ScURA3* | 2x VP2, 2x VP6 |
| 4-6 | SYD4 | diploid; MATα / MATa | diploid strains obtained from the mating of SY278 and SY282 | 2x VP2, 2x VP4, 2x VP6, 2x VP7 |

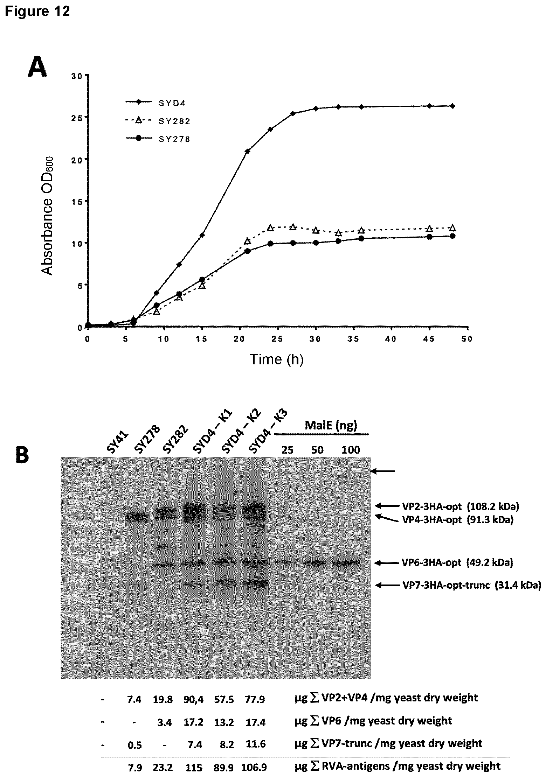

**Figure 12****:** Comparison of growth and recombinant protein production between haploid strains (SY278, SY282) and an isogenic diploid strain resulting from mating of the two haploids (SYD4).
(A) Growth curves for the haploid and diploid yeast strains in SD medium containing 2% glucose. Growth was measured by the optical density at 600 nm. Averages from two independent experiments are shown.
(B) Western blot quantification of VP2, VP4, VP6 and VP7 protein levels in freshly harvested yeast material after 24 h incubation. 0.3 OD (SY41), 0.15 OD (SY278), 0.075 OD (SY282) and 0.019 OD units (SYD4 -K1-K3) of yeast cultures were loaded onto 4-20% Mini-PROTEAN TGX Stain free gradient gels (Bio-Rad). The HA-tagged proteins were detected with anti-HA (F-7, sc-7392; 1:3000, Santa Cruz Biotechnology) and HRP-conjugated goat anti-mouse secondary antibody (1:6000, Invitrogen). *PRVA* antigens are indicated by arrows. The numbers above the gel lanes represent the amount of each antigen (µg per mg yeast dry weight) calculated from the band intensities using an internal standard (MalE) and quantified densitometrically by Imagelab 6.0 software (Biorad) taking into account the loaded OD units.
It is shown that the diploid strain SYD4 expresses higher levels of VP2, VP4, VP6 and VP7 and exhibits a higher growth rate.

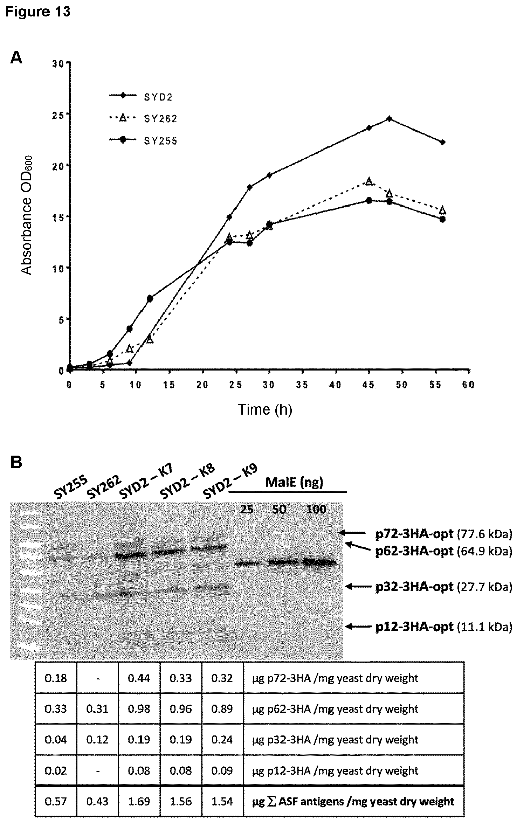

**Figure 13****:** Comparison of growth and recombinant protein production between haploid strains expressing African Swine Fever Virus (ASFV) proteins p12, p32, p62 and p72 (SY255, SY262) and the corresponding isogenic diploid strain SYD2 obtained by mating.
(A) Growth curves for the haploid and diploid yeast strains in SD medium containing 2% glucose. The optical density was measured at 600 nm and values were summed over time and averaged from two independent experiments.
(B) Western blot quantification of p12, p32, p62 and p72 protein levels in freshly harvested yeast material after 24 h incubation. 0.3 OD units of each yeast cultures were loaded onto 4-20% Mini-PROTEAN TGX Stain free gradient gels (Bio-Rad). The epitope tagged proteins were detected with anti-HA (F-7, sc-7392; 1:3000, Santa Cruz Biotechnology) and HRP-conjugated goat anti-mouse secondary antibody (1:6000, Invitrogen). ASFV antigens are indicated with arrows. The numbers above the gel lanes represent the amount of each antigen (µg per mg yeast dry weight) calculated from the band intensities using an internal standard (MalE) and quantified densitometrically by Imagelab 6.0 software (Biorad) taking into account the loaded OD units.
It is shown that the diploid strain SYD2 expresses higher quantities of p12, p32, p62 and p72 and has advantageous growth characteristics.

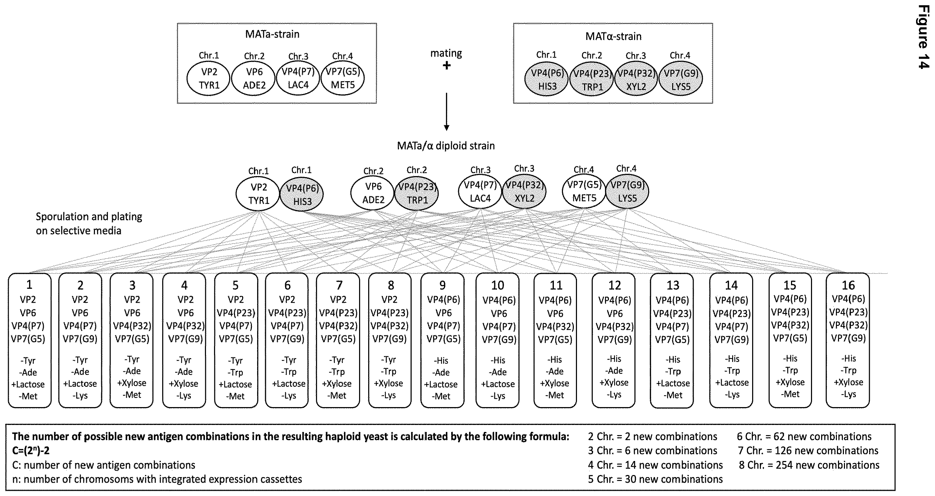

**Figure 14****:** Schematic workflow for the generation of new selectable antigen combinations by mating and sporulation of genetically marked haploid strains.
A haploid strain, in which each of 4 different chromosomes contains a different expression cassette encoding an immunogenic polypeptide coupled to a genetic marker, is crossed with another haploid strain with 4 additional antigen encoding expression cassettes. The genetic markers of the resulting diploid cell linked to the respective antigen encoding expression cassettes are displayed by an oval in the middle. Upon sporulation, the diploid cell undergoes meiosis and each spore inherits a single copy of each chromosome. Since inheritance of the MATa (white) or MATα (grey) copy occurs at random, a set of new combinations of antigen encoding expression cassettes is generated. A desired combination can be easily identified by plating on selective media due to the specific selection markers, linked to each expression cassette. In the schematic example four sets of two homologous chromosomes give 2⁴ =16 combinations, where 2 of them have the genotype of the parental haploids. Hence the number of new combinations is (2⁴)-2=14. In the case of *K. marxianus* having 8 chromosomes 2⁸-2 = 256-2 = 254 new combinations can be generated. The linked marker assures that selection for that marker is equivalent to selection for the linked antigen.

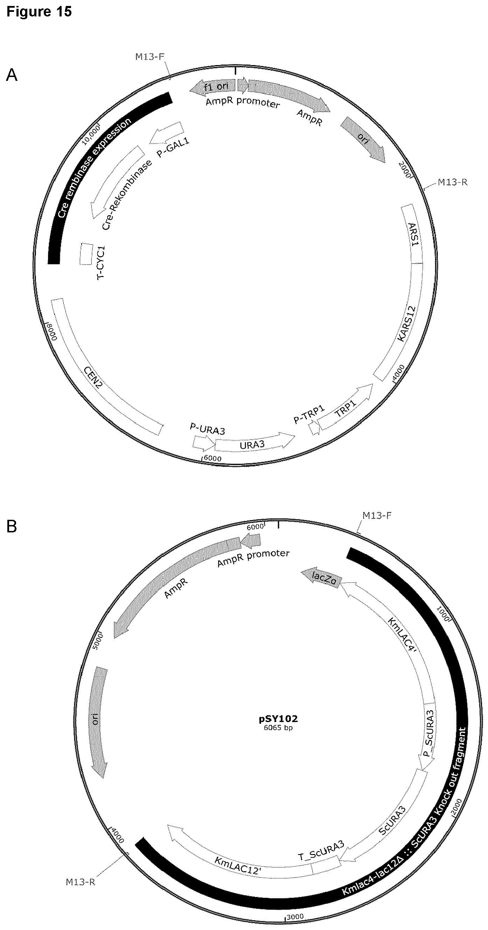

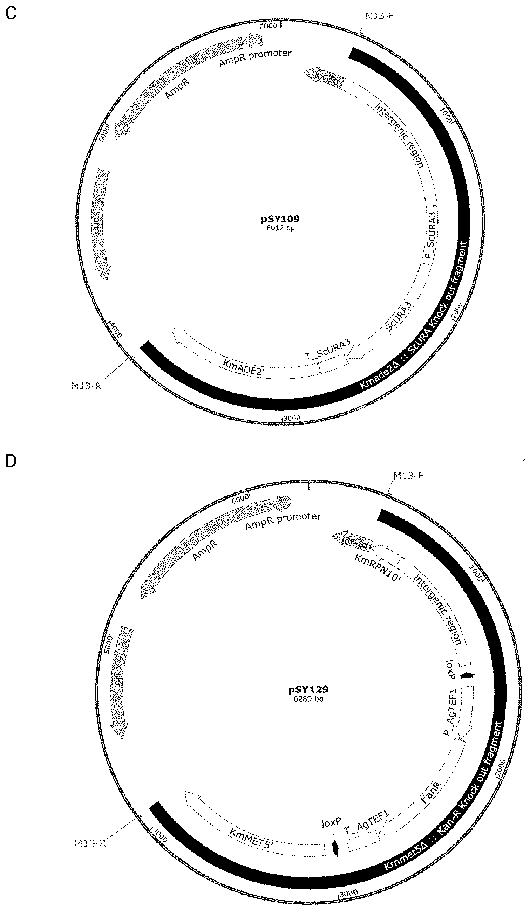

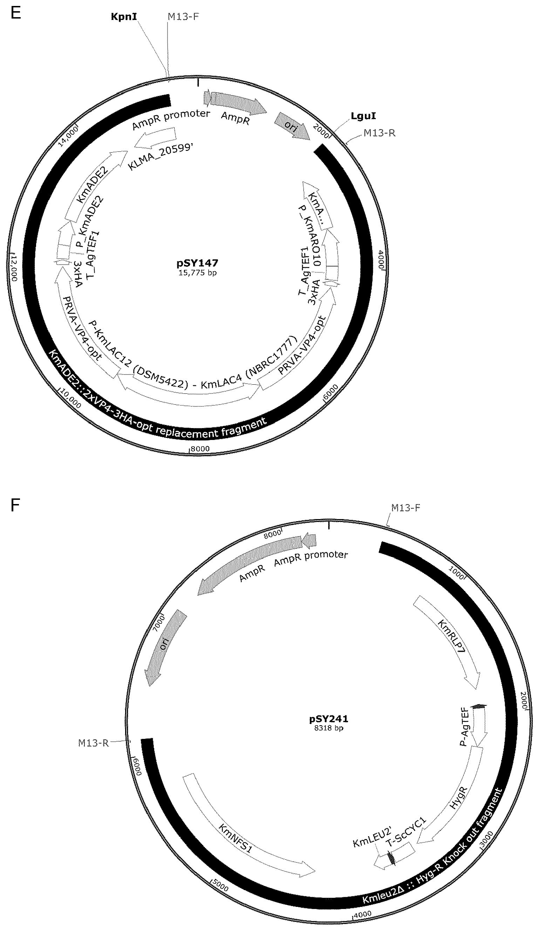

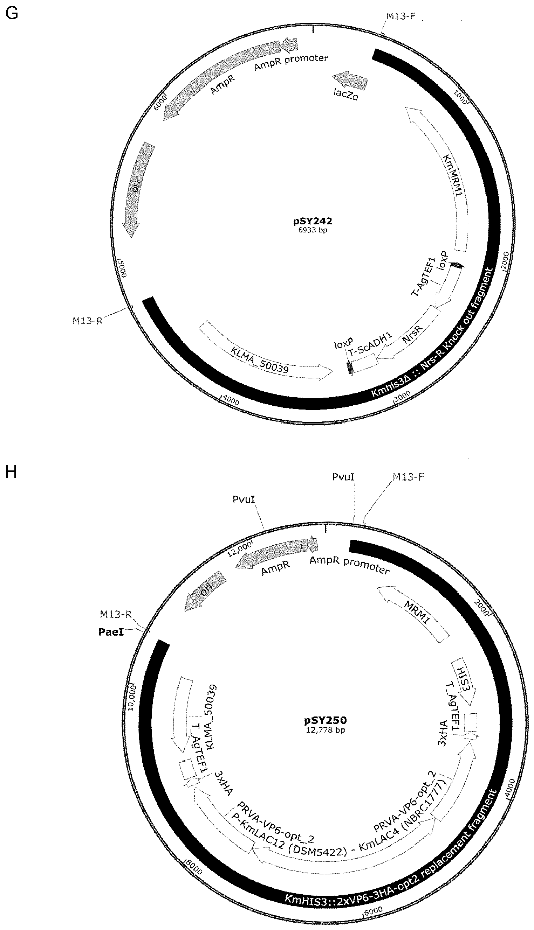

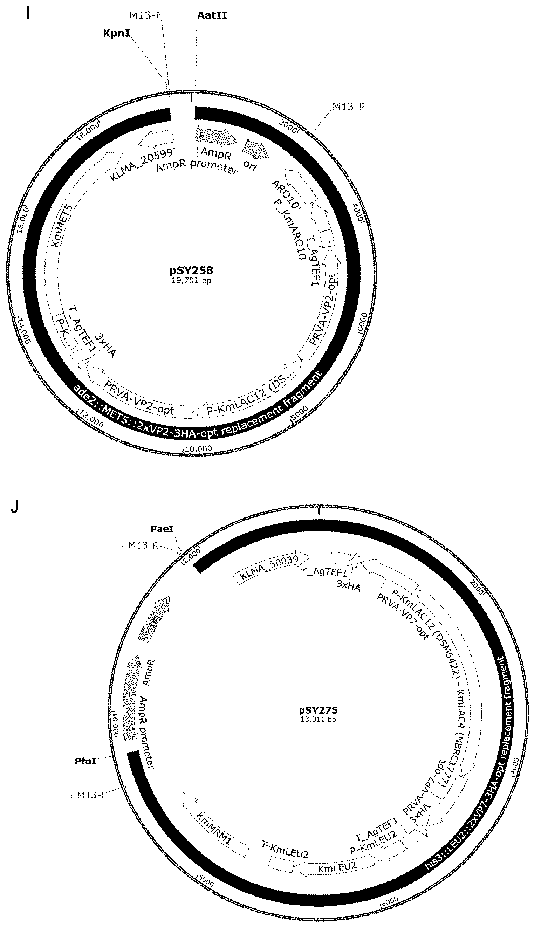

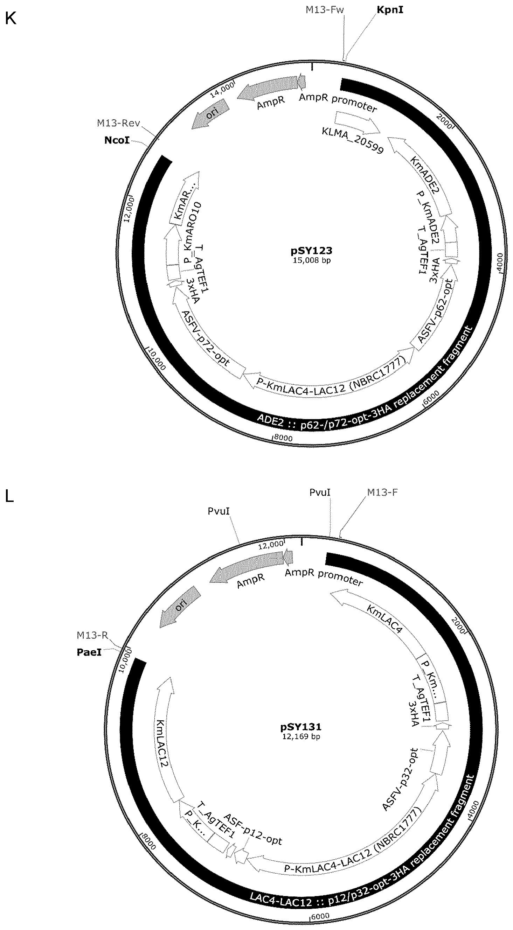

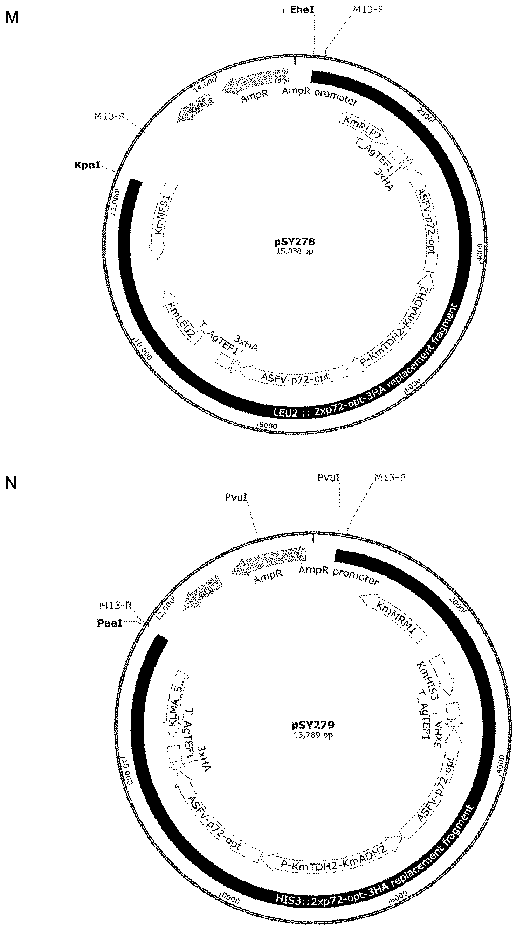

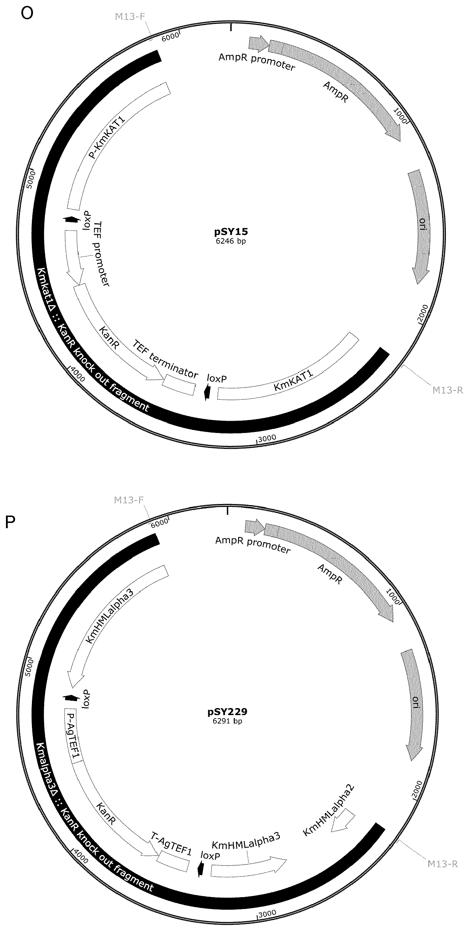

**Figure 15****:** Plasmid maps of plasmids used herein: (A) pSY16, (B) pSY102, (C) pSY109, (D) pSY129, (E) pSY147, (F) pSY241, (G) pSY242, (H) pSY250, (I) pSY258, (J) pSY275, (K) pSY123, (L) pSY131, (M) pSY278, (N) pSY279, (O) pSY15 and (P) pSY229.

### Detailed Description of the Invention

The present invention relates to recombinant yeast cells for highly efficient expression of transgene-encoded gene products, such as immunogenic polypeptides, methods of producing recombinant yeast cells and uses of recombinant yeast cells as vaccines.

A recombinant yeast cell according to the invention comprises at least one genomically integrated expression cassette, wherein each expression cassette comprises (i) a bidirectional promoter element; (ii) a first transgene and a second transgene, wherein said first and second transgene are located at opposite ends of the bidirectional promoter element and wherein each transgene is operably linked to one side of the bidirectional promoter element; (iii) a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene; and (iv) at least one selection marker.

The term "recombinant" as used herein refers to "being prepared by or the result of genetic engineering". Thus, a recombinant microorganism or host cell (e.g., a yeast cell) comprises at least one "recombinant nucleic acid", i.e., a nucleic acid that has been prepared by or is the result of genetic engineering. A recombinant yeast cell specifically comprises an expression cassette that has been stably integrated into the yeast cell genome by means of genetic engineering.

The term "expression cassette" refers to nucleic acid molecules containing desired coding sequences (transgenes) and control sequences in operable linkage, so that host cells transformed with these sequences are capable of producing the polypeptides encoded by the transgenes. In order to obtain genetic stability, the expression cassette is integrated into the host genome (i.e., it is genomically integrated), for example by homologous recombination.

The recombinant yeast cell according to the invention may comprise more than one genomically integrated expression cassette. In one embodiment, recombinant yeast cell comprises at least two genomically integrated expression cassettes. For example, the recombinant yeast cell according to the invention may comprise 2, 3, 4, 5, 6, 7 or 8 genomically integrated expression cassettes. Preferably, the recombinant yeast cell comprises 2, 4 or 6 genomically integrated expression cassettes. In a particularly preferred embodiment, the recombinant yeast cell comprises 2 genomically integrated expression cassettes. In another particularly preferred embodiment, the recombinant yeast cell comprises 4 genomically integrated expression cassettes. In yet another particularly preferred embodiment, the recombinant yeast cell comprises 6 genomically integrated expression cassettes.

The expression cassette according to the invention specifically comprises a bidirectional promoter element, operably linked to coding regions of nucleotide sequences located on both sides of the promoter, in opposite directions, i.e., a first transgene and a second transgene, each under the transcriptional control of said bidirectional promoter element. The bidirectional promoter element is not natively associated with the transgenes.

The term "transgene" as used herein shall refer to any coding gene, e.g. encoding a protein of interest (POI), including polypeptides. The terms "protein" and "polypeptide" are used herein interchangeably. A transgene encodes a protein not naturally occurring in the host cell, i.e. a heterologous protein. The protein of interest can be expressed upon integration by recombinant techniques of one or more copies of the nucleic acid sequence encoding the protein of interest into the genome of the host cell.

In a preferred embodiment, the nucleic acid sequence of the transgene has been codon optimized. The term "codon-optimized" refers to adaption in the design of a coding sequence to the codon usage in the host cell, which often improves the translation efficiency of the protein of interest in the chosen expression system (e.g., the recombinant yeast cell). By virtue of the degeneracy of the genetic code, the translation of a nucleotide sequence into an amino acid sequence is unambiguous whereas for the "reverse translation" of an amino acid sequence into a nucleotide sequence there are several options because some amino acids are encoded by more than one codon. Highly expressed proteins tend to be encoded by codons that make use of abundant tRNA species. Since even closely related organisms may differ in the set of isoaccepting tRNA genes in their genomes, algorithms for codon optimization take advantage of genomic information on the set of isoaccepting tRNA genes and the codon usage in highly expressed genes of the host.

The transgenes may encode various proteins of interest. In one embodiment, each transgene encodes an immunogenic polypeptide derived from a pathogen, or an immunogenic fragment thereof. The pathogen may be a bacterial pathogen, a viral pathogen, a fungal pathogen, a protozoan pathogen, or a multi-cellular parasitic pathogen. Preferably, the pathogen is a viral pathogen. Exemplary viral pathogens include viral pathogens affecting livestock, such as ASFV (African Swine Fever Virus), PPV (Porcine Parvovirus), PRV (Porcine Rotavirus), FMDV (Foot and Mouth Disease Virus), BTV (Bluetongue Virus), PEDV (Porcine Epidemic Diarrhea Virus), PRRSV (Porcine Respiratory and Reproductive Syndrome Virus), PPRV (Peste des Petits Ruminants Virus), RVFV (Rift Valley Fever Virus). Further exemplary viral pathogens include those occurring in aquaculture, such as IPNV (Infectious Pancreatic Necrosis Virus), HSMIV (Heart Skeletal Muscle Inflammation Virus), PDV (Pancreas Disease Virus), ISA (Infectious Salmon Anemic Virus), CyHV-2 (Cyprinid Herpesvirus 2) as well as viral pathogens relevant in companion animals, e.g., AHSV (African Horse Sickness Virus), WNV (West Nile Virus), FIV (Feline Immunodeficiency Virus), EVA (Equine Viral Arteritis). In a particularly advantageous embodiment, the viral pathogen is a viral pathogen with a multilayer capsid and one or more spike proteins.

As used herein, the term "immunogenic" or "immunogenicity" refers to the property of polypeptides, nucleic acids, or other components of a pathogen of raising an immune response in a host cell. A molecular structure (e.g., a polypeptide chain) with a specific shape and/or a short peptide with a specific sequence that is recognized by the immune system (antibodies, B cells and/or T cells) of an animal and elicits an immune response is termed epitope. The immune response of the host organism can be specific or non-specific. In some cases, an immunogenic polypeptide of a pathogen results in both, a non-specific and a specific immune response. Preferably, the immune response comprises an immune response that is specific for the immunogenic polypeptide, or immunogenic polypeptides that are encoded by each transgene comprised in the at least one genomically integrated expression cassette. Further preferably, the immune response is protective against the pathogen from which the immunogenic polypeptide has been derived. An immune response is protective if it prevents or attenuates a disease or condition arising from infection of a host organism or patient with the pathogen, or pathogens that an immunogenic polypeptide has been derived from.

In some embodiments, the transgenes encode immunogenic polypeptides derived from more than one pathogen, or an immunogenic fragment thereof. In one embodiment, the transgenes encode immunogenic polypeptides derived from more than one strain of the same viral pathogen, or an immunogenic fragment thereof. In another embodiment, the transgenes encode immunogenic polypeptides derived from more than one viral pathogen, or an immunogenic fragment thereof.

Apart from the native coding sequence and/or a codon optimized variant thereof, the transgenes may encode further variants of the native immunogenic polypeptide. The variants of the native immunogenic polypeptide essentially retain or increase the immunogenicity of the native form of the immunogenic polypeptide. Such variants include amino acid (aa) substitutions, additions or deletions. The coding DNA sequence can be designed by reverse translation and is introduced into an expression cassette as a synthetic piece of DNA or by modifying the DNA of an existing genetic locus. Generally, the modification may serve a function including but not restricted to elimination of a post-transcriptional modification site (e.g. glycosylation), prevention of misfolding or aggregation, as well as enhancement of immunogenicity. Variant immunogenic polypeptides also include fragments of the polypeptides disclosed herein, particularly immunogenic fragments. Also encompassed are immunogenic polypeptides that have been modified to improve resistance to proteolytic degradation, increase solubility or render them more suitable as an immunogen, or antigenic component of a vaccine composition. Further variants include fusion polypeptides comprising an immunogenic polypeptide and a functional fragment changing the intracellular localization of the fusion polypeptide, or an epitope tag.

In a specific embodiment, the pathogen is a viral pathogen that belongs to the family of *Reoviridae.* Preferably, the viral pathogen is a rotavirus. For example, rotavirus A, B, C, D, E, F, G, H or I. More preferably, the viral pathogen is a Porcine Rotavirus, such as Porcine Rotavirus A, B or C. Even more preferably, the viral pathogen is Porcine Rotavirus A (PRVA) or Porcine Rotavirus C (PRVC), most preferably Porcine Rotavirus A (PRVA). In another preferred embodiment, the viral pathogen is an orbivirus, most preferably African Horse Sickness Virus (AHSV) or Bluetongue virus (BTV). In yet another preferred embodiment, the viral pathogen is Porcine Parvovirus (PPV).

In a particularly preferred embodiment, each immunogenic polypeptide comprises a polypeptide selected from the group consisting of Porcine Rotavirus A (PRVA) VP2, VP4, VP6, VP7, NSP2 and NSP4 and one or more immunogenic fragment thereof, most preferably from the group consisting of Porcine Rotavirus A (PRVA) VP2, VP4, VP6 and VP7 and one or more immunogenic fragment thereof. In a specific example, each immunogenic polypeptide is derived from Porcine Rotavirus A strain RVA/Pig-tc/ESP/OSU-C5111/2010/G5P[7]. For example, each immunogenic polypeptide comprises a polypeptide selected from the group consisting of SEQ ID NO: 1 (PRVA VP2), SEQ ID NO: 2 (PRVA VP4), SEQ ID NO: 3 (PRVA VP6), SEQ ID NO: 4 (PRVA VP7), SEQ ID NO: 5 (PRVA NSP2) and SEQ ID NO: 6 (PRVA NSP4) and one or more immunogenic fragment thereof, preferably from the group consisting of SEQ ID NOs: 1, 2, 3, and 4 and one or more immunogenic fragment thereof.

The nucleotide sequences encoding PRVA VP2, VP4, VP6, VP7, NSP2 and NSP4 are set forth in SEQ ID NO: 7 (PRVA VP2, GenBank accession number: KJ450843.1), SEQ ID NO: 8 (PRVA VP4, GenBank accession number: KJ450845.1), SEQ ID NO: 9 (PRVA VP6, GenBank accession number: KJ450847.1), SEQ ID NO: 10 (PRVA VP7, GenBank accession number: KJ450849.1), SEQ ID NO: 11 (PRVA NSP2, GenBank accession number: KJ450850.1) and SEQ ID NO: 12 (PRVA NSP4, GenBank accession number: KJ450851.1).

Advantageously, the nucleotide sequences are codon optimized for expression in yeast. Preferred codon optimized nucleotide sequences encoding PRVA VP2, VP4, VP6, VP7, NSP2 and NSP4, are set forth in SEQ ID NO: 13 (PRVA VP2), SEQ ID NO: 14 (PRVA VP4), SEQ ID NO: 15 (PRVA VP6), SEQ ID NO: 16 (PRVA VP7), SEQ ID NO: 17 (PRVA NSP2) and SEQ ID NO: 18 (PRVA NSP4). Optionally, the immunogenic polypeptides comprise a peptide tag, such as an HA tag. Exemplary amino acid sequences, each comprising an HA tag are set forth in SEQ ID NO: 19 (PRVA VP2), SEQ ID NO: 20 (PRVA VP4), SEQ ID NO: 21 (PRVA VP6), SEQ ID NO: 22 (PRVA VP7), SEQ ID NO: 23 (PRVA NSP2) and SEQ ID NO: 24 (PRVA NSP4).

Thus, in a particularly preferred embodiment, each immunogenic polypeptide comprises a polypeptide selected from the group consisting of Porcine Rotavirus A (PRVA) VP2, VP4, VP6 and VP7 and one or more immunogenic fragment thereof, wherein the nucleotide sequences encoding Porcine Rotavirus A (PRVA) VP2, VP4, VP6 and VP7 are set forth in SEQ ID NO: 13 (PRVA VP2), SEQ ID NO: 14 (PRVA VP4), SEQ ID NO: 15 (PRVA VP6) and SEQ ID NO: 16 (PRVA VP7). In a highly preferred embodiment, each immunogenic polypeptide further comprises an HA tag and consists of an amino acid sequence selected from the group consisting of SEQ ID NO: 19 (PRVA VP2), SEQ ID NO: 20 (PRVA VP4), SEQ ID NO: 21 (PRVA VP6) and SEQ ID NO: 22 (PRVA VP7).

In one embodiment, the recombinant yeast cell comprises two genomically integrated expression cassettes, wherein the first and second transgene of each expression cassette encode one immunogenic polypeptide derived from a pathogen. In a further embodiment, the recombinant yeast cell comprises three genomically integrated expression cassettes, wherein the first and second transgene of each expression cassette encode one immunogenic polypeptide derived from a pathogen. In an even further embodiment, the recombinant yeast cell comprises four genomically integrated expression cassettes, wherein the first and second transgene of each expression cassette encode one immunogenic polypeptide derived from a pathogen.

Each transgene may encode an immunogenic polypeptide (or immunogenic fragment thereof) that is derived from a viral pathogen (e.g., PRVA). In one embodiment, the immunogenic polypeptide, or polypeptides, are capable of assembling into a virus-like particle (VLP) inside the recombinant yeast cell. This means that the immunogenic polypeptide(s) is/are a structural component of the capsid of a viral pathogen and thus capable of taking part in the assembly of a VLP, optionally in combination with further immunogenic polypeptides. Said further immunogenic polypeptides may be derived from the same viral pathogen, or from a further viral pathogen that is closely related with the first viral pathogen. In the case of viral pathogens with a simple symmetric capsid, one major structural protein of the virus can form virus-like particles (VLPs) when expressed recombinantly in a yeast cell.

The homo- or hetero-multimerization of one or more structural proteins into complex VLPs inside a recombinant yeast was found to be highly beneficial for the development of whole yeast vaccines. For structurally simple viral pathogens, where the capsid is composed of a single layer of a single structural protein, it has been confirmed that the immune response of the host organism is substantially enhanced when the structural protein has assembled into VLPs. However, the capsid structure of many viruses is composed of more than one protein in more than one layer, and more than one of these structural proteins possesses individual immunogenic properties.

Rotavirus has a complex triple-layered capsid structure composed of VP2 forming the innermost layer, VP6 forming the middle layer and VP4 and VP7 as parts of the outer layer. Co-expression of three of these antigens, VP2, VP6 and VP7, in *Saccharomyces cerevisiae* has been shown to yield rotavirus-VLPs (RLPs). However, the antigens were expressed from one or more plasmid(s), which is cumbersome and leads to low and unpredictable antigen expression.

A prerequisite for VLP formation from a single immunogenic polypeptide in yeast is the ability of that polypeptide to associate spontaneously into an essentially regular geometrical structure. One example is the capsid protein of parvovirus. Generally, many viral capsid structures are more complex (e.g., the capsid of rotavirus). They can form multimeric protein complexes composed of several different structural polypeptides, optionally associated with non-structural proteins that may or may not be immunogenic. The possibility to form multi-component VLPs in yeast has been shown to require the coordinate expression of several transgenes encoding the required polypeptides in a single cell. Co-expression is greatly facilitated by employing the bidirectional promoter elements and genomically integrated expression cassettes according to the invention. The invention enables accumulation of the VLP components at high concentrations in the recombinant yeast cell, a prerequisite for efficient assembly of the often highly immunogenic VLP particles.

Virus Like Particles (VLPs) generally contain one or more proteins from a virus optionally combined with a phospholipid. VLPs are generally non-pathogenic, non-replicating and generally do not contain any of the native viral genome. Since VLPs cannot replicate, they provide a safer alternative to attenuated viruses for vaccine development or vaccination. The VLPs may be produced in the recombinant yeast cell of the invention. Due to their virosome-like structure, VLPs contain repetitive, high density displays of viral surface proteins that present conformational viral epitopes which can elicit strong T cell and B cell immune responses. The small radius of common VLPs (generally 20-200 nm) allows for sufficient draining into lymph nodes.

The recombinant yeast cell comprises at least one genomically integrated expression cassette, wherein each expression cassette comprises a first and a second transgene. Each expression cassette may comprise different combinations of transgenes. In one embodiment, the first transgene and the second transgene are identical within each expression cassette. In another embodiment, the first transgene and the second transgene are different within each expression cassette.

The recombinant yeast cell may comprise one genomically integrated expression cassette, or more than one genomically integrated expression cassette. In one embodiment, the recombinant yeast cell comprises at least two genomically integrated expression cassettes (e.g., 2, 3, 4, 5, 6, 7 or 8 genomically integrated expression cassettes) and the first transgene and the second transgene of each expression cassette are different from the first and second transgene of each further expression cassette. Thus, in a specific embodiment of the invention, the first transgene and the second transgene of each expression cassette are different from the first and second transgene of each further expression cassette and the first transgene and the second transgene are identical within each expression cassette. In another specific embodiment, the first transgene and the second transgene of each expression cassette are different from the first and second transgene of each further expression cassette and the first transgene and the second transgene are different within each expression cassette.

To date, the choice of promoters for heterologous gene expression, especially for eukaryotic organisms, such as yeast, has focussed on monodirectional promoters. The assembly of VLPs or the coverage of diverging serotypes/genotypes of pathogenic viruses often requires the simultaneous expression of numerous immunogenic polypeptides and variants thereof in one (yeast) cell. Classically, monodirectional promoters have been used for such applications, but monodirectional promoters are limited in terms of multi-gene co-expression capabilities. Furthermore, each individual chromosomal integration of an insert comprising a monodirectional promoter and one transgene requires (and "consumes") one selection marker, with only a limited number of such marker genes being available. The use of bidirectional promoter elements necessitates only one selection marker gene per pair of transgenes.

Additionally, bidirectional promoter elements offer the ability to double the number of transgenes inserted into the genome of a host cell in one transformation step, which reduces the effort for verification of the correct recombination events significantly. The bidirectional promoter elements allow the coordinate expression of two transgenes, and thus of two heterologous proteins from one control region. A major advantage of using the bidirectional promoters is a shielding effect against influences of flanking sequences on promoter activity. The chromatin of promoter elements is generally more open allowing access of DNA binding proteins whereas protein coding sequences of a transcription unit are generally organised in a regular nucleosome array. Fusing a promoter element to a region with extremely open or closed chromatin structure runs the risk of having an unwanted influence of the flanking region on the activity of the selected promoter element. This well documented phenomenon of insertion site specific influence on the expression of genes randomly integrated into the eukaryotic genome is reduced, at least in yeast, when protein coding sequences are flanking both sides of a bidirectional promoter. In addition, bidirectional promoters are also reducing the cloning efforts for expression cassettes.

Moreover, mating haploid recombinant yeast cells comprising such expression cassettes at different selected positions in the yeast genome substantially speeds up the development of multivalent whole yeast vaccines, since the number of antigens increases by the factor two with each integration.

For example, a haploid recombinant yeast cell according to the invention, comprising two genomically integrated expression cassettes can be generated through only two transformation steps. This recombinant yeast cell may encode up to four different transgenes, e.g., four different immunogenic polypeptides. Mating of said haploid recombinant yeast cell with a further haploid recombinant yeast cell, which also comprises two expression cassettes, each comprising two transgenes would result in a diploid recombinant yeast cell comprising eight transgenes, encoding up to eight different immunogenic polypeptides.

Generally, a bidirectional promoter element is located within the intergenic region between the 5' ends of a bidirectional gene pair. A "bidirectional gene pair" refers to two adjacent and divergently (as opposed to convergently) transcribed genes encoded on opposite strands. Natively, the two genes are often functionally related, and their shared upstream region allows them to be co-regulated and thus co-expressed. Bidirectional promoter elements enable efficient and controllable expression of two transgenes located at opposite ends of the bidirectional promoter element wherein each transgene is operably linked to one side of the bidirectional promoter element.

Generally, the term "promoter element" as used herein refers to a DNA sequence capable of controlling the expression of a protein coding sequence. It consists of a segment that determines the start site(s) and the direction of transcription for each transcription unit and additional elements that contribute to control the rate of transcription and thus the expression level of a gene operably linked to the promoter by serving as binding sites for regulatory factors. Such regulatory factors influence the promoter activity in response to extracellular or intracellular conditions. As used herein, the terms "transcription rate" and "transcription level" are used interchangeably. Regulatory sequences of bidirectional promoters can cooperate and act on the two core promoter elements driving divergent transcription, as in the case of the *GAL1-GAL10* or *LAC4-LAC12* promoters or act unidirectionally on only one of the two core promoters. These different types of regulatory elements can co-exist in the same bidirectional promoter element. In the expression cassettes constructed in this invention the fusion point for the protein coding region of the transgene and the promoter element is at or close to the translation start codon.

The term "operably linked" (or "*cis*-acting") as used herein refers to the association of nucleotide sequences on a single nucleic acid molecule, e.g. an expression cassette or a vector, in a way such that the function of one or more nucleotide sequences is affected by at least one other nucleotide sequence present on said nucleic acid molecule. For example, a promoter element is operably linked with a coding sequence of a transgene, when it is capable of effecting the expression of that coding sequence. Operable linking is accomplished by ligation of polynucleotides at convenient restriction sites or by PCR and recombination methods familiar to those skilled in the art, that allow seamless fusion of DNA fragments, such as In-Fusion Cloning (Zhu, Baogong; Cai, Guifang; Hall, Emily O.; Freeman, Gordon J. (2007), BioTechniques 43 (3), S. 354-359).

Since the core eukaryotic transcription apparatus is conserved, promoter DNA and coding DNA may be from the same or from different organisms. Preferably, the bidirectional promoter element comprised in the genomically integrated expression cassette according to the invention is a native bidirectional yeast promoter element. This means that the bidirectional promoter element naturally or natively occurs as a bidirectional promoter element that controls transcription of a homologous gene pair in yeast. In a specific embodiment, the native bidirectional yeast promoter element is evolutionarily conserved in at least three divergently evolved yeast genera (Byrne, K. P. (2005), Genome Research 15 (10), p. 1456-1461; see yeast gene order browser, http://ygob.ucd.ie). In a further specific embodiment, the native bidirectional yeast promoter element is derived from the yeast genus *Kluyveromyces,* more specifically from the yeast species *Kluyveromyces marxianus.*

In an advantageous embodiment, the native bidirectional yeast promoter element is selected from the group consisting of a *LAC4*/*LAC12* promoter, a *GAL1*/*GAL10* promoter and a *GAP1*/*ADH2* promoter.

Further advantageous native bidirectional yeast promoter elements are listed in Table 2 (Erirksson, Peter R.; Ganguli, D.; Nagarjavel, V.; Clark, David J. (2012), Genetics 191(1):7-20; Nishizawa, M; Komai, T. et al. (2008), Eukaryotic Cell 7(6), 949-957; Hu, Haiyan; Li, Xiaoman (2007), Genomics 90(4), 421-423).

**Table 2: Preferred native bidirectional yeast promoter elements.**

| **Promoter** | **Organism** | **Class** | **Induction** | **Repression** |
|---|---|---|---|---|
| *P_{LAC4-LAC12}* | *K. marxianus* | regulated | Galactose / Lactose | Glucose |
| *P_{LAC4-LAC12}* | *K. lactis* | | | |
| *P_{GAL1-GAL10}* | *K. marxianus* | | galactose | Glucose |
| *P_{GAL1-GAL10}* | *K. lactis* | regulated | | |
| *P_{GAL1-GAL10}* | *S. cerevisiae* | | | |
| *P_{GAP1-ADH2}* | *K. marxianus* | *P_{GAP1}* - constitutive | No | |
| | | *P_{ADH2}*-regulated | Ethanol-& stationary phase-dependent regulated- | |
| *P_{HTA1-HTB1}* | *K. marxianus* | regulated | cell cycle regulated | |
| *P_{HTA1-HTB1}* | *K. lactis* | | | |
| *P_{HTA1-HTB1}* | *S. cerevisiae* | | | |
| *P_{HHT1-HHF1}* | *K. marxianus* | regulated | cell cycle regulated | |
| *P_{HHT1-HHF1}* | *K. lactis* | | | |
| *P_{HHT1-HHF1}* | *S. cerevisiae* | | | |
| *P_{HHT2-HHF2}* | *K. marxianus* | regulated | cell cycle regulated | |
| *P_{HHT2-HHF2}* | *K. lactis* | | | |
| *P_{HHT2-HHF2}* | *S. cerevisiae* | | | |
| *P_{SNO3-SNZ3}* | *K. marxianus* | regulated | stationary phase-induced | |
| *P_{SNO1-SNZ1}* | *K. lactis* | | | |
| *P_{SNO1-SNZ1}* | *S. cerevisiae* | | | |
| *P_{RPL15B-KLMA_40078}* | *K. marxianus* | regulated | various cellular states-regulated | |
| *P_{RPL15A-KLLA0_F17611g}* | *K. lactis* | | | |
| *P_{RPL15A-YLR030w}* | *S. cerevisiae* | | | |

These native bidirectional yeast promoter elements include the *GAL1-GAL10* (*P_{GAL1-GAL10}*) promoter element (in short: *GAL* promoter) of *S. cerevisiae* (nucleotides 278744 to 279411 of the sequence according to NCBI accession number CP020124), *K. lactis* (nucleotides 781010 to 781507 of the sequence according to NCBI accession number CR382126) and *K. marxianus* (SEQ ID NO: 25) and the *LAC4-LAC12* (*P_{LAC4-LAC12}*) promoter element (in short: *LAC* promoter) of *K. lactis* (nucleotides 1307691 to 1310500 of the sequence according to NCBI accession number CR382122) and *K. marxianus* (SEQ ID NO: 26), which are regulated by the transcription activator Gal4p. In a preferred embodiment, the native bidirectional yeast promoter element comprises a nucleic acid sequence selected from the group consisting of SEQ ID NOs 25 and 26.

The *GAL* and *LAC* genes that are naturally regulated by these promoter elements are required for the metabolism of the sugars galactose and lactose (a galactose-glucose disaccharide). The consensus sequence for the binding site (5'-CGGnnnA/TnnnCCG-3') within the *GAL* and *LAC* bidirectional promoter elements is conserved, indicating that the specificity of the DNA-binding domain of the homologous Gal4p variants, e.g., ScGal4p, KIGal4p (also named Lac9) and KmGal4, is also conserved. The expression of the native gene pairs under the control of the *GAL1-GAL10* and *LAC4-LAC12* promoter elements is co-ordinately regulated by the Gal4p-regulated bidirectional promoters and can be induced by supplementation of the growth medium with galactose or lactose. Likewise, two (or four) transgenes linked to these bidirectional promoter elements can be co-ordinately regulated by Gal4p

In glucose medium, the *LAC* and *GAL* promoter elements are silent, which is beneficial when the antigen expression leads to protein-stress in the recombinant yeast cell. Accordingly, recombinant yeast cells can first be cultivated on glucose medium for biomass accumulation before lactose is added to activate the *LAC4-LAC12* promoter and transgene expression is induced. Transcriptional repression of the *LAC* and *GAL* promoter elements requires the regulatory protein Gal80p, which can block the transcription activation function of Gal4p by direct protein-protein interaction. The regulatory network regulating the *LAC*/*GAL* promoters operates one of the most effective eukaryotic transcriptional switches known. On the one hand Gal4p controlled gene expression can be modulated by a small nutrient, galactose, on the other hand it can be modulated by the ratio of its regulators Gal80p and Gal4p. The inventors have surprisingly found that, when transgenes are linked to the bidirectional *LAC* and *GAL* promoter elements, the intracellular concentration of multiple transgene-encoded proteins, such as PRVA antigens, can be elevated substantially even beyond the fully induced levels upon deletion of the *KmGAL80* gene.

Thus, in a particularly advantageous embodiment, the bidirectional promoter element is selected from a *LAC4-LAC12* promoter or a *GAL1-GAL10* promoter and the recombinant yeast cell comprises a deletion and/or disruption of *GAL80.* If the recombinant yeast cell comprises more than one genomically integrated expression cassette (i.e., at least two genomically integrated expression cassettes), each cassette may comprise a *LAC4-LAC12* promoter, each cassette may comprise a *GAL1-GAL10* promoter, or the expression cassettes may comprise either a *LAC4-LAC12* or a *GAL1-GAL10* promoter element and the recombinant yeast cell comprises a deletion and/or disruption of *GAL80.*

Also encompassed by the invention are native bidirectional yeast promoter elements that have been adapted to increase transgene expression, or to enable expression control using a different stimulus or to give high constitutive promoter activity (adapted native bidirectional yeast promoter elements).

In a specific embodiment, the bidirectional promoter element is a hybrid bidirectional promoter element. Such hybrid promoter elements are composed of sections from different monodirectional native (natural) yeast or heterologous promoter elements, preferably from different monodirectional natural yeast promoter elements. Said monodirectional promoter elements may natively be part of a bidirectional promoter element. Both monodirectional promoter elements are selected such that they do not interfere with each other. The different monodirectional promoter elements are fused in head-to-head fashion, to control bidirectional transcription of the linked genes. For example, hybrid promoter elements for use in *S. cerevisiae* (and potentially other yeast species) can be constructed by combining heterologous regulatory promoter motifs with homologous core promoters (Hubmann, Georg; Thevelein, Johan M.; Nevoigt, Elke (2014), Methods in molecular biology (Clifton, N.J.) 1152, S. 17-42). Efficient and robust recombinant protein expression by hybrid-regulated bidirectional promoter elements has already been described in the literature for some yeast species, e.g., *P_{TDH3-ADH1}* (Miller, C. A.; Martinat, M. A.; Hyman, L. E. (1998), Nucleic acids research 26 (15), S. 3577-3583); *P_{GAL1 or GAL10-GPD}* (Li, Aimin; Liu, Zengshan; Li, Qianxue; Yu, Lu; Wang, Dacheng; Deng, Xuming (2008), FEMS yeast research 8 (1), S. 6-9); *P_{TEF1-PGK1}* (Partow, Siavash; Siewers, Verena; Bjørn, Sara; Nielsen, Jens; Maury, Jérôme (2010), Yeast (Chichester, England) 27 (11), S. 955-964). Thus, in a preferred embodiment, the hybrid bidirectional promoter element is selected from the group consisting of a *TDH3*/*ADH1* promoter, a *GAL1*/*GAL10-GPD* promoter and a *TEF1*/*PGK1* promoter.

In *K. marxianus,* the sequence of the *LAC4-LAC12* promoter may differ between strains. Thus, a hybrid bidirectional promoter element may comprise monodirectional promoter elements from different yeast strains, in particular different strains of *K. marxianus.* For example, the hybrid bidirectional promoter element may comprise the nucleic acid sequence set forth in SEQ ID NO: 27. This particular hybrid bidirectional promoter element was obtained by fusing the *LAC12* proximal part of the *LAC4-LAC12* promoter of *K. marxianus* strain DSM5422 with the *LAC4* proximal part of *K. marxianus* strain NBRC1777.

In a further specific embodiment, the hybrid bidirectional promoter element is a bidirectional *GAP1-ADH2* (e.g., *KmGAP1-ADH2,* SEQ ID NO: 28) promoter (*P_{GAP1-ADH2}*), which has the advantage that it is not shut down when the recombinant yeast cell enters a stationary phase. This property is highly advantageous for the accumulation of one or more transgene-expressed polypeptides, such as immunogenic polypeptides in a recombinant yeast cell. The *GAP1-ADH2* bidirectional promoter element is regulated by the nitrogen source available in the culture medium. The strongest inducing effect has been observed for proline as the nitrogen source.

Thus, in a particularly preferred embodiment, the hybrid bidirectional promoter element comprises a nucleic acid sequence selected from the group consisting of SEQ ID NOs: 27 and 28.

In a further embodiment, the hybrid bidirectional promoter element is composed of two monodirectional promoter elements of different transcriptional strength and/or regulation which is retained upon fusion into a hybrid bidirectional promoter element. Hence, the rate of divergent transcription of the first and the second transgene can be individually regulated giving high/low expression levels for the first or the second transgene or for both transgenes depending on the conditions of cultivation.

If a transgene encodes a gene product that is toxic for the recombinant yeast cell, it is advantageous to separate cell growth and expression of the transgene-encoded polypeptide. For example, a transgene encoding a toxic gene product is placed under the control of an inducible/repressible promoter element and kept repressed in the initial phase of fermentation and is derepressed in the late phase after biomass has accumulated and growth inhibition by the toxic transgene product is irrelevant.

Thus, the hybrid bidirectional promoter element may comprise one inducible or repressible monodirectional promoter element and one constitutive monodirectional promoter element. For example, the inducible or repressible part of the hybrid bidirectional promoter element is selected from the group consisting of a *GAL1, GAL10, GAL80, LAC4, LAC12, ADH2, CUP1, DAN1 and MET3*/*MET17* promoter element. The constitutive part of the hybrid bidirectional promoter element may be selected from the group consisting of a *GAP1, GPD1, TEF1, PGK1, ACT1, HXT7, TDH2*/*TDH3, ADH1, TPI1 and PYK1* promoter element.

A hybrid bidirectional promoter element may also be a synthetic promoter element comprising combined sequence motifs from known promoter elements, wherein the spacer regions are filled with random sequences (Hubmann, Georg; Thevelein, Johan M.; Nevoigt, Elke (2014), Methods in molecular biology (Clifton, N.J.) 1152, S. 17-42).

Promoter activity is typically assessed by its transcriptional efficiency, or expression level. A reporter gene assay can be used, wherein the promoter is fused to a DNA sequence (transgene) encoding a reporter protein that can easily be quantified (e.g., β-galactosidase or Green Fluorescent Protein (GFP)). Reporter protein levels, measured in the form enzymatic activity in the case of β-galactosidase or fluorescence intensity in the case of fluorescent proteins, such as GFP, serve as readout for promoter activity. More directly, promoter activity can be assessed by quantitation of the (steady-state) RNA level (expression level) of the functionally linked transgene, e.g. via quantitative reverse transcription PCR (qRT-PCR). Since the number of transcription initiation events per time unit can usually not be measured, promoter strength is commonly expressed as RNA level in relation to a given standard RNA with a long halve life. The expression level analysis may be qualitative, quantitative or semi-quantitative, e.g. employing a microarray, Northern blot, RNA sequencing or qRT-PCR. To quantity the gene expression of a given transgene, it is usually sufficient to determine the level of the protein product (e.g., by Western Blot), instead of the mRNA level, because generally the protein level (e. g. level of an immunogenic polypeptide) is the relevant parameter for process optimization.

The bidirectional promoter element may enable similar, or essentially identical expression levels of the first and second transgene it is operably linked to. Alternatively, the bidirectional promoter element may enable different expression levels of the first and second transgene it is operably linked to. Thus, in one embodiment, the bidirectional promoter element enables similar expression levels of the first transgene and the second transgene. In another embodiment, the bidirectional promoter element enables different expression levels of the first transgene and the second transgene.

As used herein, "similar expression levels" means that the expression level of a first transgene deviates from the expression level of a second transgene by 30% or less, 20% or less, 10% or less or 5% or less, preferably by 20% or less. "Different expression levels" means that the expression level of a first transgene deviates from the expression level of a second transgene by at least 50%, at least 60%, at least 70%, at least 80%, at least 90% or at least 100%, preferably by at least 50%. The expression level can be determined by qRT-PCR, Western blot or reporter gene assay as readout, preferably by qRT-PCR or reporter gene assay using a reporter protein. Notably, in *Kluyveromyces spp.* the endogenous β-galactosidase gene *LAC4* interferes with the use of β-galactosidase as reporter protein.

Promoter elements generally fall into several functional classes: constitutive or regulated, i.e., inducible or repressible promoter elements. Repressible promoter elements increase levels of transcription in response to the absence of a repressor, inducible promoters decrease levels of transcription in the absence of an activator or in response to some change in culture conditions, that render the respective activator inactive.

Accordingly, the bidirectional promoter element according to the invention may enable constitutive or regulated expression of the first and second transgene it is operably linked to. Regulated expression refers to expression that is inducible or repressible (e.g., in response to a stimulus), preferably inducible (e.g., by addition of an inducing factor). The sequences regulating the transcription in one and the other direction of the bidirectional promoter element may be the same, or they may be different in sequence, structure and function, such as promoter sequences of different transcriptional activity or strength, e.g. to obtain different transcription or expression levels and a specific transcription or expression ratio of the first and second transgene. Further, the sequences regulating the transcription in one and the other direction of the bidirectional promoter element may be differently regulated with a specific regulatory profile.

In the case of a toxic gene product, encoded by a transgene, it is preferred to initially keep the transgene repressed under the control of a regulated (e.g., inducible) promoter to allow for biomass accumulation and to induce gene expression only at the end of the fermentation by activating or derepressing the promoter. Genes required for cell growth and proliferation are generally under the control of constitutive promoters that are active right from the start of the fermentation.

Thus, in one embodiment, the bidirectional promoter element enables inducible or repressible expression of the first or the second transgene. In a further embodiment, the bidirectional promoter element enables inducible or repressible expression of the first and the second transgene. In a specific embodiment, the bidirectional promoter element enables inducible or repressible expression of the first and the second transgene, wherein the inducer or repressor of promoter activity for the first and the second transgene is different, e.g., a different carbon source (such as galactose instead of glucose), or a different specific chemical substance (e.g., addition of Cu²⁺ ions), antibiotic or by exposure to environmental factor (such as low/high temperature or light intensity). In another specific embodiment, the bidirectional promoter element enables inducible or repressible expression of the first and the second transgene, wherein the inducer or repressor of promoter activity for the first and the second transgene is identical.

In another embodiment, the bidirectional promoter element enables constitutive expression of the first or the second transgene. In a further embodiment, the bidirectional promoter element enables constitutive expression of the first and the second transgene. In yet another embodiment, the bidirectional promoter element enables inducible or repressible expression of the first transgene and constitutive expression of the second transgene, or *vice versa.*

Some embodiments of the recombinant yeast cell comprise more than one (i.e., at least two) genomically integrated expression cassette. In these embodiments, each genomically integrated expression cassette may comprise identical bidirectional promoter elements, or each genomically integrated expression cassette may comprise a different bidirectional promoter element, as described herein. Alternatively, some of the genomically integrated expression cassettes may comprise identical bidirectional promoter elements and some may comprise different bidirectional promoter elements.

The recombinant yeast cell according to the invention may comprise more than one genomically integrated expression cassette. In a preferred embodiment, the bidirectional promoter element of each expression cassette is different from the bidirectional promoter element of each further expression cassette. This enables individual regulation of expression levels for each of the encoded transgenes, or pair of transgenes.

In a further preferred embodiment, the bidirectional promoter element of each expression cassette is identical to the bidirectional promoter element of each further expression cassette. Alternatively, the inducer or repressor of promoter activity for the bidirectional promoter element of each expression cassette is the same as compared to the inducer or repressor for the bidirectional promoter element of each further expression cassette (e.g., all bidirectional promoter elements are regulated by Gal80-mediated inhibition of the activator Gal4p in the absence of galactose or lactose). In these cases, coordinated, or concerted co-expression of multiple components of a heterodimeric or multimeric protein complex, such as a VLP, in a host cell, enables efficient expression and assembly of such a heterodimeric or multimeric protein complex, e.g., a VLP. This can be in response to an inducing signal, e.g. galactose addition to the medium.

The recombinant yeast cell comprises at least one expression cassette that is stably integrated into the yeast cell genome. The expression cassette may be integrated at various chromosomal loci within the genome of the yeast cell. Particularly useful chromosomal loci for genomic integration of the expression cassette are listed in Table 3. In preferred embodiments, the at least one expression cassette is genomically integrated at a chromosomal locus which is selected such that genomic integration of the expression cassette leads to disruption of a gene coding for a metabolic enzyme rendering the recombinant yeast cell auxotrophic. This enables selection for successful integration of the expression cassettes by selection for restoration of prototrophy (in the form of restoration of the resident gene coding for a metabolic enzyme, or integration of a new prototrophic marker gene).

**Table 2: Exemplary yeast chromosomal loci for genomic integration of an expression cassette.**

| **Target Locus** | **Systematic Name / Annotation in *Km*** | **Gene Product** | **Selection Condition** |
|---|---|---|---|
| *ADE1* | KMAR_10170 / *ADE1* - | Phosphoribosylaminoimidazole-succinocarboxamide synthase | w/o adenine |
| *ADE2* | KMAR_20566/ *ADE2* | Phosphoribosylaminoimidazole carboxylase | w/o adenine |
| *ADE8* | KMAR_30620/ *ADE8* | Phosphoribosylglycinamide formyltransferase | w/o adenine |
| *ECM31* | KMAR_1 0771/ *ECM31* | 3-methyl-2-oxobutanoate hydroxymethyltransferase | w/o pantothenic acid |
| *HIS2* | KMAR_10422/ *HIS2* | Histidinol-phosphatase | w/o histidine |
| *HIS3* | KMAR_50033/ *HIS3* | Imidazoleglycerol-phosphate dehydratase | w/o histidine |
| *HIS5* | KMAR_80162/ HIS5 | Histidinol-phosphate aminotransferase | w/o histidine |
| *LEU1* | KMAR_50143/ *LEU1* | 3-Isopropylmalate isomerase | w/o leucine |
| *LEU2* | KMAR_20461/ *LEU2* | 3-isopropylmalate dehydrogenase | w/o leucine |
| *LYS2* | KMAR_80290/ *LYS2* | L-aminoadipate-semialdehyde dehydrogenase | w/o lysine |
| *LYS5* | MAR_*80103* / *LYS5* | L-aminoadipate-semialdehyde dehydrogenase-phosphopantetheinyl transferase | w/o lysine |
| *MET5* | KMAR_*MET5* | 40492/ Sulfite reductase [NADPH] subunit beta | w/o methionine. w/o cysteine |
| *MET17* | KMAR_10653/ *MET17* | O-acetyl homoserine-O-acetyl serine sulfhydrylase | w/o methionine. w/o cysteine |
| *TRP1* | KMAR_60134/ *TRP1* | N-(5'-phosphoribosyl) anthranilate isomerase | w/o tryptophan |
| *TRP3* | KMAR_30022/ *TRP3* | Indole-3-glycerol phosphate synthase | w/o tryptophan |
| *TRP4* | KMAR_20433/ *TRP4* | Anthranilate phosphoribosyltransferase | w/o tryptophan |
| *TRP5* | KMAR_20042 / *TRP5* | Tryptophan synthase | w/o tryptophan |
| *TYR1* | KMAR_10786 / *TYR1* | Prephenate dehydrogenase | w/o tyrosine |
| *URA3* | KMAR_10705 / *URA3* | Orotidine 5'-phosphate decarboxylase | w/o uracil |
| *URA5* | KMAR_10567 / *URA5* | Orotate phosphoribosyltransferase | w/o uracil |

Another group of preferred chromosomal loci for genomic integration of the expression cassette are genes coding for enzymes involved in carbon or nitrogen source metabolism. Examples are listed in Table 4. Disruption or deletion of these chromosomal loci deprives the yeast cell of its ability to utilize specific metabolites as sole sources of carbon or nitrogen (see Table 4).

**Table 4: Further exemplary yeast chromosomal loci for genomic integration of an expression cassette.**

| **Gene Name** | **Systematic Name / Annotation in *Km*** | **Gene Product** | **Selection Condition** |
|---|---|---|---|
| *FCY1* | KMAR_80322/ *FCY1* | Cytosine deaminase | Cytosine as sole nitrogen source |
| *GAP1* | KMAR_10360 / *GAP1* | General amino acid permease | L -citrulline as sole nitrogen source |
| *LAC4* + *LAC12* | KMAR_30002 / *LAC4* + KMAR_30003 / *LAC12* | β-galactosidase and lactose permease | Lactose as sole carbon source |
| *XYL1* | KMAR_10659/ *XYL1* | NAD(P)H-dependent D-xylose reductase | Xylose as sole carbon source |
| *XYL2* | KMAR_70036/ SOR1 | Xylitol dehydrogenase / Sorbitol dehydrogenase 1 | Xylose as sole carbon source |

Thus, in one embodiment, each expression cassette is genomically integrated at a chromosomal locus selected from the group consisting of *ADE1, ADE2, ADE8, ECM31, HIS2, HIS3, HIS5, LEU1, LEU2, LYS2, LYS5, MET5, MET17, TRP1, TRP3, TRP4, TRP5, TYR1, URA3, URA5, FCY1, GAP1, LAC4* + *LAC12, XYL1 andXYL2.* Preferably, each expression cassette is genomically integrated at a chromosomal locus selected from the group consisting of *TYR1, ADE2, LEU2, LAC4-LAC12, MET5, HIS3, TRP1, XYL2, and LYS5,* more preferably from the group consisting of *ADE2, LEU2, MET5* and *HIS3.*

In case the recombinant yeast cell comprises more than one genomically integrated expression cassette, it is preferred that the expression cassettes are not linked, i.e., they are preferably genomically integrated at chromosomal loci located on different chromosomes. This will assure independent segregation in mitosis thereby offering an easy way to generate different combinations of markers (and as a consequence of different expression cassettes) in the same cell. Accordingly, it is particularly preferred, that the at least two genomically integrated expression cassettes are each genomically integrated at chromosomal loci located on different chromosomes, wherein the chromosomal loci are selected from the group consisting of *ADE1, ECM31, HIS2, MET17, TYR1, URA3, URA5, GAP1, XYL1* (chromosome 1); *ADE2, LEU2, TRP4, TRP5* (chromosome 2); *ADE8, TRP3, LAC4-LAC12* (chromosome 3); *MET5* (chromosome 4); *HIS3, LEU1* (chromosome 5); *TRP1* (chromosome 6), *XYL2* (chromosome 7) and *HIS5, LYS2, LYS5, FCY1* (chromosome 8). Preferably, the expression cassettes are genomically integrated at chromosomal loci selected from the group consisting of *TYR1; ADE2, LEU2; LAC4-LAC12; MET5; HIS3; TRP1, XYL2* and *LYS5,* more preferably from the group consisting of *ADE2, LEU2, MET5* and *HIS3.*

The genomically integrated expression cassette comprises at least one selection marker. Generally, the term "selection marker" (or selection marker gene) refers to a gene or part (fragment) of a gene that confers a selectable growth phenotype (physical growth characteristics) on a cell receiving that (part of a) gene as, for example through a transformation event. Selection markers generally fall into several types, including positive selectable marker genes which confer a selectable growth phenotype to a cell that receives the marker or negative selectable (counter-selectable) markers that allow only those cells to grow, i.e. to form colonies, that do not comprise the selection marker (e.g., that have lost the selection marker). In the case of negative selectable markers, selection requires a growth medium that inhibits growth of the marker containing host cells. For example, the biosynthetic pathway for the nucleobase uracil is inhibited by the drug 5-fluoro-orotic acid (FOA). This fluorinated orotic acid (orotic acid is a biosynthetic precursor of uracil) is converted into the toxic fluoro-uracil by the enzyme encoded by the *URA3* gene. *Ura3* mutants are lacking the enzyme and are therefore resistant to 5-FOA and can be selected on plates administered with the drug. The intact *URA3* gene is one of the rare examples for a counter-selectable or negative selection marker.

Dominant selection markers, such as genes conferring resistance to an antibiotic or other drug, have the advantage that they can be used for selection in any genetic background sensitive to the drug, including diploid cells. Transformation of an expression cassette as described herein, comprising an antibiotic resistance gene as selection marker, allows the identification of a transformed recombinant yeast cell comprising the expression cassette through the ability of this cell to form detectable colonies on or in the medium comprising the antibiotic. Colony formation requires stable inheritance of the dominant selection marker, which can be achieved by genomic integration. In the absence of a vector that enables inheritance of the marker by autonomous replication in the host cell, colony formation is a safe indicator for genomic integration of the expression cassette.

Some selection markers described herein are metabolic selection markers that enable an otherwise auxotrophic yeast cell to grow, i.e., to form colonies on a selective medium. The selective medium lacks the specific metabolite(s) that is/are essential for the cell, e.g., uracil. For example, the *URA3* marker gene can complement a uracil auxotrophy by providing the essential metabolite via the activity of the *URA3*-encoded enzyme. Alternatively, the selective medium supplies a specific nutrient for selection and colony formation requires that said nutrient is metabolized to provide a component essential for growth, wherein metabolism of the specific nutrient depends on the gene product of the selection marker. For example, lactose can be used as a specific metabolite to select for the enzyme β-galactosidase, which is required to hydrolyse β-galactosides into the respective monosaccharides. Selection using metabolic selection markers may be based on the genomic integration of a gene encoding a metabolic enzyme (the selection marker comprised in the expression cassette), or a fragment thereof, wherein the respective native gene encoding said metabolic enzyme (or a functional equivalent) has been deleted, mutated or disrupted prior to genomic integration of the expression cassette. Said genomic integration of the expression cassette can take place at the chromosomal locus that natively comprises the gene coding for the metabolic enzyme, or at a different chromosomal locus. In other words, genomic integration of the expression cassette can result in reconstitution of a gene coding for a metabolic enzyme at the native chromosomal locus of said gene, or it can lead to introduction of a gene coding for a metabolic enzyme at an alternative locus, wherein the native gene has been rendered non-functional. In the case of genomic integration at the native chromosomal locus, the native gene coding for the metabolic enzyme may have been deleted only partially and the metabolic selection marker may comprise the deleted fragment of said gene coding for the metabolic enzyme, thus reconstituting said gene upon genomic integration of the expression cassette.

The at least one selection marker resides within the expression cassette such that it does not interfere with the function of the bidirectional promoter element, the transgenes or the transcription terminators.

The genes listed as potential chromosomal target loci in Tables 3 and 4 (or fragments thereof) can serve as auxotrophic selection markers. Prior to introduction of the expression cassette at one of these exemplary target loci coding for a metabolic enzyme, the respective locus is mutated, disrupted or deleted, rendering the host cell auxotrophic and thus selectable using the gene coding for the metabolic enzyme (or a fragment thereof) as a positive selection marker.

Further exemplary selection markers are listed in Table 5.

**Table 5: Exemplary selection marker genes.**

| **Marker** | **Name of resistance marker gene** | **Gene Product** | **Selection Condition** |
|---|---|---|---|
| Heterologous genes | | | |
| *kanMX* | *KanR* | *E. coli* transposon Tn 903 *kanR* gene | resistance to Geneticin (G418) |
| *hphMX6* | *HygR* | *Klebsiella pneumoniae* phosphotransferase | resistance to hygromycin B |
| *natMX6* | *NrsR* | *Streptomyces noursei* acetyltransferase | resistance to nourseothricin |
| *bleMX6* | *BleoR* | Tn5 phleomycin-binding protein | resistance to phleomycin |
| *cat* | *CatR* | *Bacillus subtilis* Tn9 acetyltransferase | resistance to chloramphenicol |
| *dehH1* | *dehH1R* | *Moraxella* sp. Haloacetate dehalogenase H-1 | resistance to fluoroacetate |
| *dsdA* | *dsdAR* | *E. coli* D-serine dehydratase | resistance to D -serine + L - proline |
| *mdr3* | *mdr3R* | *Mus musculus* P-glycoprotein | resistance to FK520 |
| aroA | aroAR | *E. coli* 5-enolpyruvylshikimate-3- phosphate synthase | resistance to glyphosate |
| *pat* | *patR* | *Streptomyces viridochromogenes* Phosphinothricin N-acetyltransferase | resistance to bialaphos |
| *R dhfr* | *R dhfrR* | *E. coli* dihydrofolate reductase | resistance to methotrexate + sulfanilamide |
| *CAN1* | *CAN1R* | *S. cerevisiae* arginine permease | resistance to canavanine |

| Endogenous genes | | | |
|---|---|---|---|
| *CUP1* | *CUP1R* | *S. cerevisiae* Metallothionein | resistance to copper and cadmium |

Thus, in one embodiment, the selection marker is a resistance marker or a metabolic selection marker, preferably a metabolic selection marker. In particularly preferred embodiments, the selection marker is selected from the group consisting of *ADE1, ADE2, ADE8, ECM31, HIS2, HIS3, HIS5 LEU1, LEU2, LYS2, LYS5, MET5, MET17, TRP1, TRP3, TRP4, TRP5, TYR1, URA3, URA5, FCY1, GAP1, LAC4* + *LAC12, XYL1, XYL2, kanMX, hphMX6, natMX6, bleMX6, cat, dehH1, dsdA, mdr3, aroA, pat, R dhfr, CAN1* and *CUP1.* In a particularly advantageous embodiment, the marker gene is selected from the group consisting of *ADE2, MET5, HIS3, LEU2, URA3, kanMX, hphMX6* and *natMX6.*

Advantageously, the selection marker of the genomically integrated expression cassette is coupled to and sandwiched in between a pair of site-specific recombination sequences, optionally between a pair of IoxP sites. The selection marker interposing between the paired site-specific recombination sequences is made to be removable from the genomically integrated expression cassettes. These site-specific recombination sequences are special polynucleotide sequences recognizable by a corresponding enzyme or regulatory element to act on. The site-specific recombination sequences can be IoxP sites recognizable and capable of reacting with Cre recombinase or other equivalents to carry out recombinase-mediated marker recovery. The Cre/loxP recombination system was developed, originally for *S. cerevisiae* and later adapted for *K. marxianus.* Expression of the Cre recombinase from a plasmid results in Cre catalysed excision of DNA sequences between the two IoxP sites, e.g., the selection marker gene. Thus, the marker can be used several times while its original site is now marked by a IoxP sequence that is left behind by the recombination reaction.

For further specific markers, mainly drug resistance markers (*kanMX, hphMX6, natMX6*) an analogous recycling is possible (Goldstein, A. L.; McCusker, J. H. (1999), Yeast (Chichester, England) 15 (14), S. 1541-1553). The *URA3* auxotrophic marker can be recycled by selection for FOA resistance in strains that contain the *URA3* marker in knockout cassettes.

In a further preferred embodiment, a specific counter-selection marker may also be included between the paired site-specific recombination sequences to facilitate the marker recovery. Meganuclease recognition sites of I-Scel from the mitochondria of baker's yeast, I-Crel from the chloroplasts of *Chlamydomonas reinhardtii* and I-Dmol from the archaebacterium *Desulfurcoccus mobilis* will be of interest to enable marker recovery and counter selection via elimination of DNA elements located between the paired site by site-specific recombination mediated by the specific nuclease. Alternatively, CRISPR/Cas9-mediated counter-selection using guide RNA targeting at the selection marker can also be of interest to enable complete marker recovery.

The genomically integrated expression cassette comprises a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene. The transcription terminator or terminator sequence signifies and effects end of transcription and release of the transcribed mRNA from the template for peptide or protein translation. In specific embodiments, the first and second transcription terminator are each selected from the group consisting of a *TEF1* terminator from *Ashbya gossypii* (SEQ ID NO: 29), a *HIS5* terminator from *K. marxianus* (SEQ ID NO: 30), a CPS1 terminator from *K. marxianus* (SEQ ID NO: 31), a *CYC1* terminator from S. *cerevisiae* (SEQ ID NO: 32) and an *ADH1* terminator from S. *cerevisiae* (SEQ ID NO: 33). In a particularly advantageous embodiment, the first and second transcription terminator each are a *TEF1* terminator from *A. gossypii.*

The genomically integrated expression cassette may comprise further transcriptional and translational regulatory sequences, such as enhancer elements. Enhancer elements serve as binding sites for regulatory factors, which may increase promoter activity, when operably linked to a coding sequence. Enhancer elements are cis-acting elements of DNA, usually about from 10 to 300 bp, serving as binding site(s) for an activator which can act on a promoter element to increase the transcription rate. Transcriptional enhancers are commonly orientation and position independent, having been found 5' and 3' to the transcription unit, within an intron, as well as within the coding sequence itself.

Expression cassettes for use in eukaryotic host cells (such as yeast cells) usually contain further sequences necessary for the termination of transcription and for stabilizing the mRNA. Such sequences are commonly located 3' to the translation termination codon, in untranslated regions of polynucleotide sequences coding for eukaryotic or viral polypeptides and contain nucleotide segments transcribed as polyadenylated fragments in the untranslated portion of the mRNA.

The recombinant yeast cell according to the invention may be a member of the *Saccharomycetaceae* family. In one embodiment, the recombinant yeast cell is derived from a genus selected from the group consisting of *Andozyma, Ascobotryozyma, Citeromyces; Debaryomyces, Dekkera, Eremothecium, Issatchenkia, Kazachstania, Kluyveromyces, Kodamaea, Lodderomyces, Pachysolen, Pichia, Saccharomyces, Saturnispora, Tetrapisispora, Torulaspora, Williopsis* and *Zygosaccharomyces.* In a preferred embodiment, the recombinant yeast cell is derived from the yeast genus *Kluyveromyces.* More preferably, the recombinant yeast cell is derived from the yeast species *Kluyveromyces marxianus.* In a particularly preferred embodiment, the recombinant yeast cell is derived from *Kluyveromyces marxianus* strain NBRC1777.

Generally, cells of yeast exist in a haploid, a diploid or a tetraploid form. The diploid cells may, under appropriate conditions, proliferate for indefinite number of generations in the diploid form. In species with a sexual cycle, diploid cells can also undergo meiosis and sporulate to form haploid cells.

The recombinant yeast cell of the invention may be haploid, diploid, or tetraploid. In one embodiment, the recombinant yeast cell is diploid or tetraploid. The inventors have found that diploid or tetraploid yeast cells are particularly advantageous in that they provide robustness, high rates of growth and protein biosynthesis and thus enable highly efficient expression of the gene products encoded by the first and second transgene.

As used herein, the term "haploid" refers to a cell having a single copy of each chromosome of its normal genomic (chromosomal) complement. The term "diploid" refers to a cell having two copies of each chromosome of its normal genomic complement. The term "allele" designates a specific variant of the gene. Generally, the two parental strains can be distinguished by differences in the DNA sequence between the parental allele (maternally vs paternally-derived or MATa-derived vs. MATα-derived). Diploids can be formed by the process of mating between two mating-competent haploid cells. "Mating" of yeast describes a genetically controlled process by which two haploid yeast cells naturally fuse to form one diploid yeast cell with a single nucleus. When two diploid yeast cells naturally fuse, they can form one tetraploid yeast cell with a single nucleus. A yeast cell is "mating competent", if it is capable of fusing with a further yeast cell by mating or spheroplast fusion. Mating of haploid yeast cells results in diploid yeast cells and sequential mating can further result in tetraploid strains through further mating of the auxotrophic diploids.

In a specific embodiment, the recombinant yeast cell is haploid. Preferably, the haploid recombinant yeast cell is heterothallic, i.e., it has a stable mating type. In other words, it is unable to switch its mating type. Further preferably, the yeast cell comprises deletions and/or mutations rendering a gene required for mating type switching from MATa to MATα or a gene required for mating type switching from MATα to MATa non-functional. In a particular embodiment, the yeast cell comprises deletions and/or mutations rendering the *KAT1* gene or the *ALPHA3* gene non-functional. Deletions and/or mutations of the *KAT1* gene or the *ALPHA3* render the recombinant yeast cell heterothallic.

Advantageously, the haploid recombinant yeast cell is mating competent. In a particularly preferred embodiment, the recombinant yeast cell is haploid, comprises deletions and/or mutations rendering a gene required for mating type switching from MATa to MATα or a gene required for mating type switching from MATα to MATa (e.g., the *KAT1* gene or the *ALPHA3* gene) non-functional and is mating competent.

The invention further relates to a diploid recombinant yeast cell obtained by mating a first and a second haploid recombinant yeast cell as described herein. Generally, diploid yeast cells may be generated by mating a first and a second haploid yeast cell, wherein the first and the second yeast cell are of a different mating type, i.e., the first haploid yeast cell is of mating type MATa and the second haploid yeast cell is of mating type MATα, or *vice versa.*

Mating competent haploid yeast cells, derived from, e.g., *Ascomyceta,* including *K. marxianus,* offer several advantages in the context of the present invention. Mating is a genetic program that enables two haploid recombinant yeast cells of opposite mating types to fuse and generate a diploid recombinant yeast cell with a single nucleus. Mating occurs between cells of the same strain and between cells of different strains but generally requires that strains belong to the same species. The resulting diploid recombinant yeast cell inherits a complete set of chromosomes from each of the haploid parents and mating of two haploid recombinant yeast cells possessing different genetic elements, e.g. different expression cassettes, genetic markers and/or transgenes will give diploid recombinant yeast cells that contain the complete genetic information from both parental haploids. Mating haploid recombinant yeast cells therefore provides a convenient and fast route for recombinant yeast strain construction and consolidation. To select diploid recombinant yeast cells after mating, at least one selectable marker is required in each of the haploid parents, preferably an auxotrophic marker in each haploid recombinant yeast cell. Advantageously, the markers are complementary between the two haploid strains.

Diploid strains have been observed to have greater thermostability as well as a higher tolerance to acid, ethanol, and other fermentation inhibitors than haploid strains. Thus, yeast mating provides an efficient means for recombinant yeast strain construction and diverse possibilities of combining haploid recombinant yeast cells comprising multiple transgenes from a reservoir of haploid recombinant yeast cells, i.e., it is time saving through quick and reliable combination of a variety of parental strains. In addition, the resulting diploid recombinant yeast cells offer superior growth and transgene expression characteristics (e.g., higher possible cell density, higher tolerance with regard to inhibitory compounds, higher yield of transgene-encoded gene product).

However, the diploid recombinant yeast cells of species with a sexual cycle may undergo meiosis and sporulation resulting in, haploid spores that germinate and proliferate, often under conditions of nutritional stresses. Meiosis requires crossing over events between homologous chromosomes, which can lead to loss of genomically integrated sequences (e.g., expression cassettes) present in only one of the two homologous chromosomes. Moreover, meiotic recombination and crossing over events may give haploid progeny that can grow on the diploid selective medium such that a culture of a recombinant production strain contains a mixture of diploids and haploids. Such a situation is unfavourable since it affects reproducibility of the fermentation processes and genetic stability of the production strain. It may also negatively impact the production rate of the gene product encoded by the transgene (e.g., an immunogenic polypeptide). Due to the concern about diploid stability, especially in bioreactor fermentation processes, strategies are necessary to provide genetic stability as well as efficient and reproducible transgene expression in the diploid recombinant yeast cells that are used as production strain. The success of such an approach in bioprocesses is based on careful selection of the integration sites for expression cassettes, a suitable marker strategy and a high genetic similarity between the haploid parent cells.

The enrichment of haploid cells during cultivation of diploids can be prevented by linking each expression cassette to a prototrophic marker gene and by using two haploid recombinant yeast cells that are each auxotrophic and require supplementation of media for growth of the haploid recombinant yeast cells. The tight genetic linkage, i.e. close proximity of transgenes and selection marker means that homologous recombination events that separate the transgene from the marker are extremely rare and can be recognized by genetic screening. Advantageously, the auxotrophic markers are complementary between the two haploid strains (e.g., Ade⁻ and His⁻ or Met⁻ and Leu⁻), such that the diploid recombinant yeast cell will grow in the absence of all supplements (leu, his, met and ade, ura) required by the haploid recombinant yeast cells.

Thus, in a preferred embodiment, the diploid recombinant yeast cell obtained by mating a first and a second haploid recombinant yeast cell as described herein, comprises at least two selection markers genomically integrated at chromosomal loci located on homologous chromosomes. Advantageously, said selection markers are comprised in the genomically integrated expression cassette(s). In a specific embodiment, the first and the second haploid recombinant yeast cell are each auxotrophic and the diploid recombinant yeast cell is able to grow under selection conditions under which the first and the second haploid recombinant yeast cell exhibit reduced or no growth or proliferation. This means that the auxotrophy of the first haploid recombinant yeast cell is (partially) complemented by the genome of the second recombinant yeast cell and vice versa. For example, the first haploid recombinant yeast cell may comprise a (partial) deletion, mutation or disruption of a gene encoding a metabolic enzyme, thus rendering the first haploid recombinant yeast cell auxotrophic. If the second haploid recombinant yeast cell comprises an intact (functional) gene encoding said metabolic enzyme, the resulting diploid recombinant yeast cell is able to grow under selection conditions under which the first haploid recombinant yeast cell exhibits reduced or no growth or proliferation. Preferably, the first and the second haploid recombinant yeast cell are each auxotrophic and the diploid recombinant yeast cell is prototrophic. Further preferably, the selection markers are metabolic selection markers. Further preferably, the chromosomal loci located on homologous chromosomes are the same chromosomal loci on each of the homologous chromosomes (equivalent chromosomal loci of homologous chromosomes).

The diploid recombinant yeast cell may be obtained by mating a first and a second haploid recombinant yeast cell, wherein the first and the second haploid recombinant yeast cell each comprise one, two, three or four genomically integrated expression cassettes, resulting in a diploid recombinant yeast cell comprising two, four, six or eight genomically integrated expression cassettes.

Advantageously, the diploid recombinant yeast cell is obtained by mating a first and a second haploid recombinant yeast cell that comprise different expression cassettes, i.e., expression cassettes comprising different transgenes. Accordingly, in a preferred embodiment of the diploid recombinant yeast cell, generated from a first and a second haploid recombinant yeast cell according to the invention, the first transgene and the second transgene of each expression cassette are different from the first and second transgene of each further expression cassette.

In a specific embodiment, the first and the second haploid recombinant yeast cell each comprise one genomically integrated expression cassette, wherein the genomically integrated expression cassette of the first haploid recombinant yeast cell comprises a first pair of transgenes and the genomically integrated expression cassette of the second haploid recombinant yeast cell comprises a second pair of transgenes, wherein the first and the second pair of transgenes are different. In a further embodiment, the first and the second haploid recombinant yeast cell each comprise two genomically integrated expression cassettes, wherein the genomically integrated expression cassettes of the first haploid recombinant yeast cell comprise a first and a second pair of transgenes and the genomically integrated expression cassettes of the second haploid recombinant yeast cell comprise a third and a fourth pair of transgenes, wherein the first, second, third and fourth pair of transgenes are different. In another embodiment, the first and the second haploid recombinant yeast cell each comprise three genomically integrated expression cassettes, wherein the genomically integrated expression cassettes of the first haploid recombinant yeast cell comprise a first, a second and a third pair of transgenes and the genomically integrated expression cassettes of the second haploid recombinant yeast cell comprise a fourth, a fifth and a sixth pair of transgenes, wherein the first, second, third, fourth, fifth and sixth pair of transgenes are different. In yet another embodiment, the first and the second haploid recombinant yeast cell each comprise four genomically integrated expression cassettes, wherein the genomically integrated expression cassettes of the first haploid recombinant yeast cell comprise a first, a second, a third and a fourth pair of transgenes and the genomically integrated expression cassettes of the second haploid recombinant yeast cell comprise a fifth, a sixth, a seventh and an eighth pair of transgenes, wherein the first, second, third, fourth, fifth, sixth, seventh and eighth pair of transgenes are different.

In another advantageous embodiment, the diploid recombinant yeast cell is obtained by mating a first and a second haploid recombinant yeast cell according to the invention, wherein the first and the second haploid recombinant yeast cell each comprise an identical number of genomically integrated expression cassettes and wherein the expression cassettes are genomically integrated at chromosomal loci located on homologous chromosomes. Preferably, the expression cassettes are genomically integrated at the same chromosomal loci in the first and the second haploid recombinant yeast cell. This results in a diploid recombinant yeast cell comprising pairs of expression cassettes that are genomically integrated at corresponding (homologous) loci in homologous chromosomes.

In one specific embodiment, the diploid recombinant yeast cell is obtained by mating a first and a second haploid recombinant yeast cell, wherein the first and the second haploid recombinant yeast cell each comprise an identical number of genomically integrated expression cassettes and wherein the expression cassettes are genomically integrated at the same chromosomal loci in the first and the second haploid recombinant yeast cell, such that the diploid recombinant yeast cell comprises pairs of expression cassettes that are genomically integrated at corresponding (homologous) loci in homologous chromosomes, which are selected from the group consisting of *ADE1, ADE2, ADE8, ECM31, HIS2, HIS3, HIS5, LEU1, LEU2, LYS2, LYS5, MET5, MET17, TRP1, TRP3, TRP4, TRP5, TYR1, URA3, URA5, FCY1, GAP1, LAC4* + *LAC12, XYL1 and XYL2.* Preferably, the chromosomal loci are selected from the group consisting of *TYR1, ADE2, LEU2, LAC4-LAC12, MET5, HIS3, TRP1, XYL2, and LYS5,* more preferably from the group consisting of *ADE2, LEU2, MET5* and *HIS3.*

The resulting diploid recombinant yeast cell comprises pairs (e.g., one, two, three or four pairs) of expression cassettes that are integrated at corresponding (homologous) chromosomal loci in homologous chromosomes, wherein the corresponding (homologous) chromosomal loci are selected from the group consisting of *ADE1, ADE2, ADE8, ECM31, HIS2, HIS3, HIS5, LEU1, LEU2, LYS2, LYS5, MET5, MET17, TRP1, TRP3, TRP4, TRP5, TYR1, URA3, URA5, FCY1, GAP1, LAC4* + *LAC12, XYL1 and XYL2, preferably* from the group consisting of *TYR1, ADE2, LEU2, LAC4-LAC12, MET5, HIS3, TRP1, XYL2 and LYS5* and most preferably from the group consisting of *ADE2, LEU2, MET5* and *HIS3.*

Thus, in an exemplary embodiment, the first and the second haploid recombinant yeast cell each comprise a genomically integrated expression cassette at the *ADE2* locus. Mating of said first and second haploid recombinant yeast cell results in a diploid recombinant yeast cell comprising a genomically integrated expression cassette at each one of the homologous *ADE2* loci. As a result, loss of the genomically integrated expression cassettes due to sister chromatid exchange is suppressed under constant selection pressure for the selection marker genes comprised in each of the genomically integrated expression cassettes. In a further embodiment, the first and the second haploid recombinant yeast cell each comprise two genomically integrated expression cassettes, one at the *ADE2* locus and one at the *HIS3* locus. In another embodiment, the first and the second haploid recombinant yeast cell each comprise three genomically integrated expression cassettes, one at the *ADE2* locus, one at the *HIS3* locus and one at the *MET5* locus. In yet another embodiment, the first and the second haploid recombinant yeast cell each comprise four genomically integrated expression cassettes, one at the *ADE2* locus, one at the *HIS3* locus, one at the *MET5* locus and one at the *LEU2* locus.

Provision of a diploid recombinant yeast cell by mating or spheroplast fusion of a first and a second haploid recombinant yeast cell according to the invention enables efficient generation of diploid recombinant yeast cells comprising a multitude of genomically integrated expression cassettes and thus comprising various different transgenes. In specific embodiments, the transgenes encode immunogenic polypeptides derived from more than one pathogen. For example, the transgenes may encode immunogenic polypeptides derived from more than one strain of the same viral pathogen. Alternatively, the transgenes may encode immunogenic polypeptides derived from different viral pathogens.

In a specific embodiment, the diploid recombinant yeast cell is obtained by mating a first and a second haploid recombinant yeast cell according to the invention, wherein the first and the second haploid recombinant yeast cell each comprise an identical number of genomically integrated expression cassettes and wherein the expression cassettes are genomically integrated at the same chromosomal loci in the first and the second haploid recombinant yeast cell, such that the diploid recombinant yeast cell comprises pairs of expression cassettes that are genomically integrated at corresponding (homologous) loci in homologous chromosomes, wherein each transgene encodes an immunogenic polypeptide derived from a viral pathogen (or an immunogenic fragment thereof), preferably wherein the viral pathogen belongs to the family of *Reoviridae,* more preferably wherein the viral pathogen is a rotavirus, even more preferably wherein the viral pathogen is Porcine Rotavirus A (PRVA) and most preferably wherein each immunogenic polypeptide comprises a polypeptide selected from the group consisting of Porcine Rotavirus A (PRVA) VP2, VP4, VP6, VP7, NSP2 and NSP4 and one or more immunogenic fragment thereof. For example, each immunogenic polypeptide advantageously comprises an amino acid sequence selected from the group consisting of SEQ ID NO: 1 (PRVA VP2), SEQ ID NO: 2 (PRVA VP4), SEQ ID NO: 3 (PRVA VP6), SEQ ID NO: 4 (PRVA VP7), SEQ ID NO: 5 (PRVA NSP2) and SEQ ID NO: 6 (PRVA NSP4) and one or more immunogenic fragment thereof, preferably from the group consisting of SEQ ID NOs: 1, 2, 3, and 4 and one or more immunogenic fragment thereof.

Advantageously, each immunogenic polypeptide comprises a polypeptide selected from the group consisting of Porcine Rotavirus A (PRVA) VP2, VP4, VP6 and VP7 and immunogenic fragments thereof, wherein the nucleotide sequences encoding the immunogenic polypeptides are set forth in SEQ ID NO: 13 (PRVA VP2), SEQ ID NO: 14 (PRVA VP4), SEQ ID NO: 15 (PRVA VP6) and SEQ ID NO: 16 (PRVA VP7). Each pair of expression cassettes is preferably genomically integrated at corresponding (homologous) chromosomal loci selected from the group consisting of *ADE1, ADE2, ADE8, ECM31, HIS2, HIS3, HIS5, LEU1, LEU2, LYS2, LYS5, MET5, MET17, TRP1, TRP3, TRP4, TRP5, TYR1, URA3, URA5, FCY1, GAP1, LAC4* + *LAC12, XYL1 and XYL2,* more preferably from the group consisting of *TYR1, ADE2, LEU2, LAC4-LAC12, MET5, HIS3, TRP1, XYL2 and LYS5,* most preferably from the group consisting of *ADE2, LEU2, MET5* and *HIS3.*

In a further specific embodiment, the diploid recombinant yeast cell is obtained by mating a first and a second haploid recombinant yeast cell according to the invention, wherein the first and the second haploid recombinant yeast cell each comprise two genomically integrated expression cassettes and wherein the expression cassettes are genomically integrated at the same chromosomal loci in the first and the second haploid recombinant yeast cell, such that the diploid recombinant yeast cell comprises two pairs of expression cassettes (i.e., four expression cassettes) wherein each pair is genomically integrated at corresponding (homologous) loci in homologous chromosomes, wherein the first and second transgene of the first expression cassette comprise the nucleic acid sequence of SEQ ID NO: 13 (PRVA VP2), the first and second transgene of the second expression cassette comprise the nucleic acid sequence of SEQ ID NO: 14 (PRVA VP4), the first and second transgene of the third expression cassette comprise the nucleic acid sequence of SEQ ID NO: 15 (PRVA VP6) and the first and second transgene of the fourth expression cassette comprise the nucleic acid sequence of SEQ ID NO: 16 (PRVA VP7). Preferably, each pair of expression cassettes is genomically integrated at corresponding (homologous) chromosomal loci selected from the group consisting of *ADE1, ADE2, ADE8, ECM31, HIS2, HIS3, HIS5 LEU1, LEU2, LYS2, LYS5, MET5, MET17, TRP1, TRP3, TRP4, TRP5, TYR1, URA3, URA5, FCY1, GAP1, LAC4* + *LAC12, XYL1 and XYL2,* more preferably from the group consisting of *TYR1, ADE2, LEU2, LAC4-LAC12, MET5, HIS3, TRP1, XYL2 and LYS5,* most preferably from the group consisting of *ADE2, LEU2, MET5* and *HIS3.*

In the abovementioned embodiments of the diploid recombinant yeast cell, each pair of expression cassettes at corresponding (homologous) chromosomal loci preferably comprises different selection markers in the first and in the second expression cassette of each pair. Preferably, said different selection markers are each auxotrophic selection markers and the auxotrophic markers are complementary between the first and the second expression cassette of each pair. As a result, the diploid recombinant yeast cell is prototrophic, i.e., it will grow in the absence of all supplements required by the haploid recombinant yeast cells, whereas growth of said haploid recombinant yeast cells (e.g., resulting from sporulation) is suppressed under the same conditions. Moreover, the occurrence of genetic cross-over, which may result in relocation or loss of the expression cassettes, is very low, resulting in a genetically highly stable recombinant yeast strain. It should be noted that, while it is generally desirable to suppress sporulation events and growth of the resulting haploid recombinant yeast cells when using diploid recombinant yeast cells for production purposes (e.g. production of a whole yeast vaccine), sporulation can be advantageous for fast strain construction if new antigen-combinations are of interest (Figure 14). The possible number of new antigen combinations resulting from meiotic recombination can be calculated by the formula C=(2n)-2[C= number of new antigen-combinations; n= number of chromosomes with expression cassettes], assuming the expression cassettes are integrated at the corresponding (homologous) chromosomal loci. When mating two haploid yeast cells, each bearing four expression cassettes in chromosomes 1, 2, 3 and 4 (e.g., MATa: Chr.1 *TYR1::VP2;* Chr. *2ADE2::VP6;* Chr. *3LAC4::VP4[P7];* Chr.4 *MET5::VP7[G5]* / MATα: Chr.1 *HIS3::VP4[P6];* Chr. *2::TRP1::VP4[P23];* Chr. 3 *::XYL2::VP4[P32];* Chr.4 *::LYS5::VP7[G9]),* the resulting diploid yeast cell expresses eight antigens (VP2; VP6; VP4[P7]; VP7[G5]; VP4[P6]; VP4[P23]; VP4[P32]; VP7[G9]). By induced sporulation, 14 new antigenic arrangements can be selected by suitable choice of selection markers, which are comprised in and thus genetically closely linked to the corresponding expression cassettes.

As for each chromosome at least one integration locus is available, the maximum number of new antigenic arrangements (if n=8) is 254. That means 254 new antigenic combinations in the corresponding haploid yeast cells can be selected. The occurring haploids can again be used for mating and subsequent sporulation until a desired antigen combination is created.

By creating a set of haploids covering all known serotypes from a certain virus (e.g. to date, 27 different G- and 37 P-genotypes have been described in both humans and animals for rotaviruses of type A), this method enables fast reaction on new circulating serotype combinations, without time consuming strain construction and genetic verifications (e.g., by PCR, Southern Blot, Western Blot).

The haploid and diploid, preferably, diploid recombinant yeast cells of the invention can advantageously be cultured for production purposes (e.g., production of a whole yeast vaccine).

Such production purposes may include growth in minimal media, which media lacks preformed amino acids, and other complex biomolecules, e.g. media comprising a nitrogen source (proline, glutamine or ammonia) and glucose, lactose or galactose as an energy and carbon source and salts as a source of phosphate, calcium and the like. Preferably, such production media lacks selective agents such as antibiotics, amino acids, purines, pyrimidines, etc. Diploid recombinant yeast cells can be grown to high cell density, for example at least about 10 g/L, at least about 25 g/L, at least about 50 g/L, at least about 75g/L or at least about 100 g/L dry weight.

The invention further relates to a vaccine composition comprising at least one recombinant yeast cell as disclosed hereinabove. The vaccine composition may comprise a haploid, a diploid or a tetraploid recombinant yeast cell. Preferably, the vaccine composition comprises a diploid recombinant yeast cell, most preferably a diploid recombinant yeast cell obtained by mating a first and a second haploid recombinant yeast cell according to the invention.

In another aspect, the invention relates to the recombinant yeast cell or the vaccine composition as described herein for use in vaccinating a subject against an infectious disease. The invention also relates to a method of vaccinating a subject against an infectious disease, comprising administering the recombinant yeast cell or the vaccine composition as described herein. In a specific embodiment, the subject is a mammal, preferably a pig. In a further specific embodiment, the infectious disease is a viral disease, preferably a viral disease selected from the group consisting of Porcine Rotavirus A or Porcine Rotavirus C, more preferably Porcine Rotavirus A. Thus, in a particularly preferred embodiment, the invention relates to the recombinant yeast cell or the vaccine composition as described herein for use in vaccinating a pig against Porcine Rotavirus A or Porcine Rotavirus C, in particular Porcine Rotavirus A.

Administration of the recombinant yeast cell or the vaccine composition may be parenteral, enteral, intramuscular, mucosal or oral, optionally in combination with conventional carriers and/or excipients. Preferably, administration is parenteral, most preferably subcutaneous.

The vaccine composition may comprise at least one physiologically acceptable carrier, diluent, adjuvant and/or excipient. For example, recombinant yeast cells according to the invention may be prepared in a pharmaceutically acceptable carrier, such as in aqueous saline or buffer in a vaccine composition for administration. Such carrier may comprise common pharmaceutic excipients, such as physiologically acceptable salts to adjust osmotic pressure, buffers, preservatives and the like.

Suitable pharmaceutically acceptable carriers are known from, e.g., Remington's Practice of Pharmacy, 13th edition and J. of. Pharmaceutical Science & Technology, Vol. 52, No. 5, Sept-Oct., pp. 238-311.

The recombinant yeast cells are inactivated/killed after cultivation and expression of the transgenes and prior to incorporation into the vaccine composition, or prior to use in the method for vaccination. The inactivation can be carried out with any conventionally available method. Particularly suitable for use in the process according to the invention are liquid inactivation (e.g. liquid inactivation for 3 minutes at 68°C), lyophilization or gamma irradiation (e.g. with 25 or 50 kGy).

As used herein, "vaccinating" or "vaccination" refers to the prevention and/or treatment of a medical condition, such as an infectious disease. The recombinant yeast cell or the vaccine composition are administered to an animal or to a human subject, preferably an animal, in an amount sufficient to elicit an immune response in the subject, preferably, a protective immune response. More preferably, the immune response comprises an immune response that is protective and specific for the immunogenic polypeptide or immunogenic polypeptides that are encoded by each transgene comprised in the at least one genomically integrated expression cassettes.

In a further aspect, the invention relates to a method for the production of a whole yeast vaccine comprising at least one diploid recombinant yeast cell obtained by mating a first and a second haploid recombinant yeast cell according to the invention, the method comprising generation of the diploid recombinant yeast cell by mating of a first and second haploid yeast cell, wherein the first yeast cell is of mating type MATa and comprises a first expression cassette and the second yeast cell is of mating type MATα and comprises a second expression cassette, wherein the first and second expression cassette are stably integrated into the genome of the first and second yeast cell at the same chromosomal locus, wherein the first and second yeast cell are haploid, heterothallic and mating competent; and wherein each transgene encodes an immunogenic polypeptide derived from a pathogen.

In one embodiment of the method for the production of a whole yeast vaccine, the first yeast cell of mating type MATa comprises a third expression cassette and the second yeast cell of mating type MATα comprises a fourth expression cassette, wherein the third and fourth expression cassette are stably integrated into the genome of the first and second yeast cell at the same chromosomal locus. Optionally, the first yeast cell of mating type MATa further comprises a fifth expression cassette and the second yeast cell of mating type MATα further comprises a sixth expression cassette, wherein the fifth and sixth expression cassette are stably integrated into the genome of the first and second yeast cell at the same chromosomal locus. Further optionally, the first yeast cell of mating type MATa further comprises a seventh expression cassette and the second yeast cell of mating type MATα further comprises an eighth expression cassette, wherein the seventh and eighth expression cassette are stably integrated into the genome of the first and second yeast cell at the same chromosomal locus.

In the method for the production of a whole yeast vaccine, the genomically integrated expression cassettes, as well as the transgenes, bidirectional promoter elements, selection marker genes, chromosomal loci, transcription terminators and/or yeast genus, species or strain described hereinabove may be used.

In a preferred embodiment of the method for the production of a whole yeast vaccine, a selection marker gene comprised in the first haploid yeast cell is *ADE2* and a selection marker gene comprised in the second haploid yeast cell is *URA3,* or *vice versa.*

The cultivation and propagation of the recombinant yeasts according to the invention can be carried out with any conventionally available method. Particularly preferred are processes that lead to high cell yields at low cost. These include fermentation processes, especially processes of high cell density fermentation. Fermentation using a fed-batch fermentation protocol has proven to be particularly advantageous.

In another aspect, the invention relates to a method for the production of a diploid recombinant yeast cell as described herein, from a wild type yeast strain comprising (a) generating a first yeast cell of mating type MATa and a second yeast cell of mating type MATα from the wild type yeast strain, such that the first and second yeast cell are each heterothallic, isogenic and mating competent; (b) transforming each of the first and second yeast cell with at least one deletion cassette, such that the at least one deletion cassette stably integrates into the host cell genome at the same chromosomal locus in the first and in the second yeast cell; such that stable integration into the yeast cell genome results in the disruption of a gene required for the synthesis of an essential compound, preferably wherein the essential compound is an amino acid or a nucleobase; (c) transforming each of the first and second yeast cell obtained in step (b) with at least one expression cassette, such that the at least one expression cassette stably integrates into the yeast cell genome at the chromosomal locus comprising the deletion cassette of step (b), such that the ability of the first and second yeast cell to synthesize the essential compound of step (b) is restored; and (d) generating the diploid yeast cell by mating or spheroplast fusion of the first and second haploid yeast cell obtained in step (c); wherein the wild-type yeast strain comprises no genetic selection marker and wherein each deletion cassette comprises at least one marker gene, preferably an auxotrophic marker gene; and optionally IoxP sites flanking the marker gene; and wherein each expression cassette comprises (i) a bidirectional promoter element; (ii) a first transgene and a second transgene, wherein said first and second transgene are located at opposite ends of the bidirectional promoter element and wherein each transgene is operably linked to one side of the bidirectional promoter element; (iii) a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene; and (iv) at least one selection marker; and (v) a sequence for restoring the gene required for the synthesis of an essential compound of step (b).

An "essential compound" is a compound (e.g., a growth factor or metabolite) that is required for the cell to grow, perform biosynthesis and/or proliferate. The cell may be able to produce all essential compounds, in which case it is prototrophic. Cells that are not able to produce all essential compounds require supplementation of growth media with the essential compound, i.e., said cells are auxotrophic for the essential compound. Essential compounds can be vitamins, amino acids, nucleosides, nucleobases or one or more metabolic precursor thereof. Preferably, the essential compound is an amino acid or a nucleobase or one or more metabolic precursor thereof. For example, the essential compound is selected from the group consisting of adenine, uracil, histidine, leucine, lysine, methionine, cysteine, tryptophan, tyrosine and pantothenic acid.

As used herein, the term "wild-type" refers to a yeast strain that comprises no genetic selection marker. The wild-type yeast strain does not comprise a transgene. In some embodiments, the wild-type yeast strain is a yeast strain that occurs in nature, or that has been isolated from a natural source, i.e., a yeast strain that carries no genetic modification that is not found in the naturally occurring yeast strain.

In a specific embodiment, the wild type yeast strain is derived from a genus selected from the group consisting of *Andozyma, Ascobotryozyma, Citeromyces; Debaryomyces, Dekkera, Eremothecium, Issatchenkia, Kazachstania, Kluyveromyces, Kodamaea, Lodderomyces, Pachysolen, Pichia, Saccharomyces, Saturnispora, Tetrapisispora, Torulaspora, Williopsis,* and *Zygosaccharomyces,* preferably from the yeast genus *Kluyveromyces.* In a particularly preferred embodiment, the wild type yeast strain is derived from the yeast species *Kluyveromyces marxianus.* Most preferably, the wild type yeast strain is *Kluyveromyces marxianus* strain NBRC1777.

### Examples

### Example 1: Generation of a parental yeast strain for antigen production

*K. marxianus* wild strain NBRC1777 was used for the generation of a parental yeast strain for antigen production. *K. marxianus* wild strain NBRC1777 was originally isolated from the soil in Japan and has been deposited in the NITE Biological Resource Centers (Shibuya City, Tokyo Prefecture, Japan) under the accession number NBRC1777. DNA sequence information for this strain is available under the GenBank accession numbers AP014599 to AP014607 for chromosomes 1 to 8, respectively.

In contrast to the information in the NITE catalog, the *K. marxianus* wildtype strain NBRC1777 was observed to be homothallic. Most naturally isolated *K. marxianus* strains are homothallic: i.e., they change their mating type spontaneously by "mating-type switching" to create mixed populations of haploid MATa and MATα, and diploid MATa /MATα cells. A two-component mating-type switching mechanism has been identified in *Kluyveromyces lactis,* which requires two transposases (Kat1 and Alpha3) for MAT switching. The Alpha3 transposase switches MATα type cells into the MATa type, and Kat1 switches MATa to MATα type. Cernak et al. identified the *K. marxianus* orthologs of the *K*. *lactis KAT1* and *ALPHA3* genes by reciprocal BLASTp searching followed by molecular-genetic experiments (Cernak, Paul; Estrela, Raissa; Poddar, Snigdha; Skerker, Jeffrey M.; Cheng, Ya-Fang; Carlson, Annika K. et al. (2018), mBio 9 (5)).

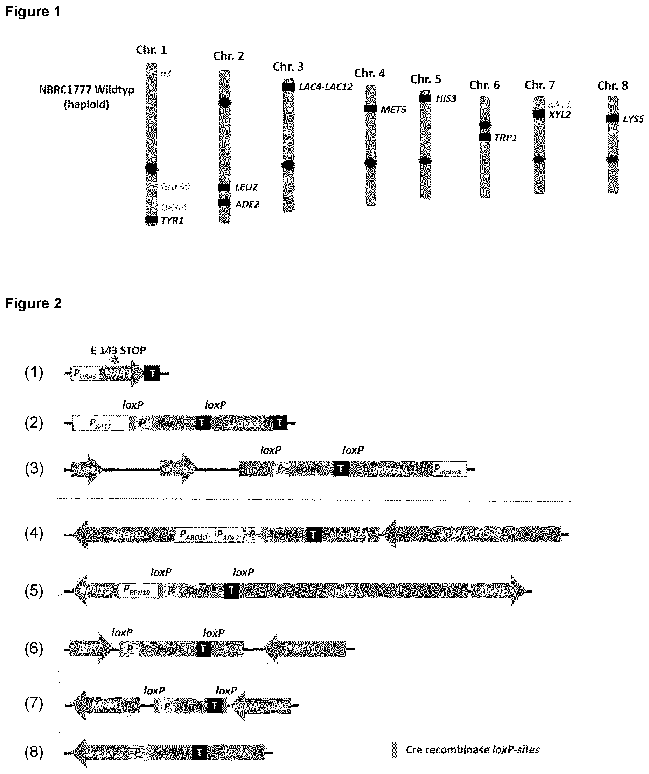

Using *K. marxianus* wild strain NBRC1777, a *ura3* strain SY3 was isolated by selection towards 5-fluoroorotic acid (5-FOA) resistance (Boeke, J. D.; LaCroute, F.; Fink, G. R. (1984), Molecular & general genetics: MGG 197 (2), p. 345-346). To generate a MATa strain, the *KAT1* gene was subsequently inactivated by insertion of a pSY15-based linear M13-F/-R-PCR fragment (Fig. 2-(2)) containing the selectable marker kanMX, flanked by sequences homologous to the chromosomal KAT1 locus. KanR is an antibiotic resistance gene, a dominant marker (Table 5) conferring resistance to the kanamycin derivative G418. Genomic integration results in the formation of G418-resistant yeast colonies. In the cassette, the kanMX marker is flanked by two recognition sites (loxP sites) for the recombinase Cre. By expression of Cre recombinase, the kanMX cassette can be excised very efficiently. One IoxP sequence remains as an insertion mutation and the selection for G418 resistance can be used again for genetic modifications at a further genomic locus.

In order to obtain a suitable MATα strain for crossing experiments, the *ALPHA3* gene was inactivated in a similar way (Fig. 2-(3)) to obtain strain SY41. *ALPHA3* is essential for the mating type change from α to a. After transient expression of the *KmKAT1* gene from pSY16 in such a strain (MATa, *kat1Δ, α*3Δ), clones were obtained that had changed the genotype from MATa to MATα. Due to the successful inactivation of the *ALPHA3* gene, the reciprocal change was no longer possible. The resulting strains are SY11 and SY41 (MATa, *kat1*Δ*::IoxP-kanMX-IoxP, ura3-* and MATa, *kat1*Δ*::IoxP, ura3-*) and SY200 and SY201 (MATα, *kat1*Δ*::IoxP, α3Δ::IoxP-kanMX-IoxP, ura3-* and MATα, *kat1*Δ*::IoxP, α3Δ::IoxP, ura3-*). The four strains are isogeneic meaning that they are genetically identical except for their transposases and the resulting (stable) mating type, which is MATa in SY11 and SY41 and MATα in SY200 and SY201. This allows for crossing to generate homozygous diploids. SY41 and SY201 were used as starting strains for the *K. marxianus* platform provided herein.

The selection marker gene was in each case flanked by direct repeats of the recognition sites (*IoxP*) for the Cre recombinase. The IoxP sites were flanked by sequences homologous to the chromosomal target loci. The length of homologous regions depends on the yeast species that is used as a host. Regions of up to 30-50 bp, which can easily be added to the marker cassette by extending the primers in a single PCR amplification step, can be sufficient to obtain site-specific integration of DNA into the chromosome (e.g. S. *cerevisiae*)*.* In the case of *K. marxianus,* homologous regions of about 1000 bp are recommended. The cloning of integration cassettes for replacement, disruption or mutational purposes requires fusion of multiple DNA fragments (preferential PCR-based) into a linearized vector. For this purpose, individual PCR-generated fragments with appropriately 15 to 20bp of complementary base pairs overlapping at their ends were cloned simultaneously into the linearized pUC19 vector using the In-Fusion cloning method according to manufacturer's instructions (Takara Bio USA Inc).

In-Fusion cloning system functions through a ligase-independent mechanism. Firstly, the In-Fusion enzyme (VVpol, *Vaccinia Virus DNA-polymerase*) creates single-stranded regions at the end of the oligo/PCR insert and linearized vector. This exposes complementary regions on the insert and vector DNA molecules that then spontaneously anneal/fuse through base pairing. Introduction of this annealed/fused DNA into *Escherichia coli* repairs any single-stranded gaps and results in the synthesis of a recombinant vector containing the oligo/PCR insert (Irwin et al., Vaccinia Virus and Poxvirology: Methods and Protocols, Methods in Molecular Biology 2012, vol. 890*).* The pUC19 contained in the In-Fusion HD Cloning kit is a commonly used plasmid cloning vector in *E. coli* that conveys the ampicillin resistance. The molecule is a small double-stranded circle, 2686 base pairs in length, and has a high copy number in *E. coli.* pUC19 carries a 54 base-pair multiple cloning site polylinker that contains unique sites for 13 different hexanucleotide-specific restriction endonucleases (Yanisch-Perron, C.; Vieira, J.; Messing, J. (1985), Gene 33 (1), S. 103-119). pUC19 cannot replicate autonomously in yeast.

The integration cassette was separated from the bacterial vector using suitable restriction enzymes or PCR amplification. The resulting DNA fragment do not contain bacterial sequences. Transformation of host yeast strains (here *K. marxianus*) was carried out according to a protocol described in (Nonklang, Sanom; Abdel-Banat, Babiker M. A.; Cha-aim, Kamonchai; Moonjai, Nareerat; Hoshida, Hisashi; Limtong, Savitree et al. (2008), Applied and environmental microbiology 74 (24), S. 7514-7521). Transformants were verified by PCR and Southern blot analyses, respectively.

### Example 2: Genetic marking of genomic sites for targeted integration into the K. marxianus genome

The strains SY41 (MATa, *kat1*Δ*::IoxP, ura3-*) and SY201 (MATα, *kat1*Δ*::IoxP, α3*Δ*::IoxP, ura3-*) were transformed with linear fragments containing a selectable marker cassette, as described above.

More gene deletions were performed in iterative cycles of such integrations of various marker genes (prototrophic markers genes (*URA3*) or drug resistance marker (*kanMX, hphMX6, natMX6*) successively.

The general procedure for the selection step to test for gene deletions was as follows:
a) Amplification of the cassettes for integrations (i.e., cassettes for replacements, deletions, disruptions or mutations) using pUC19-specific primers (M13-forward/-reverse) in a final volume of 50 µl using Phusion DNA polymerase (Thermo Fisher Scientific).
b) Preparation of a pre-inoculum yeast culture by transferring cells from a YPD plate or frozen stock into YPD medium and incubation by shaking overnight at 30°C (120 rpm).
c) Inoculation of the main culture by transferring a defined cell count from the overnight culture to reach an optical density at 600 nm (OD 600) of 0.1. Incubate the culture at 30°C with shaking (120 rpm) until it reaches OD 600 0.8-1.0.
d) Transformation of deletion cassettes into desired host yeast strains.
e) Selection of transformants by plating of cell suspension on selective SD or YPD-drug plates (YPD-G418, YPD-ClonNat, YPD-Hygromycin) and incubation for 48 h at 30°C.
f) Picking of colonies and transfer onto fresh selective SD plates and appropriate YPD-drug plates (YPD-G418, YPD-ClonNat, YPD-Hygromycin) and incubation for 24 h at 30 °C. Only colonies that grew on YPD-drug plates were used for further analysis.
g) Pick colonies for colony PCR.
h) Confirmation of the described deletions/disruptions by PCR analysis and subsequent Southern blot analysis.

The following *K. marxianus* strains were generated by this method.

### ADE2 locus - deletion (ade2Δ :: ScURA3):

In *K. marxianus,* a defined *ade2*Δ allele was obtained after transformation of PCR fragments generated with template plasmid pSY109 and M13-F/M13-R primer pair.

The region 0 bp to +99 bp in the *ADE2* locus was replaced by the *S. cerevisiae URA3* (*ScURA3*) marker gene via homologous recombination (Figure 2). As consequence the *ADE2* gene is non-functional. The strains become adenine auxotroph and shows a red coloration of the colonies due to the accumulation of an intermediate of adenine biosynthesis. The *ade2*Δ strain can synthesize uracil because the marker in the knockout cassette is the *ScURA3* gene. This strain, now marked by the *ade2* mutant phenotype, allows the integration of foreign genes at the *ADE2* locus. If a suitable integration vector such as pSY147 (Figure 3) and pSY258 (Figure 4) is used, the disruption cassette can be replaced by homologous recombination so that an intact *ADE2* gene is reconstituted (Figure 3) or an intact *MET5* is integrated (Figure 4) with loss of the *ScURA3* marker gene. This is described in further detail below. The loss of *ScURA3* enable a second selection step by 5-FOA counterselection and reduces the effort to identify second site integrations by non-homologous recombinations.

### MET5 locus - deletion (met5Δ :: KanR)

In *K. marxianus,* a defined *met5*Δ allele was obtained after transformation of PCR fragments generated with template plasmid pSY129 and M13-F/M13-R primer pair.

The region -883 bp to +299 bp in the *MET5* locus was replaced by the *E. coli* Kanamycin-resistance gene (*KanR*) via homologous recombination (Figure 2). As a consequence, the *met5*Δ gene is non-functional. The strains become methionine auxotroph but can grow in the presence of the antibiotic G418. Such a *met5*Δ strain allows the integration of foreign genes at the *MET5* locus by selection for methionine growth. If a suitable integration vector is used, the disruption cassette at the *met5* locus can be replaced by homologous recombination and *MET5* is reconstituted by the loss of the *KanR* resistance marker gene (as described below).

Furthermore, *IoxP* sites allow recycling of the *KanR* resistance cassette from the yeast genome by plasmid-based Cre recombinase expression (plasmid pSY16, a pKATUC4-based *K. lactis* plasmid).

### LEU2 locus - deletion (leu2Δ :: HygR)

In *K. marxianus,* a defined *leu2*Δ allele was obtained after transformation of PCR fragments generated with template plasmid pSY241 and M13-F/M13-R primer pair.

The region -455 bp to +942 bp in the *LEU2* locus was replaced by the *E. coli* Hygromycin-B resistance gene (*HygR*) via homologous recombination (Figure 2). As a consequence, the *LEU2* gene is non-functional. The strains become leucine auxotrophic but can grow in the presence of the antibiotic Hygromycin-B. Such a *leu2*Δ strain allows the integration of foreign genes at the *LEU2* locus by selection for leucine growth. If a suitable integration vector is used the disruption cassette can be replaced by homologous recombination so that an intact *LEU2* gene is reconstituted with loss of the *HygR* resistance gene (as described below).

Furthermore, *IoxP* sites allow recycling of the *HygR* resistance cassette (comprising the *hphMX6* marker gene) from the yeast genome by plasmid-based Cre recombinase expression (plasmid pSY16).

### HIS3 locus - deletion (his3Δ :: NrsR) and integration of replacement cassettes

In *K. marxianus,* a defined *his3*Δ null allele was obtained after transformation of PCR fragments generated with template plasmid pSY242 and M13-F/M13-R primer pair.

The region -260 bp to +108 bp in the *HIS3* locus was replaced by the *Streptomyces noursei* Nourseothricin (ClonNAT) resistance gene (*NrsR*) via homologous recombination (Figure 2). As a consequence, the *HIS3* gene is non-functional. The strains become histidine auxotrophic but can grow in the presence of the antibiotic Nourseothricin. Such a *his3*Δ strain allows the integration of foreign genes at the *HIS3* locus. If a suitable integration vector such as pSY275 (Figure 6) and pSY250 (Figure 5) is used the disruption cassette can be replaced by homologous recombination so that an intact *HIS3* gene is reconstituted (Figure 5) or an intact *LEU2* gene is integrated (Figure 6) with loss of the *NrsR* marker gene (as described below). Furthermore, IoxP sites allow recycling of the *NrsR* resistance cassette (comprising the *NrsR* marker gene) from the yeast genome by plasmid-based Cre recombinase expression (plasmid pSY16).

### LAC4-LAC12 locus - deletion (lac4Δ-lac12Δ :: ScURA3)

In *K. marxianus,* defined *lac4Δ-lac12Δ* alleles were obtained after transformation of PCR fragments generated with template plasmid pSY102 and M13-F/M13-R primer pair.

The region +205 bp in *LAC4* to +183 bp in *LAC12* was replaced by the *S. cerevisiae URA3* marker gene via homologous recombination (Figure 2). As a consequence, the *LAC4-LAC12* genes are non-functional and the strain is not able to grow on lactose as sole carbon source but can synthesize uracil due to the integrated *ScURA3* gene. Such a *lac4-lac12Δ* strain allows the integration of foreign genes at the *LAC4-LAC12* locus by selection for lactose growth. If a suitable integration vector is used the disruption cassette can be replaced by homologous recombination so that an intact *LAC4* and *LAC12* genes, respectively, are reconstituted with loss of the *ScURA3* marker gene (as described below).

### Example 3: Codon optimization of PRVA antigens and gene synthesis

Adaptation of the natural *VP2, VP4, VP6* and *VP7* genes of the *Porcine Rotavirus A* strain RVA/Pig-tc/ESP/OSU-C5111/2010/G5P[7] to the codon usage of *K. marxianus* was analyzed acccording to (Villada, Juan C.; Brustolini, Otávio Jose Bernardes; Batista da Silveira, Wendel (2017), DNA research: an international journal for rapid publication of reports on genes and genomes 24 (4), S. 419-434) and using the genomic tRNA database GtRNAdb containing tRNA gene predictions. The translation efficiency of a codon was obtained from the tRNA Adaptation Index (tAl5), where codon relative adaptiveness (w) is the adaptation of a codon to the pool of available cognate tRNAs in the genome which incorporates the different possible tRNA and the wobble pairing rules (Villada et al. 2017).

The *VP2, VP4, VP6* and *VP7* genes were codon optimized, the encoded protein amino acid sequence of each gene remaining unchanged. Codon optimized *VP2, VP4, VP6* and *VP7* genes all with linker and C-terminal 3HA epitope were synthesized by GENSCRIPT company, subsequently cloned in pUC19 cloning vector to obtain pUC19-PRVA-VP2-opt, pUC19-PRVA-VP4-opt, pUC19-PRVA-VP6-opt2 and pUC19-PRVA-VP7-opt. Confirmation that the synthetic gene sequence is correct were performed by sequencing.
GenBank accession number of *PRVA VP2* gene: KJ450843.1: SEQ ID NO: 7
GenBank accession number of *PRVA VP4* gene: KJ450845.1: SEQ ID NO: 8
GenBank accession number of *PRVA VP6* gene: KJ450847.1: SEQ ID NO: 9
GenBank accession number of *PRVA VP7* gene: KJ450849.1: SEQ ID NO: 10

All used antigenic sequences mentioned are derived from the virus strain *Rotavirus A strain* RVA/Pig-tc/ESP/OSU-C5111/2010/G5P[7].

### Example 4: Generation of integration vectors which allow for inducible antigenic gene expression into the host genome

All modifications were performed in two steps essentially using a selection/counter-selection strategy. Genetic modifications were conducted in two steps: a selection step, in which the disruption/deletion cassette (e.g. *ScURA3, kanMX, natMX6, hphMX6,* see Figure 2) was inserted in the targeted genomic location (as described above), and a special counter-selection step, where the knock out cassette is replaced by an expression cassette resulting in the desired genetic modification (e.g. two antigenic sequences (transgenes) with a bidirectional promoter and transcription terminators).

Integration vectors for expression cassettes were generated as follows.Cloning of expression cassettes for replacements require fusions of multiple DNA fragments (preferential PCR-based) directionally into a linearized vector. For this purpose, independent PCR-generated fragments (for example PCR fragment 1: GOI1-3HA-opt, PCR fragment 2: bidirectional promoter, PCR fragment 3: GOI2-3HA-opt,) with appropriate complementary ends of 15 or 20bp overlap at their ends were cloned simultaneously into the linearized pUC19-based target vector with homologous sequences to the appropriate target chromosomal loci for homologous recombination using the In-Fusion cloning method according to manufacturer's instructions (Takara Bio USA Inc).

### Example 5: Integration of expression cassettes into the host chromosome

The general strategy implies that in one of the haploid strains, the mutated gene marking the insertion site is restored by the insertion of the expression cassette comprising the transgenes whereas in the other haploid strains, a new selective marker is introduced as part of the expression cassette linking the old auxotrophic marker gene to a new prototrophic one and both marker genes to the expression cassette containing the antigen coding sequence. This has the advantage that upon crossing of these strains, diploid recombinant yeast cells can be separated from haploids by selection. Since each transgene is tightly linked to the selectable marker gene(s), it is possible to distinguish diploid progeny from haploids by their phenotypes on plates. Since haploids may arise from diploids by meiosis, which is associated with meiotic recombination leading to reciprocal exchanges between homologous chromosomes, it is important for stability of the generated strains to counter-select against meiotic segregants or to find meiotic segregant with new combinations of antigen genes, if desired.

The general procedure of insertion of expression cassettes following counter-selection steps to test for removal of knock out cassettes:
a) Separation of the replacement cassettes from the pUC19 based vector by PCR or digestion using restrictions enzymes, respectively.
b) Transformation of replacement cassettes in desired host yeast strains as described in (Nonklang, Sanom; Abdel-Banat, Babiker M. A.; Cha-aim, Kamonchai; Moonjai, Nareerat; Hoshida, Hisashi; Limtong, Savitree et al. (2008), Applied and environmental microbiology 74 (24), S. 7514-7521).
c) Selection of transformants by plating of cell suspension on selective SD plates and incubation 48 h at 30 °C.
d) Picking of colonies and transfer onto fresh selective SD plates and appropriate YPD-drug plates (YPD-G418, YPD-ClonNat, YPD-Hygromycin) and incubate for 24 h at 30 °C. Only colonies that did not grow on YPD-drug plates were used for further analysis.
e) Picking of colonies that grew on SD plates and which were sensitive to the appropriate drug substance for colony PCR.
f) Confirmation of successful integration of expression cassettes by sequencing the PCR products.

The following *K. marxianus* recombinant yeast cells were generated by this method.

### Replacements on ADE2 locus with restoration of ADE2 gene in strains with MATa-background

The expression cassette of the pUC19 based vector pSY147 contains consecutively *ARO10*, *ARO10* promoter (*P_{ARO10}*), *TEF1* terminator from *Ashbya gossypii* (*T_{AgTEF1}*)*, PRVA-VP4-3HA-opt* (SEQ ID NO: 35), bidirectional promoter (*P_{LAC4-LAC12}*), *PRVA-VP4-3HA-opt* (SEQ ID NO: 35), *T_{AgTEF1}, ADE2* promoter (*P_{ADE2}*), *ADE2* and the ORF *KLMA_20599'* (not characterized). For generation of recombinant *K. marxianus* strains, the pUC19-based vector is linearized with appropriate restriction enzymes (*Lgu*I & *Kpn*I) and transformed into SY267. After successful homologous recombination into the chromosomal *ADE2* region of the host genome, the *URA3* gene is replaced and the *ADE2* gene is restored. The resulting strain (SY277) can synthesize adenine (Ade+), is uracil-auxotrophic and resistant to FOA (ura-, FOA^{R}) and expresses two copies of *PRVA-VP4-3HA-opt* under control of a bidirectional promoter (*P_{LAC4-LAC12}*), respectively (Figure 3).

### Replacements on ADE2 locus with integration of intact MET5 instead of ADE2 in strains with MATα-background

The expression cassette of the pUC19 based vector pSY258 contains consecutively *ARO10, P_{ARO10}, T_{AgTEF1}, PRVA-VP2-3HA-opt* (SEQ ID NO: 34), *P_{LAC4-LAC12}, PRVA-VP2-3HA-opt* (SEQ ID NO: 34), *T_{AgTEF1}, MET5* promoter (*P_{MET5}*), *MET5* and the ORF *KLMA20599* (not characterized). For generation of recombinant *K. marxianus* strains, the pUC19-based vector is linearized with appropriate restriction enzymes (A*at*II & *Kpn*I) and transformed into SY280. After successful homologous recombination into the chromosomal *ADE2* region of the host genome, the *URA3* gene is replaced and the *MET5* gene is integrated. The resulting strain (SY281) can synthesize methionine (Met+), is uracil-auxotrophic and resistant to FOA (ura-, FOA^{R}) and expresses two copies of *PRVA-VP2-3HA-opt* under control of a bidirectional promoter (*P_{LAC4-LAC12}*), respectively (Figure 4).

### Replacements on HIS3 locus with restoration of HIS3 in strains with MATα-background

The expression cassette of the pUC19 based vector pSY250 contains consecutively *MRM1, P_{MRM1}, T_{AgTEF1}, PRVA-VP6-3HA-opt2* (SEQ ID NO: 36), *P_{LAC4-LAC12}, PRVA-VP6-3HA-opt2* (SEQ ID NO: 36), *T_{AgTEF1}, HIS3* promoter *(P_{His3}), HIS3,* ORF KLMA_50039 (not characterized). For generation of recombinant *K. marxianus* strains, the pUC19-based vector is linearized with appropriate restriction enzymes (*Pvu*I & *Pae*I) and transformed into SY281. After successful homologous recombination into the chromosomal *HIS3* region of the host genome, the Nourseothricin resistance gene *(NrsR)* is replaced and the *HIS3* gene is integrated. The resulting strain (SY282) can synthesize histidine (His+), is sensitive to Nourseothricin and expresses two copies of *PRVA-VP6-3HA-opt2* under control of a bidirectional promoter (*P_{LAC4-LAC12}*), respectively (Figure 5).

### Replacements on HIS3 locus with integration of intact LEU2 instead of HIS3 in strains with MATa-background

The expression cassette of the pUC19 based vector pSY275 contains consecutively *MRM1, P_{MRM1}, T_{AgTEF1}, PRVA-VP7-3HA-opt* (SEQ ID NO: 37), *P_{LAC4-LAC12}, PRVA-VP7-3HA-opt* (SEQ ID NO: 37), *T_{AgTEF1}, LEU2* promoter *(P_{LEU2}), LEU2,* ORF KLMA50039 (not characterized). For generation of recombinant *K. marxianus* strains, the pUC19-based vector is linearized with appropriate restriction enzymes (*Bgl*I & *Pae*I) and transformed into SY277. After successful homologous recombination into the chromosomal *HIS3* region of the host genome, the Nourseothricin resistance gene *(NrsR)* is replaced and the *LEU2* gene is integrated. The resulting strain (SY278) can synthesize leucine (Leu+), is sensitive to Nourseothricin and expresses two copies of *PRVA-VP7-3HA-opt* whose expression is controlled by one of the bidirectional promoters (*P_{LAC4-LAC12}*) (Figure 6).

### Example 6: Optimization of immunogenic polypeptide production by modification of the GAL80 locus

The effect of *gal80* deletion on transgene expression under the control of *P_{LAC4-LAC12}* and *P_{GAL1-GAL10}* was assessed. Different *K. marxianus* strains expressing *PRVA*-derived immunogenic polypeptides (VP2, VP4, VP6 and/or VP7) were modified by deletion of the gene coding for repressor protein Gal80. The effect of *gal80* deletion in *P_{LAC4-LAC12}* and *P_{GAL1-GAL10}* dependent immunogenic polypeptide production was assessed by Western blotting. The corresponding yeast strain with wild-type *GAL80* was used as reference strain. Presence (+) or absence (-) of a *gal80* deletion are shown above.

Western blots were carried out using 4-20% Mini-PROTEAN TGX Stain free gradient gels (Bio-Rad, CA) loaded with about 0.3 ODE units of freshly harvested yeast cultures. As loading control, gels were activated for Stain free total protein staining by UV exposure for 45 seconds using a Bio-Rad Chemidoc MP imager. Subsequently, proteins were transferred to nitrocellulose membranes (Bio-Rad) using a Transblot Turbo apparatus (Bio-Rad). Membranes were blocked with 5% (w/v) non-fat milk in TBST (Tris-buffered saline (TBS) with tween, 0.05 M Tris, pH 7.4, 0.1 M NaCl, 0.05% (v/v) Tween 20) for 1 hour and then incubated with anti-HA (F-7, sc-7392; 1:3000, Santa Cruz Biotechnology) for 1 hours. Membranes were then washed three times for 10 minutes and then incubated with goat anti-mouse horseradish peroxidase antibody (1:3000, Invitrogen) for 1 h. Membranes were again washed with TBST three times for 10 minutes and incubated with Clarity chemiluminescence substrate (Bio-Rad), imaged on the Chemidoc MP device. Band intensities were quantified by Imagelab 6.0 software (Biorad) and calculated using an internal standard (MalE) taking into account the loaded OD units.

The deletion of *gal80* leads to a significant increase in the concentration of immunogenic polypeptide (Fig. 9). The effect is particularly pronounced in glucose containing media.

### Example 7: Immunization of mice and serological tests

The immunogenic potential of recombinant haploid yeast cells as whole yeast vaccines (WYV) was tested. The cells obtained in Examples 5 and 6 were used for subcutaneous application in mice. No abnormalities in weight gain or animal behaviour towards the WYV suspension were observed in previous tolerance studies.

Figures 10 describe the vaccination in mice with subcutaneously applied, thermal-inactivated, whole yeast cells of the *K. marxianus* strains generated in Example 5 and 6, i.e., haploid recombinant *K*. *marxianus* yeast cells expressing PRVA VP2, VP4, VP6 or VP7 individually.

The immunization study addressed the question, whether the vaccination induce specific antibodies against the applied antigens of the Porcine Rotavirus A.

The immunization experiments were performed with eight weeks old female BALB/C mice (Charles River Laboratories) in groups of five individuals. Per immunization/boost application, 100 µl suspension (containing 1.0 to 5.0 mg of yeast dry weight in physiologic NaCl solution was injected per mouse. During the injection procedure, the mice were anesthetized with 1.5% isoflurane.

Immunization was performed following the schedule which is shown in Figure 10A. After initial injection, the mice were boosted twice in two weeks intervals (i.e., at day 14 and 28, respectively). Two weeks after the last application, the mice were euthanized with 1.5% isoflurane and blood samples were taken. Mouse serum samples were assayed for the presence of anti-PRVA specific antibodies (VP2, VP4, VP6 and VP7) using an indirect IgG ELISA.

### Indirect ELISA assay

An indirect IgG ELISA for detection of PRVA-specific antibodies in sera (murine, porcine or other) was developed and evaluated. For this purpose, the expression vector pET15b and the E. *coli* strain Rosetta(DE3)pLysS (Novagen) were used for the expression of recombinant VP2, VP4, VP6 (expressed as full length proteins of 105.5 kDa, 88.6 kDa and 46.6 kDa, respectively) and VP7 (expressed as aa50Δ N-terminal truncated variant of 33.6 kDa) with C-terminal 6xHIS epitope. Protein expressions and purifications were carried out as described below.

*E. coli* cultures carrying PRVA expression vectors (pET15b, C-terminal tandem 6xHis) were grown at 37 °C until OD600 ∼2. Protein expression was induced using 1.0 mM IPTG, further incubated at 37 °C for 2h and harvested by centrifugation. The bacteria were resuspended in lysis buffer (50 mM Tris pH 7.8, 300 mM NaCl, 10 mM imidazole, 5% glycerol, 1 mM β-mercaptoethanot, protease inhibitor tablets (Roche),1/10(v/v) lysozyme [10mg/ml]) and incubated for 1h at 4°C. 1/10 volume of 10% Triton X-100 was added and the lysate was incubated for an additional hour at 4°C by shaking. Then, 1/15 (v/v) of 5% aqueous protaminsulfate for DNA-precipitation was added and incubated for 20 min at 4°C. The lysate was cleared by centrifugation (15 000 g for 1 h at 4 °C) and the supernatant was incubated with NiNTA resin for 1 h at 4 °C. The bound protein was washed once with lysis buffer, once with high salt buffer (50 mM Tris pH 7.8, 1M NaCl, 1, 5% glycerol, 1mM mM β-mercaptoethanot), once with high imidazole buffer (50 mM Tris pH 7.8, 300 mM NaCl, 40 mM imidazole, 5% glycerol, 1 mM β-mercaptoethanol) und was finally eluted with elution buffer (50 mM Tris pH 7.8, 300 mM NaCl, 250 mM imidazole, 5% glycerol, 1 mM β-mercaptoethanol). The elution fraction was dialyzed overnight in storage buffer (25 mM Tris pH 7.8, 150 mM NaCl, 10% glycerol, 1 mM β-mercaptoethanol).

Polystyrene microtiter plates (Nunc-Immuno^{™} MicroWell^{™} 96 well solid plates, Sigma-Aldrich) were coated with 50 ng of recombinant PRVA proteins per well (maximum volume: 400 µl), diluted with 100 µl phosphate buffered saline (pH 7.4) and incubated overnight at 4°C. Plates were washed three times with TBST (25 mM Tris, 0,15 M NaCl, 0,05 % Tween-20, pH 7,5, Thermo Fisher Scientific) and then blocked by the addition of 200 µl of blocking buffer per well (Tris-buffered saline pH 7.4 with 0,05% Tween 20, Thermo Fisher Scientific) and incubation at 4°C for 2 hours. After blocking, the plates were washed three times with TBST and 100 µl aliquots of serum specimens, diluted 1:250 in blocking buffer, were added to the wells. Antigen concentration and serum dilution level for this assay were determined by titration to reach optimal conditions for sensitivity and specificity (data not shown). After 2 hours of incubation at 4°C, the plates were washed three times with TBST. HRP-conjugated (HRP = horseradish peroxidase) goat anti-mouse IgG (Invitrogen) diluted 1:2500 in blocking buffer, were added to the wells in 100 µl aliquots and incubated for 1 h at 4°C. The plates were washed as described above. Binding of specific antibodies was visualized by the addition of 100µl/well of 1-Step^{™} Ultra TMB-ELISA Substrate Solution (Thermo Fisher Scientific). After 30 min of incubation at the room temperature, the reaction was stopped by adding 50 µL/well of 1N sulphuric acid and the optical density (OD) was measured at 450 nm (reference filter 540 nm).

Two weeks after the last subcutaneous application, the mice sera were analyzed for the presence of anti-VP2, -VP4, -VP6 and -VP7 antibodies, respectively, with an indirect IgG ELISA that was established for this study. In comparison to animals that were treated with non-PRVA expressing *K*. *marxianus* strain SY41, all vaccine strains showed significantly increased levels of PRVA specific antibodies anti-VP2, -VP4, -VP6 and -VP7, respectively.

PRVA-positive pig sera served as a positive control. The zero sera before the vaccination or a nonspecific antibody were used as a negative control. The results of the ELISAs are shown in Figure 10.

Using the recombinant haploid yeast cells of Example 5 and 6, successful immunization against all four transgene-expressed PRVA antigens could be achieved.

### Example 8: Generation of diploid recombinant yeast cells expressing PRVA antigens by mating

Diploid recombinant yeast cells comprising multiple transgenes were obtained by mating of previously generated haploid recombinant yeast cells. The auxotrophic strains used for this example are the SY278 (MATa, *kat1Δ, ADE2::2xVP4-3HA-opt, met5Δ, his3::LEU2::2xVP7-3HA-opt, leu2Δ, gal80, ura3)* and SY282 (MATα, *kat1Δ, α3Δ, ade2::MET5::2xVP2-3HA-opt, met5Δ, HIS33::2xVP6-3HA-opt, leu2Δ, gal80*) (Figures 7 and 8), which require supplementation with methionine, histidine and uracil or adenine and leucine, respectively, for growth.

In principle, any two complementing sets of auxotrophic strains resulting from genomic integration at chromosomal loci selected from Tables 3 and 4 could be used for the construction and maintenance of diploid strains. The haploid recombinant yeast cells used in this example are especially suited for this method for two reasons. Firstly, they grow more slowly than diploid strains that are the result of their mating or fusion. Thus, if a small number of haploid cells arise through meiosis or other mechanism, the diploid strain will outgrow them in culture. Secondly, it is easy to monitor the ploidy state by striking out the culture on plates containing all auxotrophic markers (LEU, MET, HIS, ADE, URA) vs. plates without any of these supplements. A different number of colonies comparing both plate types allow a percentage calculation of haploids present in the culture.

The diploid strains were constructed by mating the haploid strains, followed by selection on plates without any amino acids or bases. Since both expression cassettes had been integrated into the *ADE2* and *HIS3* locus, haploid progeny resulting from diploid sporulation could only contain two of the four expression cassettes that were genomically integrated at homologous chromosomal loci. Consequently, only diploid cells comprising all five prototrophic genes (*ADE2, MET5, HIS3, LEU2, URA3*) were able to grow without metabolite supplementation and capable of simultaneously expressing all immunogenic polypeptides, PRVA-VP2, -VP4, VP6 and VP7.

The general procedure for mating of haploid parental strains according the description in (Kutyna, Dariusz R.; Cordente, Antonio G.; Varela, Cristian (2014), Methods in molecular biology (Clifton, N.J.) 1152, S. 157-168):
a) Inoculation of two haploid strains from a YPD plate or frozen stocks into YPD medium (5 mL) and incubation overnight at 30 °C and shaking (120 rpm).
b) Transfer 100 µL of each (haploid) culture into the same sterile 1.5-2.0 mL microcentrifuge tube and mixing thoroughly by vortexing or pipetting.
c) Transfer of mixed culture onto a fresh selective SD plate, air-drying, and incubation at 30 °C. During this incubation period, cells of opposite mating types fuse to create diploids.
d) Pick single colonies and plate out several serial dilutions of mated mixture selective SD plates and incubate for at 30 °C until single colonies are fully developed.
e) Verify ploidy of cells by using mating type PCR primers.

Formation of diploids was confirmed by colony PCR (cPCR) using a primer set specific to the MAT loci (MATa and MATα, respectively), as well as the four antigenic sequences integrated at the *ADE2* and *HIS3* loci (Figure 11). Primer sequences are listed in Table 6. A stable diploid population was observed in the strain SYD4.

**Table 6: Primers used for PCR-based ploidy analysis.**

| **Reaction** | **Primer Name** | **Primer Sequence (5' to 3')** | **Primer Description** | **Product size** |
|---|---|---|---|---|
| MATa | P1 | CAAATGTTGTGGCTGCACCTAC | SLA2-F | 1063 bp |
| | P2 | CAAACGTATGCGTACGAAATC | MATa-R | |
| MATα | P1 | CAAATGTTGTGGCTGCACCTAC | SLA2-F | 1707 bp |
| | P3 | CTCAGGATATAGGAAACATGAAGG | MATalpha-R | |
| *ADE2-WT* | P200 | AAGAAGACTTAGCCATGGCA | KmARO10-F | 3697 bp |
| | P204 | GAAGAACCAATAATGTTAATATGAC | KmADE2-R | |
| *ade2Δ :: ScURA3*/ *ade2Δ :: Scura3* | P200 | AAGAAGACTTAGCCATGGCA | KmARO10-F | 4451 bp |
| | P204 | GAAGAACCAATAATGTTAATATGAC | KmADE2-R | |
| *ADE2::2xVP4* | P200 | AAGAAGACTTAGCCATGGCA | KmARO10-F | 7152 bp |
| | P162 | TATGAAAACTGATTATCGTCCTGT | P-LAC4-LAC12 | |
| *ade2 :: ME T5::2xVP2* | P200 | AAGAAGACTTAGCCATGGCA | KmARO10-F | 7495 bp |
| | P162 | TATGAAAACTGATTATCGTCCTGT | P-LAC4-LAC12 | |
| *HIS3-WT* | P532 | TATGTCTGCTTTGGAGCACA | KmMRM1-F | 3390 bp |
| | P512 | GTTGACGTTAACATGATTGGA | KmAGP1-R | |
| *his3Δ :: NrsR* | P532 | TATGTCTGCTTTGGAGCACA | KmMRM1-F | 3547 bp |
| | P512 | GTTGACGTTAACATGATTGGA | KmAGP1-R | |
| *HIS3::2xVP6* | P118 | ATCTTTCAGTTCTCGATGAG | P-LAC4-LAC12 | 6190 bp |
| | P512 | GTTGACGTTAACATGATTGGA | KmAGP1-R | |
| *his3 :: LEU2::2xVP7* | P118 | ATCTTTCAGTTCTCGATGAG | P-LAC4-LAC12 | 5999 bp |
| | P512 | GTTGACGTTAACATGATTGGA | KmAGP1-R | |
| *MET5-WT* | P375 | CGGCAAAAAAACGCAGGATCTG | Km-P-RPN10-F | 3627 bp |
| | P172 | ATTGAGAATCACCTAAACCAAACACGG | KmMET5-R | |
| *met5Δ :: KanR* | P375 | CGGCAAAAAAACGCAGGATCTG | Km-P-RPN10-F | 4086 bp |
| | P172 | ATTGAGAATCACCTAAACCAAACACGG | KmMET5-R | |
| *LEU2-WT* | P533 | ACTGCAGTTAAGATTCCAGC | KmRPL7-F | 4604 bp |
| | P514 | GCCAGAAATACAACACCTCT | Km-T-NFS1 | |
| *leu2Δ :: HygR* | P533 | ACTGCAGTTAAGATTCCAGC | KmRPL7-F | 4897 bp |
| | P514 | GCCAGAAATACAACACCTCT | Km-T-NFS1 | |

The diploid strains were examined for growth and antigen expression by Western blot analysis. Western blots were carried out as described above.

Growth behaviour and recombinant protein expression patterns were compared between VP2, VP4, VP6 and VP7 expressing haploid *K. marxianus* strains and their isogenic diploid counterpart. The diploid *K. marxianus* offers a higher protein production compared to its haploid counterparts (Figure 12). Therefore, the use of diploid strains as hosts for recombinant protein expression (such as immunogenic polypeptides) results in an increased yield of recombinant protein per dry yeast weight or culture volume.

Diploid recombinant yeast strain stability analysis: The percentage of diploid cells in the population was determined by plating on selective media and followed for 48 h of cultivation (in shake flask culture). Several hundred colonies were analyzed by colony PCR, revealing that more than 98% of colonies growing on selection media were diploid (data not shown). As controls, parental haploid strains carrying either leu- and ade- or met- and his- auxotrophies did not form any colonies on selection plates missing all four nutrients (amino acids or nucleobase, respectively). Therefore, despite of occasional sporulation events during fermentation, it was observed that the majority of the yeast population was maintained in diploid state.

### Example 9 Generation of diploid recombinant yeast cells expressing ASFV antigens by mating

A further diploid recombinant yeast strain expressing African Swine Fever Virus (ASFV) proteins p12, p32, p62 and p72, each with 3HA epitope tag, was generated as follows.

The full-length open reading frame (ORF) of codon optimized genes O61R (coding for the protein p12); CP204L (coding for p32); CP530R (coding for p62) and B646L (coding for p72) were synthesized based on ASFV isolate Georgia/2007 (GeneBank: FR682468.1) and cloned into the common plasmid vector pUC19 (GENESCRIPT). Confirmation of the synthetic gene sequence was performed by sequencing.

Integration vectors which allow antigenic gene expression into the host genome were generated using the In-Fusion cloning method as described above (Example 4).

For generation of recombinant strains, the pUC19-based vectors (pSY123, pSY131, pSY278, pSY279) were linearized with appropriate restriction enzymes and step-wise transformed in haploid *K. marxianus* strains (MATa or MATα). Integration into the host genome occured by homologous recombination into the chromosomal *LAC4-LAC12, ADE2, LEU2* region (SY255, MATα) or *LAC4-LAC12, ADE2, HIS3* regions (SY262, MATa) (Table 7, Fig 13). Each resulting strain expresses six copies of *ASFV* genes whose expression is controlled by one of the bidirectional promoters (*P_{LAC4-LAC12} or P_{GAP1-ADH2}*)*.* Integrity and correct integrations of the recombinant genes was confirmed by PCR, Southern Blot as well as Western Blot analysis.

**Table 7: Haploid and diploid recombinant yeast cells used in this exapmle**

| **Strain** | **MAT** | **Integrations** | ***ASFV antigens*** |
|---|---|---|---|
| SY255 | haploid; MATα | *LAC4-LAC12::p12+p32, ADE2::p62+p72, LEU2::2xp72, his3Δ::NrsR, gal80Δ::Scura3, ura3-* | 1xp12, 1xp32, 1x p62, 3x p72 |
| SY262 | haploid; MATa | *LAC4-LAC12::p12+p32, ADE2::p62+p72, HIS3::2xp72, leu2Δ::HygR, gal80Δ::ScURA3* | 1xp12, 1xp32, 1x p62, 3x p72 |
| SYD2 | diploid; MATα /MATa | mating of SY255 and SY262 | 2x p12, 2x p32, 2x p62, 6x p72 |

Optimization of immunogenic polypeptide production was achieved by deletions of the *GAL80* locus.

The diploid recombinant yeast cells SYD2 comprising multiple transgenes were obtained by mating of previously generated haploid recombinant yeast cells followed by selection on plates without any amino acids or bases, i.e., selection for prototrophic strains. Conformation of diploids was accomplished by colony PCR (cPCR) using a primer set specific to the MAT loci (MATa and MATα, respectively), as well as the four antigenic sequences integrated at the *LAC4-LAC12, ADE2, LEU2* and *HIS3* loci. A stable diploid population was observed in the strain SYD2. Subsequently, comparison of growth and recombinant protein production between haploid strains (SY255, SY262) and their isogenic diploid strain SYD2 obtained by mating were determined (Fig. 13).

### Items of the Invention

In view of the disclosure provided herein, it will be appreciated that the present invention also encompasses the following items.
1. A recombinant yeast cell, comprising at least one genomically integrated expression cassette, wherein each expression cassette comprises
   (i) a bidirectional promoter element;
   (ii) a first transgene and a second transgene, wherein said first and second transgene are located at opposite ends of the bidirectional promoter element and wherein each transgene is operably linked to one side of the bidirectional promoter element;
   (iii) a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene; and
   (iv) at least one selection marker.
2. The recombinant yeast cell according to item 1, comprising at least two genomically integrated expression cassettes, optionally comprising 2, 3, 4, 5, 6, 7 or 8 genomically integrated expression cassettes.
3. The recombinant yeast cell according to any one of the preceding items, wherein each transgene encodes an immunogenic polypeptide derived from a pathogen, or an immunogenic fragment thereof.
4. The recombinant yeast cell according to item 3, wherein the pathogen is a viral pathogen.
5. The recombinant yeast cell according to item 4, wherein the viral pathogen is a viral pathogen with a multilayer capsid and one or more spike proteins.
6. The recombinant yeast cell according to item 4, wherein the viral pathogen belongs to the family of *Reoviridae.*
7. The recombinant yeast cell according to item 6, wherein the viral pathogen is a rotavirus, preferably Porcine Rotavirus A (PRVA) or Porcine Rotavirus C (PRVC), more preferably Porcine Rotavirus A (PRVA).
8. The recombinant yeast cell according to item 6, wherein the viral pathogen is an orbivirus, preferably African Horse Sickness Virus (AHSV) or Bluetongue virus (BTV).
9. The recombinant yeast cell according to any one of items 3 to 7, wherein each immunogenic polypeptide comprises a polypeptide selected from the group consisting of Porcine Rotavirus A (PRVA) VP2, VP4, VP6, VP7, NSP2 and NSP4, and one or more immunogenic fragment thereof.
10. The recombinant yeast cell according to item 9, wherein each immunogenic polypeptide comprises an amino acid sequence selected from the group consisting of SEQ ID NO: 1 (PRVA VP2), SEQ ID NO: 2 (PRVA VP4), SEQ ID NO: 3 (PRVA VP6), SEQ ID NO: 4 (PRVA VP7), SEQ ID NO: 5 (PRVA NSP2) and SEQ ID NO: 6 (PRVA NSP4), and one or more immunogenic fragment thereof, optionally from the group consisting of the amino acid sequences set forth in SEQ ID NOs: 1, 2, 3, and 4, and one or more immunogenic fragment thereof.
11. The recombinant yeast cell according to any one of items 4 to 10, wherein said immunogenic polypeptide, or polypeptides, are capable of assembling into a virus-like particle (VLP) inside the recombinant yeast cell.
12. The recombinant yeast cell according to any one of the preceding items, wherein the first transgene and the second transgene are identical within each expression cassette.
13. The recombinant yeast cell according to any one of the preceding items, wherein the first transgene and the second transgene are different within each expression cassette.
14. The recombinant yeast cell according to any one of the preceding items, wherein the recombinant yeast cell comprises at least two genomically integrated expression cassettes and wherein the first transgene and the second transgene of each expression cassette are different from the first and second transgene of each further expression cassette.
15. The recombinant yeast cell according to any one of the preceding items, wherein the bidirectional promoter element is a native bidirectional yeast promoter element.
16. The recombinant yeast cell according to item 15, wherein the native bidirectional yeast promoter element is selected from the group consisting of a *LAC4*/*LAC12* promoter, a *GAL1*/*GAL10* promoter and a *GAP1*/*ADH2* promoter.
17. The recombinant yeast cell according to item 16, wherein the native bidirectional yeast promoter element comprises a nucleic acid sequence selected from the group consisting of SEQ ID NOs 25 and 26.
18. The recombinant yeast cell according to any one of the preceding items, wherein the bidirectional promoter element is a hybrid bidirectional promoter element.
19. The recombinant yeast cell according to item 18, wherein the hybrid bidirectional promoter element is selected from the group consisting of a *TDH3*/*ADH1* promoter, a *GAL1*/*GAL10-GPD* promoter and a *TEF1*/*PGK1* promoter.
20. The recombinant yeast cell according to item 18, wherein the hybrid bidirectional promoter element comprises a nucleic acid sequence selected from the group consisting of SEQ ID NOs: 27 and 28.
21. The recombinant yeast cell according to any one of the preceding items, wherein the bidirectional promoter element enables similar expression levels of the first transgene and the second transgene.
22. The recombinant yeast cell according to any one of items 1 to 20, wherein the bidirectional promoter element enables different expression levels of the first transgene and the second transgene.
23. The recombinant yeast cell according to any one of the preceding items, wherein the bidirectional promoter element enables inducible or repressible expression of the first and/or the second transgene.
24. The recombinant yeast cell according to any one of items 1 to 22, wherein the bidirectional promoter element enables constitutive expression of the first and/or the second transgene.
25. The recombinant yeast cell according to any one of the preceding items, wherein each expression cassette is genomically integrated at a chromosomal locus selected from the group consisting of *ADE1, ADE2, ADE8, ECM31, HIS2, HIS3, HIS5, LEU1, LEU2, LYS2, LYS5, MET5, MET17, TRP1, TRP3, TRP4, TRP5, TYR1, URA3, URA5, FCY1, GAP1, LAC4* + *LAC12, XYL1 and XYL2.*
26. The recombinant yeast cell according to item 25, wherein each expression cassette is genomically integrated at a chromosomal locus selected from the group consisting of *ADE2, LEU2, MET5* and *HIS3.*
27. The recombinant yeast cell according to any one of the preceding items, wherein the selection marker is a resistance marker or a metabolic selection marker, preferably a metabolic selection marker.
28. The recombinant yeast cell according to any one of the preceding items, wherein the first and second transcription terminator are each selected from the group consisting of a *TEF1* terminator from *Ashbya gossypii* (SEQ ID NO: 29), a *HIS5* terminator from *K. marxianus* (SEQ ID NO: 30), a *CPS1* terminator from *K. marxianus* (SEQ ID NO: 31), a *CYC1* terminator from *S. cerevisiae* (SEQ ID NO: 32) and an *ADH1* terminator from *S. cerevisiae* (SEQ ID NO: 33).
29. The recombinant yeast cell according to any one of the preceding items, wherein the recombinant yeast cell is derived from a genus selected from the group consisting of *Andozyma, Ascobotryozyma, Citeromyces; Debaryomyces, Dekkera, Eremothecium, Issatchenkia, Kazachstania, Kluyveromyces, Kodamaea, Lodderomyces, Pachysolen, Pichia, Saccharomyces, Saturnispora, Tetrapisispora, Torulaspora, Williopsis,* and *Zygosaccharomyces.*
30. The recombinant yeast cell according to item 29, wherein the recombinant yeast cell is derived from the yeast genus *Kluyveromyces.*
31. The recombinant yeast cell according to item 30, wherein the recombinant yeast cell is derived from the yeast species *Kluyveromyces marxianus.*
32. The recombinant yeast cell according to item 31, wherein the recombinant yeast cell is derived from *Kluyveromyces marxianus* strain NBRC1777.
33. The recombinant yeast cell according to any one of the preceding items, wherein the yeast cell is haploid.
34. The recombinant yeast cell according to item 33, wherein the yeast cell is heterothallic, preferably wherein the yeast cell comprises deletions and/or mutations rendering a gene required for mating type switching from MATa to MATα or a gene required for mating type switching from MATα to MATa non-functional.
35. The recombinant yeast cell according to any one of items 33 or 34, wherein the yeast cell is mating competent.
36. The recombinant yeast cell according to any one of items 1 to 32, wherein the yeast cell is diploid or tetraploid.
37. A diploid recombinant yeast cell obtained by mating a first and a second haploid recombinant yeast cell according to any one of items 33 to 35.
38. The diploid recombinant yeast cell according to item 37, comprising at least two selection markers genomically integrated at chromosomal loci located on homologous chromosomes, preferably wherein the first and the second haploid recombinant yeast cell are each auxotrophic and the diploid recombinant yeast cell is able to grow under selection conditions under which the first and the second haploid recombinant yeast cell exhibit reduced or no growth or proliferation.
39. The diploid recombinant yeast cell according to any one of items 37 and 38, wherein the first transgene and the second transgene of each expression cassette are different from the first and second transgene of each further expression cassette.
40. The diploid recombinant yeast cell according to any one of items 37 to 39, wherein the first and the second haploid recombinant yeast cell each comprise an identical number of genomically integrated expression cassettes and wherein the expression cassettes are genomically integrated at the same chromosomal loci in the first and the second haploid recombinant yeast cell.
41. A vaccine composition comprising at least one recombinant yeast cell according to any one of the preceding items.
42. The recombinant yeast cell according to any one of items 1 to 40 or the vaccine composition according to item 41, for use in vaccinating a subject against an infectious disease.
43. The recombinant yeast cell or the vaccine composition for the use according to item 42, wherein the subject is a mammal, preferably a pig.
44. The recombinant yeast cell or the vaccine composition for the use according to any one of items 42 and 43, wherein the infectious disease is a viral disease, preferably selected from the group consisting of Porcine Rotavirus A or Porcine Rotavirus C, more preferably wherein the infectious disease is Porcine Rotavirus A.
45. A method for the production of a whole yeast vaccine comprising at least one diploid recombinant yeast cell according to item 37, the method comprising generation of the diploid recombinant yeast cell by mating of a first and second haploid yeast cell; wherein
   the first yeast cell is of mating type MATa and comprises a first expression cassette and the second yeast cell is of mating type MATα and comprises a second expression cassette, wherein the first and second expression cassette are stably integrated into the genome of the first and second yeast cell at the same chromosomal locus, wherein the first and second yeast cell are haploid, heterothallic and mating competent; and
   wherein each transgene encodes an immunogenic polypeptide derived from a pathogen.
46. The method for the production of a whole yeast vaccine according to item 45, wherein the first yeast cell of mating type MATa comprises a third expression cassette and the second yeast cell of mating type MATα comprises a fourth expression cassette, wherein the third and fourth expression cassette are stably integrated into the genome of the first and second yeast cell at the same chromosomal locus;
   optionally wherein the first yeast cell of mating type MATa further comprises a fifth expression cassette and the second yeast cell of mating type MATα further comprises a sixth expression cassette, wherein the fifth and sixth expression cassette are stably integrated into the genome of the first and second yeast cell at the same chromosomal locus;
   further optionally wherein the first yeast cell of mating type MATa further comprises a seventh expression cassette and the second yeast cell of mating type MATα further comprises an eighth expression cassette, wherein the seventh and eighth expression cassette are stably integrated into the genome of the first and second yeast cell at the same chromosomal locus.
47. A method for the production of a diploid recombinant yeast cell according to item 37 from a wild type yeast strain comprising
   (a) generation of a first yeast cell of mating type MATa and a second yeast cell of mating type MATα from the wild type yeast strain, such that the first and second yeast cell are each heterothallic, isogenic and mating competent;
   (b) transforming each of the first and second yeast cell with at least one deletion cassette, such that the at least one deletion cassette stably integrates into the host cell genome at the same chromosomal locus in the first and in the second yeast cell;
      such that stable integration into the yeast cell genome results in the disruption of a gene required for the synthesis of an essential compound, preferably wherein the essential compound is a vitamin, an amino acid, a nucleoside, a nucleobase or one or more metabolic precursor thereof;
   (c) transforming each of the first and second yeast cell obtained in step (b) with a nucleic acid molecule comprising at least one expression cassette, such that the at least one expression cassette stably integrates into the yeast cell genome at the chromosomal locus comprising the deletion cassette of step (b), such that the ability of the first and second yeast cell to synthesize the essential compound of step (b) is restored; and
   (d) generating the diploid yeast cell by mating or spheroplast fusion of the first and second haploid yeast cell obtained in step (c);
   wherein the wild-type yeast strain comprises no genetic selection marker and wherein each deletion cassette comprises at least one marker gene, preferably an auxotrophic marker gene; and optionally IoxP sites flanking the marker gene; and wherein each expression cassette comprises
   (i) a bidirectional promoter element;
   (ii) a first transgene and a second transgene, wherein said first and second transgene are located at opposite ends of the bidirectional promoter element and wherein each transgene is operably linked to one side of the bidirectional promoter element;
   (iii) a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene; and
   (iv) at least one selection marker; and
   (v) a sequence for restoring the gene required for the synthesis of an essential compound of step (b).
48. The method according to item 47, wherein the wild type yeast strain is derived from a genus selected from the group consisting of *Andozyma, Ascobotryozyma, Citeromyces; Debaryomyces, Dekkera, Eremothecium, Issatchenkia, Kazachstania, Kluyveromyces, Kodamaea, Lodderomyces, Pachysolen, Pichia, Saccharomyces, Saturnispora, Tetrapisispora, Torulaspora, Williopsis,* and *Zygosaccharomyces.*
49. The method according to item 48, wherein the wild type yeast strain is derived from the yeast genus *Kluyveromyces.*
50. The method according to item 49, wherein the wild type yeast strain is derived from the yeast species *Kluyveromyces marxianus.*
51. The method according to item 50, wherein the wild type yeast strain is *Kluyveromyces marxianus* strain NBRC1777.

## Claims

1. A recombinant yeast cell comprising at least one genomically integrated expression cassette, wherein each expression cassette comprises
(i) a bidirectional promoter element;
(ii) a first transgene and a second transgene, wherein said first and second transgene are located at opposite ends of the bidirectional promoter element and wherein each transgene is operably linked to one side of the bidirectional promoter element;
(iii) a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene; and
(iv) at least one selection marker.

2. The recombinant yeast cell according to claim 1, wherein each transgene encodes an immunogenic polypeptide derived from a pathogen, or an immunogenic fragment thereof, preferably, wherein the pathogen is a viral pathogen.

3. The recombinant yeast cell according to claim 2, wherein the pathogen is a viral pathogen belonging to the family of *Reoviridae,* preferably, wherein said viral pathogen is
a) a rotavirus, preferably Porcine Rotavirus A (PRVA) or Porcine Rotavirus C (PRVC); or
b) is an orbivirus, preferably African Horse Sickness Virus (AHSV) or Bluetongue virus (BTV).

4. The recombinant yeast cell according to any one of the preceding claims, wherein the bidirectional promoter element is
a) a native bidirectional yeast promoter element, optionally selected from the group consisting of a *LAC4*/*LAC12* promoter, a *GAL1*/*GAL10* promoter and a *GAP1*/*ADH2* promoter; or
b) a hybrid bidirectional promoter element, optionally selected from the group consisting of a *TDH3*/*ADH1* promoter, a *GAL1*/*GAL10-GPD* promoter and a *TEF1*/*PGK1* promoter.

5. The recombinant yeast cell according to any one of the preceding claims, wherein the bidirectional promoter element enables inducible or repressible expression of the first and/or the second transgene.

6. The recombinant yeast cell according to any one of the preceding claims, wherein each expression cassette is genomically integrated at a chromosomal locus selected from the group consisting of *ADE1, ADE2, ADE8, ECM31, HIS2, HIS3, HIS5, LEU1, LEU2, LYS2, LYS5, MET5, MET17, TRP1, TRP3, TRP4, TRP5, TYR1, URA3, URA5, FCY1, GAP1, LAC4* + *LAC12, XYL1 and XYL2.*

7. The recombinant yeast cell according to any one of the preceding claims, wherein the selection marker is a resistance marker or a metabolic selection marker, preferably a metabolic selection marker.

8. The recombinant yeast cell according to any one of the preceding claims, wherein the yeast cell is
a) haploid; or
b) diploid or tetraploid.

9. The recombinant yeast cell according to claim 8 a), wherein the yeast cell is heterothallic, preferably, wherein the yeast cell comprises deletions and/or mutations rendering a gene required for mating type switching from MATa to MATα or a gene required for mating type switching from MATα to MATa non-functional.

10. A diploid recombinant yeast cell obtained by mating a first and a second haploid recombinant yeast cell according to any one of claims 8 a) or 9.

11. The diploid recombinant yeast cell according to claim 10, comprising at least two selection markers genomically integrated at chromosomal loci located on homologous chromosomes.

12. A vaccine composition comprising at least one recombinant yeast cell according to any one of the preceding claims.

13. The recombinant yeast cell according to any one of claims 1 to 11, or the vaccine composition according to claim 12, for use in vaccinating a subject against an infectious disease.

14. A method for the production of a whole yeast vaccine comprising at least one diploid recombinant yeast cell according to claim 10, the method comprising
generation of the diploid recombinant yeast cell by mating of a first and second haploid yeast cell according to any one of claims 8 a) or 9, wherein
the first yeast cell is of mating type MATa and comprises a first expression cassette and the second yeast cell is of mating type MATα and comprises a second expression cassette, wherein the first and second expression cassette are stably integrated into the genome of the first and second yeast cell at the same chromosomal locus, wherein the first and second yeast cell are haploid, heterothallic and mating competent; and
wherein each transgene encodes an immunogenic polypeptide derived from a pathogen.

15. A method for the production of a diploid recombinant yeast cell from a wild type yeast strain comprising
(a) generating a first yeast cell of mating type MATa and a second yeast cell of mating type MATα from the wild type yeast strain, such that the first and second yeast cell are each heterothallic, isogenic and mating competent;
(b) transforming each of the first and second yeast cell with at least one deletion cassette such that the at least one deletion cassette stably integrates into the host cell genome at the same chromosomal locus in the first and in the second yeast cell;
such that stable integration into the yeast cell genome results in the disruption of a gene required for the synthesis of an essential compound, preferably wherein the essential compound is a vitamin, an amino acid, a nucleoside, a nucleobase or one or more metabolic precursor thereof;
(c) transforming each of the first and second yeast cell obtained in step (b) with a nucleic acid molecule comprising at least one expression cassette such that the at least one expression cassette stably integrates into the yeast cell genome at the chromosomal locus comprising the deletion cassette of step (b), such that the ability of the first and second yeast cell to synthesize the essential compound of step (b) is restored; and
(d) generating the diploid yeast cell by mating or spheroplast fusion of the first and second haploid yeast cell obtained in step (c);
wherein the wild-type yeast strain comprises no genetic selection marker and wherein each deletion cassette comprises at least one marker gene, preferably an auxotrophic marker gene; and optionally IoxP sites flanking the marker gene; and wherein each expression cassette comprises
(i) a bidirectional promoter element;
(ii) a first transgene and a second transgene, wherein said first and second transgene are located at opposite ends of the bidirectional promoter element and wherein each transgene is operably linked to one side of the bidirectional promoter element;
(iii) a first transcription terminator and a second transcription terminator, said first transcription terminator being located immediately downstream of the first transgene and said second transcription terminator being located immediately downstream of the second transgene; wherein the first transcription terminator is operably linked to the first transgene and the second transcription terminator is operably linked to the second transgene; and
(iv) at least one selection marker; and
(v) a sequence for restoring the gene required for the synthesis of an essential compound of step (b).
